# EUROPEAN PATENT APPLICATION

(11) **EP 4 796 632 A1**
(43) Date of publication of application: **26.08.2026**
(21) Application number: 25159422.2
(22) Date of filing: 21.02.2025
(51) Int. Cl.: C12N 15/10, C12P 19/34, C07K 17/00, G01N 33/543

(54) **IMPROVED SYNTHESIS OF A PUROMYCIN LINKER**

(71) Applicant: Deutsches Krebsforschungszentrum Stiftung des öffentlichen Rechts, 69120 Heidelberg (DE); Albert-Ludwigs-Universität Freiburg, 79098 Freiburg (DE)
(72) Inventor: The designation of the inventor has not yet been filed
(74) Representative: Altmann Stößel Dick Patentanwälte PartG mbB

(57) **Abstract**

The present invention relates to a method for producing a display complex, the method comprising
(I) providing a covalent mRNA-Puromycin Linker conjugate;
(II) contacting the covalent mRNA-Puromcyin linker conjugate with an in vitro translation composition to produce a display complex comprising a polypeptide encoded by said mRNA,
(III) optionally reverse transcribing said mRNA to a cDNA, and
(IV) cyclisation of the polypeptide comprised in said display complex.

## Description

### FIELD OF THE INVENTION

The present invention relates to a method (P) for preparing a Puromycin Linker (I) for producing a complex of an mRNA and a polypeptide encoded by said mRNA, the method comprising linking a building block A, said building block comprising a Puromycin group (Pur) and a functional group FG^{a} being an azide via said functional group FG^{a}, with a building block B comprising a functional group FG^{b} having the structure 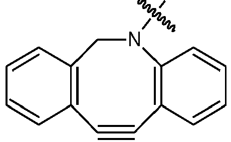 via said functional group FG^{b}, wherein B further comprises and a single-stranded polynucleotide segment essentially complementary to a sequence on the 3'-terminal side of the mRNA over a stretch of at least 3, preferably at least 12 nucleotides, more preferably at least 15, more preferably at least 22 nucleotides, and wherein the functional group FG^{a} and the functional group FG^{b} are linked via a strain-promoted azide-alkyne cycloaddition. Further, the present invention also relates to the buildings blocks A and B as such and methods for using the Puromycin Linker (I).

### BACKGROUND

Molecular display techniques have long been used for protein and peptide evolution to create molecules that can bind to a desired target. In vivo methods like phage display and bacterial display are available, but also in vitro methods like ribosome display, mRNA display, and cDNA display are commonly used for this purpose.

In vitro display methods are powerful technologies used to explore functional peptides and proteins from a huge library by in vitro selection and/or detection. In addition to expediting the selection cycle, easy and rapid functional analysis of selected candidate clones is crucial for high-throughput screening of functional peptides and proteins.

Ribosome display relies on the natural association between mRNA, Ribosome, and the nascent polypeptide chain resulting if an mRNA template lacking a stop codon is provided as a template. The nascent polypeptide chain can be screened for functionality, and by it association via the ribosome, the encoding mRNA can be amplified and used in further rounds of selection. mRNA display adds a further step of covalently connecting the nascent polypeptide to the template mRNA via puromycin, thus obviating the need to stabilize ribosomes during the screening process. Further improvement was achieved by attaching the puromycin moiety to a DNA hybridizing to the mRNA instead of the mRNA itself (for a review, cf. e.g. Arai et al. (2019) in Stefan Zielonka and Simon Krah (eds.), Genotype Phenotype Coupling: Methods and Protocols, Methods in Molecular Biology, vol. 2070, doi.org/10.1007/978-1-4939-9853-1_3).

Mochizuki et al. (Journal of Biotechnology 212 (2015) 174-180) reported a versatile puromycin-linker employing an ultrafast photo-cross-linker, 3-cyanovinylcarbazole nucleoside. Using this puromycin-linker, they demonstrated the model in vitro selection of the FLAG epitope and a SPR-based assay to measure the dissociation constant between the B domain of protein A and immunoglobulin G.

According to standard methods, the Puromycin linker is prepared by reacting two building blocks via a Michael Reaction (Arai, H., Kumachi, S., & Nemoto, N. (2020). cDNA Display: A Stable and Simple Genotype-Phenotype Coupling Using a Cell-Free Translation System. In Methods in Molecular Biology (Vol. 2070, pp. 43-56). However, the inventors of the present invention have shown that said Michael Reaction is not selective. Thus, undesired side products are formed and the desired product is only obtained in a rather low yield.

Thus, there is still the need for improved methods for preparing a Puromycin Linker for producing a complex of an mRNA and a polypeptide encoded by said mRNA and methods for using the same.

Provided herein, inter alia, are methods for preparing a Puromycin Linker (I) for producing a complex of an mRNA and a polypeptide encoded by said mRNA. The methods described herein can in particular improve the efficiency and selectivity of the preparation of the Puromycin Linker as well as the purity of the Linker.

### SUMMARY OF THE INVENTION

Thus, the present invention relates to a method (P) for preparing a Puromycin Linker (I) for producing a complex of an mRNA and a polypeptide encoded by said mRNA, the method comprising reacting (linking)
- a building block A, said building block comprising a Puromycin group (Pur) and a functional group FG^{a}, via said functional group FG^{a}, wherein the functional group FG^{a} is -N₃.
- with a building block B comprising a functional group FG^{b} via said functional group FG^{b}, wherein the functional group FG^{b} is 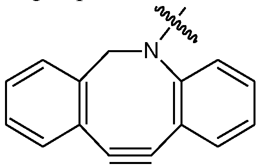
   thereby forming a covalent linkage between A and B,
   wherein B further comprises and a single-stranded polynucleotide segment essentially complementary to a sequence on the 3'-terminal side of the mRNA over a stretch of at least 3, preferably at least 12 nucleotides, more preferably at least 15, more preferably at least 22 nucleotides, wherein the functional group FG^{a} and the functional group FG^{b} are linked a strain-promoted azide-alkyne cycloaddition.

Further, the present invention relates to a Puromycin Linker for producing a complex of mRNA and a polypeptide encoded by that mRNA obtained or obtainable by the method described above and below.

Further, the present invention relates to a Puromycin Linker for producing a complex of an mRNA and a polypeptide encoded by said mRNA comprising
a building block A comprising a Puromycin group and a building block B comprising a single-stranded polynucleotide segment essentially complementary to a sequence on the 3'-terminal side of the mRNA over a stretch of at least 3, preferably at least 12 nucleotides, more preferably at least 15, more preferably at least 22 nucleotides.
wherein (A) and (B) are linked via the functional moiety a'-b', the linking group a'-b' having the structure

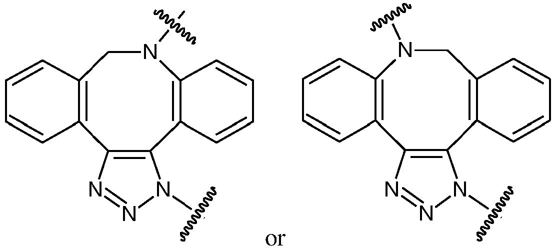

Further, the present invention relates to a building block A, having the structure FG^{a}-L²-(X)₁₋₅-(dT(Fluorescein))-(X)₃₋₁₀-L¹-(X)₁₋₅-(Pur), wherein Pur is a puromycin group, FG^{a} is an azide, each X is independently selected from, optionally substituted, A, T, G, and C, wherein L¹ and L² are linker, and wherein dT(Fluorescein) is fluorescein-labeled deoxy-thymidin and preferably has the structure: 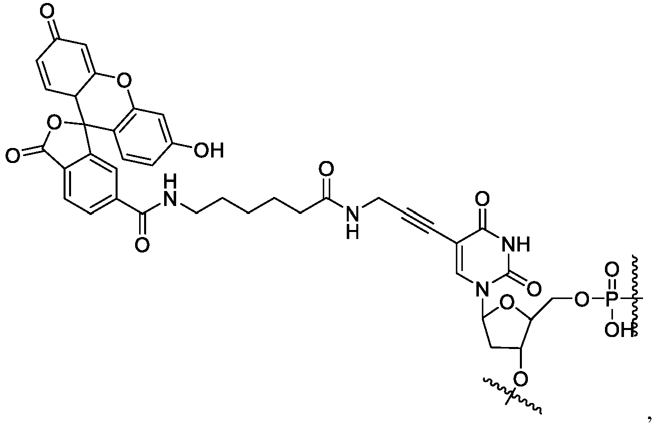 preferably, the structure FG^{a}-L²-TC-(dT(Fluorescein))-CTC-L¹-CC-(Pur).

According to an alternative preferred embodiment, the present invention relates to a building block A, having the structure FG^{a}-L²-(X)₄₋₁₅-L¹-(X)₁₋₅-(Pur), wherein Pur is a puromycin group, FG^{a} is an azide, wherein each X is independently selected from, optionally substituted, A, T, G, and C, wherein L¹ and L² are linkers, preferably having the structure FG^{a}-L²-TCTCTC-L¹-CC-(Pur).

Further, the present invention relates to a building Block B, having the structure

Z-(X)₂₋₅-rG-(X)₂₋₁₀-(CnVK)-(X)₅₋₁₅-X(L³-(FG^{b}))-(X)₃₋₅,

wherein FG^{b} 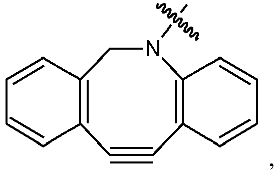
each X is independently selected from A, T, G, and C, wherein rG is ribo-guanosine, and A, T, G, an C are deoxynucleotides and wherein L³ is a linker and CnVK is a cyanovinylcarbazol group, and wherein Z is dT-Biotin or a linker biotinyl group, such as a group having the structure: 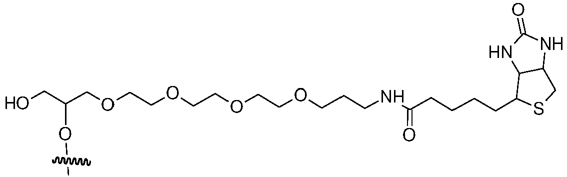
preferably the structure Z-AA-rG-AATTTCCA-(CnVK)-GCCGCCCCCCG-T(L³-(FG^{b}))-CCT, wherein L³ is a linker, CnVK is a cyanovinylcarbazol group, rG is ribo-guanosine, and A, T, G, an C are deoxynucleotides, and wherein Z is dT-Biotin or a group having the structure:

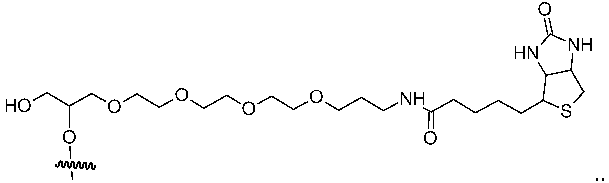

According to an alternative preferred embodiment, the present invention relates to a building Block B, having the structure

Z-(X)₂₋₅-rG-(X)₂₋₅-(dT(Fluorecein)-(X)₂₋₁₀-(CnVK)-(X)₅₋₁₅-X(L3-(FG^{b}))-(X)₃₋₅

wherein FG^{b}, as described above, and wherein each X is independently selected from A, T, G, and C, wherein rG is ribo-guanosine, and A, T, G, an C are deoxynucleotides, and wherein L³ is a linker and CnVK is a cyanovinylcarbazol group.

Furthermore, the present invention relates to a method for producing a covalent mRNA-Puromycin Linker conjugate, said method comprising the steps of the method (P) for preparing the Puromycin Linker (I), as described above and below, and the further steps
(a) contacting the Puromycin Linker produced in the preceding steps with an mRNA;
(b) photocrosslinking said mRNA and said Puromycin Linker, and thereby
(c) producing a covalent mRNA-Puromcyin Linker conjugate.

Further, the present invention relates to a method for producing a display complex, as well as to a display complex obtained or obtainable by said method, the method comprising
(I) providing a covalent mRNA-Puromycin Linker conjugate;
(II) contacting the covalent mRNA-Puromcyin linker conjugate of step (I) with an in vitro translation composition to produce a display complex comprising a polypeptide encoded by said mRNA,
(III) optionally reverse transcribing said mRNA to a cDNA,, and optionally
(IV) cyclisation of the polypeptide comprised in said display complex, preferably thereby producing a display complex, preferably a cDNA display complex.

Furthermore, the present invention relates to a protein array obtained by immobilizing a multitude of display complexes as described above and below on a substrate, preferably in a spatially ordered manner.

Furthermore, the present invention relates to a method for identifying a binding compound binding to a compound of interest via cDNA display, comprising the steps of the method for preparing a Puromycin Linker as described above and below, the steps of the method for producing a covalent mRNA-Puromycin Linker as described above and below, and/or the steps of the method for producing a display complex as described above and below.

The present invention also relates to a method (P) for preparing a Puromycin Linker (I) for producing a complex of an mRNA and a polypeptide encoded by said mRNA, the method comprising reacting
- a building block A, said building block comprising a Puromycin group (Pur) and a functional group FG^{a}, via said functional group FG^{a}, wherein the functional group FG^{a} is -N₃.
- with a building block B comprising a functional group FG^{b} via said functional group FG^{b}, wherein the functional group FG^{b} is 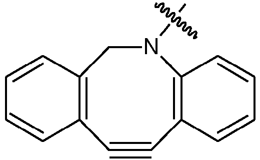
   thereby forming a covalent linkage between A and B,
   wherein B further comprises and a single-stranded polynucleotide segment essentially complementary to a sequence on the 3'-terminal side of the mRNA over a stretch of at least 3, preferably at least 12 nucleotides, more preferably at least 15, more preferably at least 22 nucleotides, wherein the functional group FG^{a} and the functional group FG^{b} are linked a strain-promoted azide-alkyne cycloaddition.

### DESCRIPTION OF THE INVENTION

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" preferably refer to "comprising one or more", i.e. are equivalent to "comprising at least one". In accordance, expressions relating to one item of a plurality, unless otherwise indicated, preferably relate to at least one such item, more preferably a plurality thereof; thus, e.g. providing "an mRNA" relates to providing at least one mRNA, preferably to providing a plurality, i.e. a multitude, of mRNAs.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

The methods specified herein below, preferably, are in vitro methods. The method steps may, in principle, be performed in any arbitrary sequence deemed suitable by the skilled person, but preferably are performed in the indicated sequence; also, one or more, preferably all, of said steps may be assisted or performed by automated equipment. Moreover, the methods may comprise steps in addition to those explicitly mentioned above.

Preferably, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1% by weight, most preferably less than 0.1% by weight of non-specified component(s).

### Cycloaddition

In the method (P) of the invention, the functional group FG^{a} and the functional group FG^{b} are reacted with each other via a strain-promoted azide-alkyne cycloaddition (SPAAC) also known as copper-free click chemistry.

Preferably, the reaction is carried out in the absence of a catalyst.

In the reaction according to the invention, the azide group reacts with the group 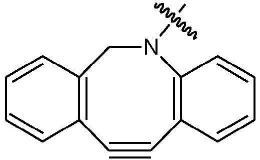 (DBCO, Dicyclobenzooctin), thereby forming a covalent linkage.

The linkage has one of the following structures.

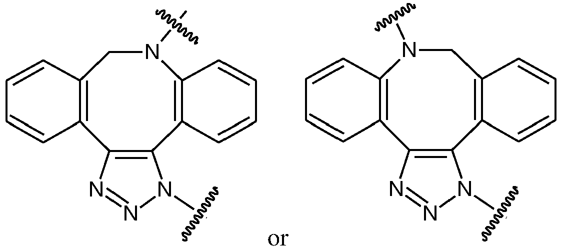

Thus, the resulting puromycin linker has preferably a structure selected from 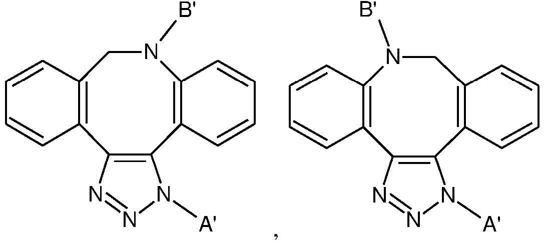 and mixtures thereof, wherein A' is the remainder of building block A and B' is the remainder of building block B.

It is to be understood that both isomers as well as any mixture thereof are equally suitable in the further methods of the invention, that is in the method for producing a covalent mRNA-Puromycin Linker as described above and below, and in the method for producing a display complex as described above and below as well as in the method for identifying a binding compound binding to a compound of interest via cDNA display.

### Building Block A

The building block A comprises a Puromycin group (Pur) and the functional group FG^{a}. preferably covalently connected by at least one linking group. As the skilled person understands, the purpose of the at least one linking group is to provide a suitable distance between the oligonucleotide comprised in building block B and the puromycin moiety. Thus, the skilled person can select the chemical nature, number, and size of the linking group(s) according to standard methods. Preferably, the linking group comprises at least one, preferably deoxy oligonucleotide, as is usual in the preparation of puromycin linkers and their building blocks (cf. e.g. Arai, et al. (2019), loc. cit.).

Thus, building block A may have the structure FG^{a}-L²-(X)₄₋₁₅-L¹-(X)₁₋₅-(Pur), wherein Pur is a puromycin group, FG^{a} is an azide, wherein each X is independently selected from, optionally substituted, A, T, G, and C, wherein L¹ and L² are linkers. More preferably, building block A preferably has the structure FG^{a}-L²-TCTCTC-L¹-CC-(Pur). More preferably, building block A has the structure FG^{a}-L²-(X)₄₋₁₅-L¹-(X)₁₋₅-(Pur), wherein Pur is a puromycin group, FG^{a} is a functional group, wherein each X is independently selected from, optionally substituted, A, T, G, and C, wherein L¹ and L² are linkers. Most preferably, building block A has the structure FG^{a}-L²-TCTCTC-L¹-CC-(Pur). Most preferably, building block A has the structure FG^{a}-L²-TCTCTC-L¹-CC-(Pur), more preferably the structure

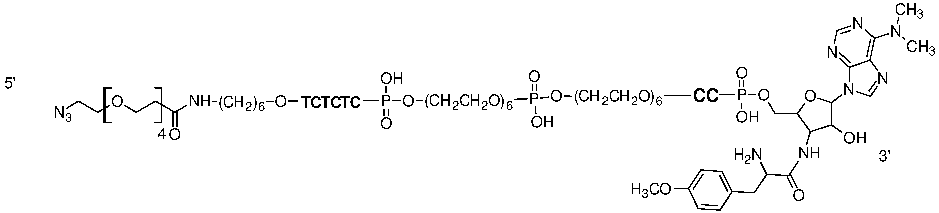

The building block A may have the structure (A1)

FG^{a}-L²-(X)₁₋₅-(dT(Fluorescein))-(X)₃₋₁₀-L¹-(X)₁₋₅-(Pur) (A1)

wherein Pur is a puromycin group, FG^{a} is an azide, each X is independently selected from A, T, G, and C, wherein L¹ and L² are linkers, and wherein dT(Fluorescein) is fluorescein-labeled deoxy-thymidin as specified herein above; more preferably, according to this embodiment, the building block A has the structure

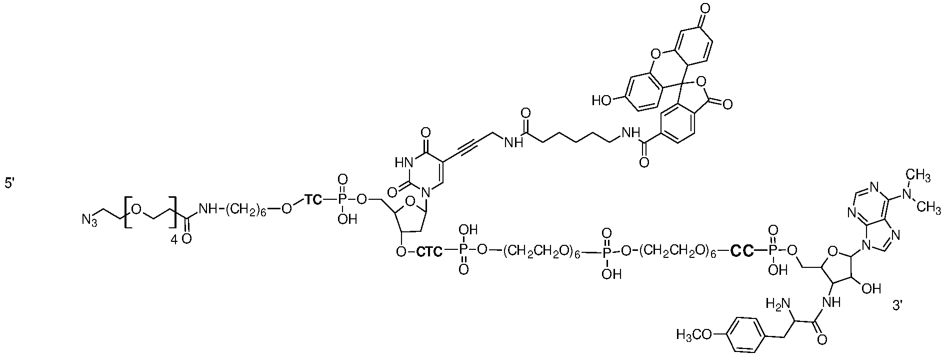

The azide according to the invention is preferably -N₃.

### Puromycin moiety

Puromycin is an aminonucleosi antibiotic, derived from the *Streptomyces alboniger* bacterium. Preferably, the puromycin group (Pur) has the structure

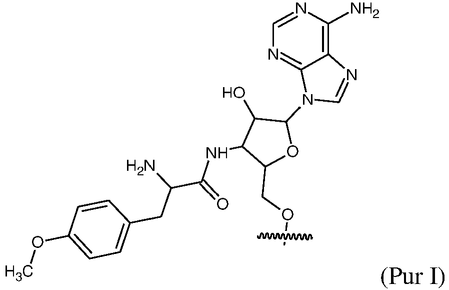

Thus, the present invention relates to a method, as described above and below, as well as to a Puromycin Linker obtained or obtainable by said method, as described above and below, comprising the group (Pur) having the structure (Pur I). Further, the present invention also relates to a Puromycin Linker, as described above and below, and a building block (A, as described above and below, comprising the group (Pur) having the structure (Pur I).

### Building Block B

Building block B is a building block comprising the functional group FG^{b} and a single-stranded polynucleotide segment essentially complementary to a sequence on the 3'-terminal side of the mRNA over a stretch of at least 14, preferably at least16, preferably at least 18, more preferably at least 20, even more preferably at least 22, even more preferably of at least 25 nucleotides. The nucleic acid sequence comprised in building block B may be selected by the skilled person according to methods well-known in the art; in particular, sequences may be selected which provide for stable base-pairing of the Puromycin Linker to the mRNA of interest under the conditions of the methods described herein. Preferably, building block B comprises the nucleic acid sequence of any one of SEQ ID NOs:1 to 5, more preferably of any one of SEQ ID NOs: 6 to 8.

Preferably, the aforesaid polynucleotide, preferably comprising the nucleic acid sequence of any one of SEQ ID NOs:1 to 8, is coupled at its 5' end to group Z, preferably as specified herein below. Also preferably, the aforesaid polynucleotide comprises at least one cnvK nucleotide as specified elsewhere herein. Also preferably, building block B further comprises a single-stranded polynucleotide segment comprising a cleavage site, said cleavage site preferably comprising ribo-guanosine (rG), more preferably wherein said cleavage site is an Rnase T1 recognition site, preferably in at least one of positions 1 to 5, more preferably at position 3. More preferably, the aforesaid oligonucleotide comprises at least one cnvK nucleotide as specified elsewhere herein and an rG nucleotide, preferably in at least one of one of positions 1 to 5, more preferably at position 3. Thus, the oligonucleotide preferably has the nucleic acid sequence of SEQ ID NO:6. Also preferably, the building block B further comprises a reverse transcription primer region at the 3'-terminal end, preferably a nucleic acid sequence CCT; Thus, building block B may in particular comprise the nucleic acid sequence of SEQ ID NO:2, 5, 6, 7, or 8.

Preferably, building block B has the structure

Z-(X)₂₋₅-rG-(X)₂₋₁₀-(CnVK)-(X)₅₋₁₅-X(L³-(FG^{b}))-(X)₃₋₅,

wherein FG^{b} is as described above, each X is independently selected from, optionally substituted, A, T, G, and C, wherein rG is ribo-guanosine, and A, T, G, an C are, optionally substituted, deoxynucleotides and wherein L³ is a linker and CnVK is a cyanovinylcarbazol group, and wherein Z is dT-Biotin or a linker-biotinyl group as specified herein above. Preferably, building block B has the structure Z-AA-rG-AATTTCCA-(CnVK)-GCCGCCCCCCG-T(L³-(FG⁸))-CCT (SEQ ID NO:6), wherein L³ is a linker, CnVK is a cyanovinylcarbazol group, rG is ribo-guanosine, and A, T, G, an C are deoxynucleotides, and wherein Z is dT-Biotin or a linker-biotinyl group as specified herein above.

Preferably, the building block B further comprises a cyanovinylcarbazol group (CnVK), preferably a group having the structure

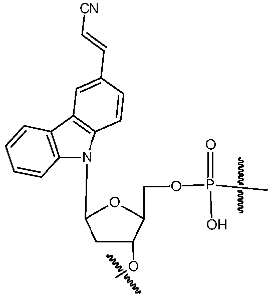

Preferably, the building block B further comprises a biotin group Z. The biotin group may be attached via any suitable linker to the building block B.

Preferably, Z is dT-Biotin or a linker-biotinyl group having the structure:

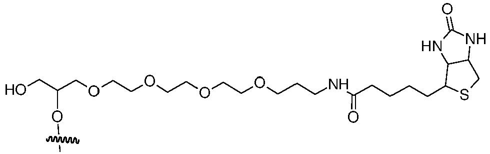

Optionally, building block B may be labeled with a labeling group as specified herein below, more preferably with fluorescein. More preferably, building block B is labeled by including a fluorescein-labeled dT residue in the structure, wherein said fluorescein-labeled dT residue preferably has the structure as specified herein above. Thus, building block B may in particular have the structure

Z-(X)₂₋₅-rG-(X)₂₋₅-(dT(Fluorecein)-(X)₂₋₁₀-(CnVK)-(X)₅₋₁₅-X(L3-(FG^{b}))-(X)₃₋₅

wherein FG^{b} is as described above, and wherein each X is independently selected from A, T, G, and C, wherein rG is ribo-guanosine, and A, T, G, an C are deoxynucleotides, and wherein L³ is a linker and CnVK is a cyanovinylcarbazol group. More preferably building block B has the structure
Z-AA-rG-AA-(dT(Fluorescein)-TTCCA-(CnVK)-GCCGCCCCCCG-T(L3-(FG^{b}))-CCT (SEQ ID NO:7),
wherein L³ is a linker, CnVK is a cyanovinylcarbazol group, rG is ribo-guanosine, and A, T, G, an C are deoxynucleotides, and Z is wherein Z is dT-Biotin or a linker-biotinyl groups specified herein above.

In particular, the building block B has the structure (B1)

Z-(X)₂₋₅-rG-(X)₂₋₁₀-(CnVK)-(X)₅₋₁₅-X(L³-(FG^{b}))-(X)₃₋₅ (B1)

wherein each X is independently selected from A, T, G, and C, wherein rG is ribo-guanosine, and A, T, G, an C are deoxynucleotides and wherein L³ is a linker and CnVK is a cyanovinylcarbazol group, and wherein Z is dT-Biotin or a group having the linker-biotinyl structure as specified herein above.

Linker L³ is a linking moiety. In general, there are no particular restrictions as to the chemical nature of the linking moiety L³ with the proviso it provides suitable chemical properties for the building block A and its intended use. Thus, the term Linker L³ refers any suitable chemical moiety linking the respective nucleotides X and typically includes moieties comprising groups selected from the group consisting of alkoxyalkyl, alkyl, alkenyl, alkynyl, alkylaryl, arylalkyl, aryl, alkylarylalkyl, heteroaryl, alkylheteroaryl, alkylheteroalkyl, heteroarylalkyl, cycloalkyl and alkyl groups.

X(L³-(FG^{b}) is a deoxynucleotide, such as A, T, G or C, being substituted with the moiety L³-FG^{b} at the base. Preferably, X(L³-(FG^{b}) has the structure:

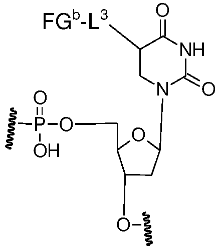

Preferably, L³ comprises alkoxyalkyl groups or alkyl groups. More preferably, L3 has the structure:

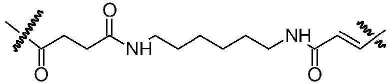

Preferably, (L³-(FG^{b}) has the structure: 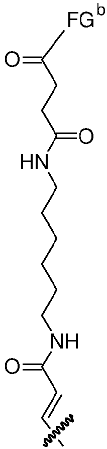 in particular

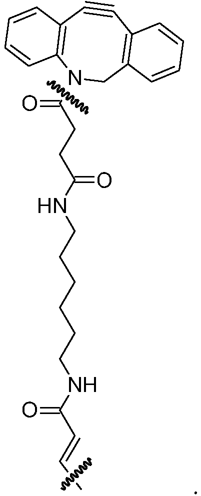

More preferably, the building block B has the structure (B2)
Z-AA-rG-AATTTCCA-(CnVK)-GCCGCCCCCCG-T(L³-(FG^{b}))-CCT (SEQ ID NO:6) (B2)
wherein L³ is the linker, CnVK is a cyanovinylcarbazol group, rG is ribo-guanosine, and A, T, G, an C are deoxynucleotides, and wherein Z is dT-Biotin (SEQ ID NO: 13) or a linker-biotinyl group as specified herein above.

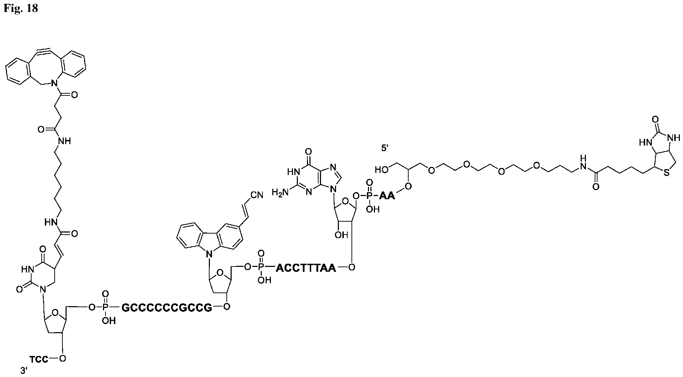

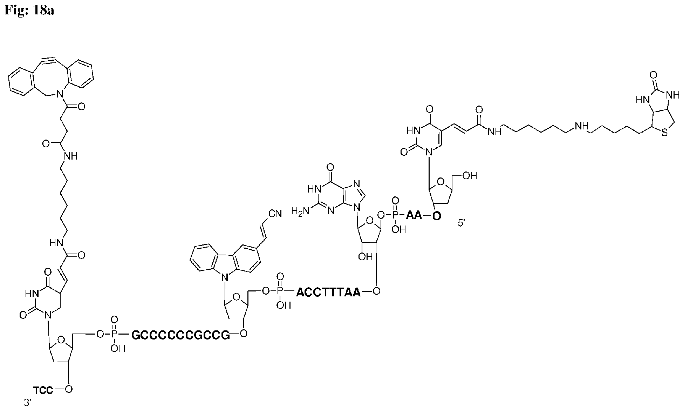

In particular, the building block B has the structure (B3), as also shown in Fig. 18, and below

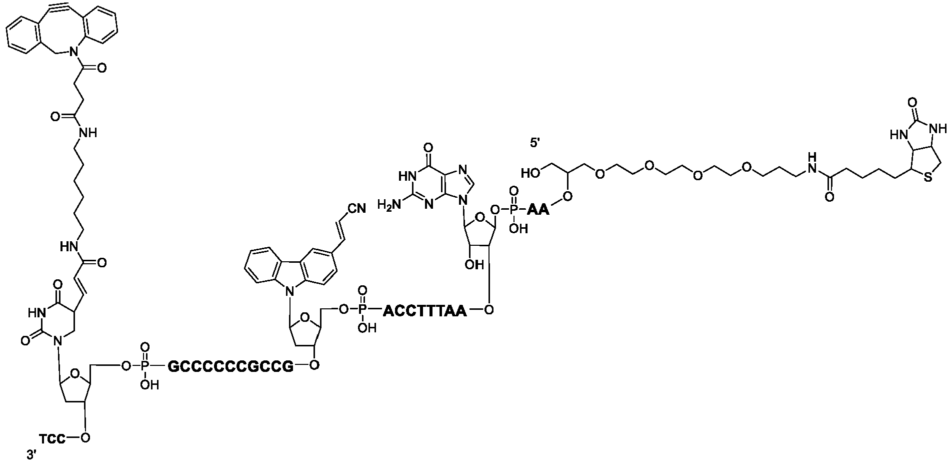

Alternatively, the building block B has the structure (B'3), as shown below and in Fig. 18a

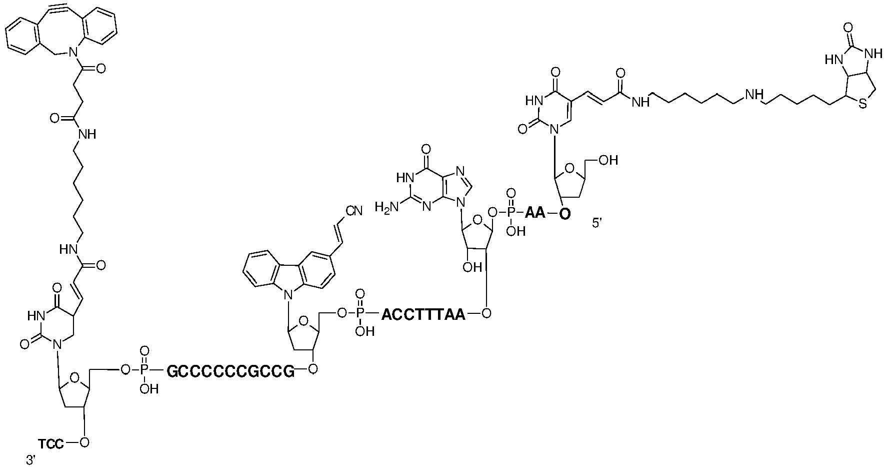

As specified herein above and below, building block B may comprise a labeling group. According to this embodiment, the building block B preferably has the structure

Z-(X)₂₋₅-rG-(X)₂₋₅-(dT(Fluorecein))-(X)₂₋₁₀-(CnVK)-(X)₅₋₁₅-X(L3-(FG^{b}))-(X)₃₋₅, (2B1)

wherein each X is independently selected from A, T, G, and C, wherein rG is ribo-guanosine, and A, T, G, an C are deoxynucleotides, and wherein L³ is a linker and CnVK is a cyanovinylcarbazol group.

Preferably, building block B has the structure (2B2):
Z-AA-rG-AA-(dT(Fluorescein)-TTCCA-(CnVK)-GCCGCCCCCCG-T(L3-(FG^{b}))-CCT, (2B2, SEQ ID NO: 7)
wherein L³ is a linker, CnVK is a cyanovinylcarbazol group, rG is ribo-guanosine, and A, T, G, an C are deoxynucleotides, and wherein Z is dT-Biotin (SEQ ID NO: 12) or a linker-biotinyl group, all as described herein above.

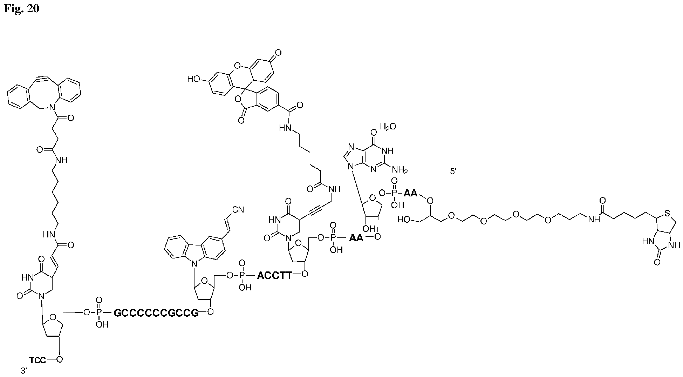

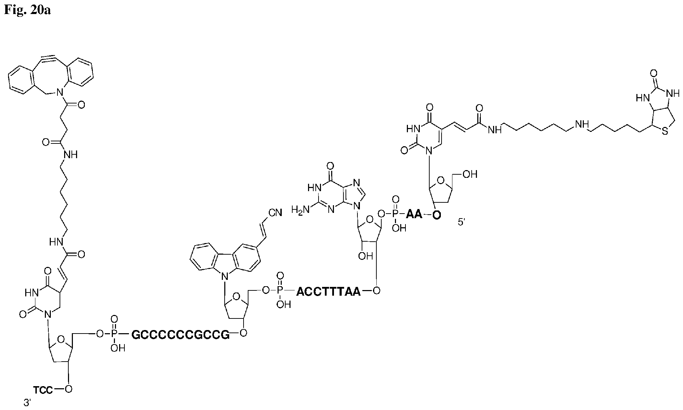

Accordingly, building block B has preferably the structure (2B3) as shown below (see Fig. 20):

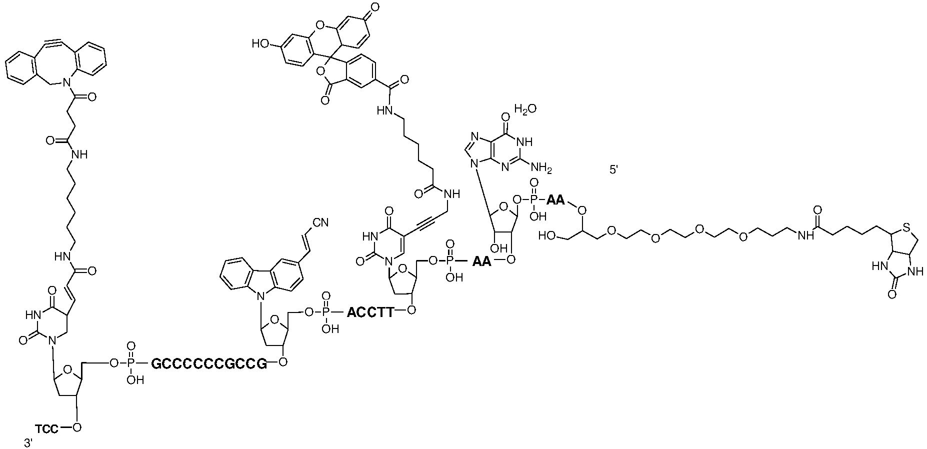

Alternatively, building block B has preferably the structure (2B'3) as shown below (see Fig. 20a):

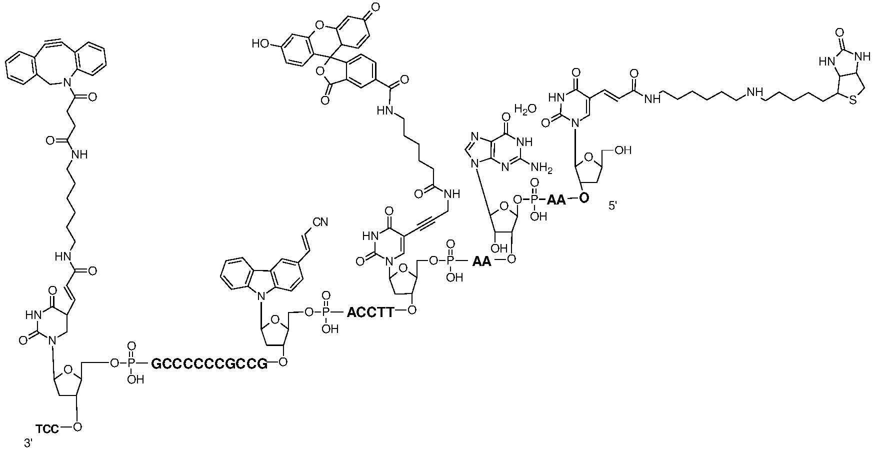

As described above, FG^{a} is an azide (-N₃) and FG^{b} is

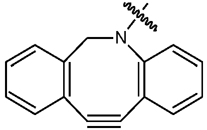

The Puromycin Linker obtained or obtainable by said method, as described above and below, as well as to a Puromycin Linker, as described above and below, thus comprises the linking group a'-b', wherein the linking group a'-b' is

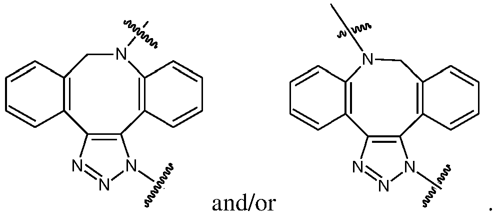

Thus, the Puromycin Linker obtained may be a mixture of two regioisomers, or a single regioisomer, wherein both regioisomers are equally suitable in the methods of the present invention. Thus, also the mixture is equally suitable in the methods of the present invention.

Further, the present invention also relates to a Puromycin-linker, as described above and below, and a building block A, as described above and below, comprising the group (Pur) having the structure (Pur I).

Preferably, in the reaction the molar ratio of building block A to building block B is > 1 : 1. Thus, an excess of building block A is used. More preferably, the molar ratio of building block A to building block B is ≥2 : 1. The use of an excess of building block A is advantageous in that a high amount, preferably all, of building block B is converted to the Puromycin linker. Although, the resulting Puromycin linker thus comprises unreacted building block A, this mixture comprising unreacted building block A may be directly used in the methods of the invention without further purification, that is in the method for producing a covalent mRNA-Puromycin Linker as described above and below, and in the method for producing a display complex as described above and below as well as in the method for identifying a binding compound of interest via cDNA display.

Thus, according to one preferred embodiment, the mixture comprising unreacted building block A is directly used in the methods of the invention without further purification. Alternatively, the reaction product may be purified.

According to an alternative embodiment, the reaction product is purified, preferably by HPLC.

Preferably, in the reaction the molar ratio of building block A to building block B is of from 1.1 : 1 to 5:1, preferably of from 1.5 :1 to 3:1, more preferably around 2:1.

Preferably, the reaction is carried out in water, preferably nuclease free water, preferably without the need to control the pH of the reaction mixture.

Preferably, the building block A and the building block B are reacted with each other for a time in the range of from 2 min to 48 h, preferably of from 5 min to 24 h, preferably of from 10 min to 5 h, more preferably around 3 h.

In particular, in the method for preparing the Puromycin Linker of the present invention an excess of building block A is used, preferably wherein the molar ratio of building block A to building block B is of from 1.1 : 1 to 5:1, preferably of from 1.5 :1 to 3:1, more preferably around 2:1, and the building block A and the building block B are reacted with each other for a time in the range of from 5min to 24h, preferably for a time in the range of from 10 min to 5 h, more preferably around 3 h.

Preferably, the building block A and the building block B are reacted with each other at a temperature in the range of from 0 °C to 45 °C, preferably in the range of from 1 °C to 40 °C, more preferably in the range of from 5 to 40 °C, more preferably in the range of from 10 °C to 40 °C, more preferably in the range of from 25 °C to 40 °C, more preferably in the range of from 30 °C to 40 °C, more preferably in the range of from 35 °C to 40 °C, in particular around 37 °C.

Preferably, the reaction yield with respect to the conversion of building block B is at least 90%, more preferably at least 95%, more preferably at least 99.9%.

### Labeling Group:

Preferably, building block A and/or building block B further comprise a labeling group, preferably a labeled nucleotide. A "labeled nucleotide" in this context refers to a nucleotide comprising a labeling group. Preferably, the labeled nucleotide is a nucleotide with a base being substituted with a labeling group.

Preferably, building block A and/or building block B comprises a single stranded polynucleotide segment, comprising a nucleotide with a base being substituted with a labeling group (labeled nucleotide.

Such labeling groups include chromophores that absorb and/or emit light of different wavelengths to provide colored indications of an event, chemiluminescent labels that can spontaneously emit light in response to a particular chemical event and fluorescent labels that emit light in response to excitation by light of a different wavelength.

Preferably, the labeling group is fluorescent group.

A fluorescent group or fluorescent dye as used herein may be understood in the broadest sense as any dye moiety enabling fluorescence detection. Preferably, such fluorescence detection is in a range of from 350 to 1000 nm, i.e. in the visible spectrum and in the Near Infrared (NIR) spectrum, in particular in a range of from 400 to 850 nm, i.e. in the visible spectrum. Numerous fluorescent dye moieties are known in the art, and will be readily apparent to one of ordinary skill. Preferably, the dye enables a fluorescence detection by radiation with a wavelength in a range of from 350 to 800 nm, preferably 450 nm to 550 nm, such as with a wavelength of 490 to 510 nm, such as as a radiation with a wavelength of 498 nm.

The fluorescent group is preferably selected from the group consisting of AlexaFluor 3, AlexaFluor 5, AlexaFluor 350, AlexaFluor 405, AlexaFluor 430, AlexaFluor 488, AlexaFluor 500, AlexaFluor 514, AlexaFluor 532, AlexaFluor 546, AlexaFluor 555, AlexaFluor 568, AlexaFluor 594, AlexaFluor 610, AlexaFluor 633, AlexaFluor 647, AlexaFluor 660, AlexaFluor 680, AlexaFluor 700, and AlexaFluor 750, Cy2, Cy3, Cy3B, Cy3.5, Cy5, sulfoCy5, Cy5.5 and Cy7, DyLight 350, DyLight 405, DyLight 488, DyLight 550, DyLight 594, DyLight 633, DyLight 650, DyLight 680, DyLight 750 and DyLight 800, FluoProbes 390, FluoProbes 488, FluoProbes 532, FluoProbes 547H, FluoProbes 594, FluoProbes 647H, FluoProbes 682, FluoProbes 752 and FluoProbes 782, AMCA, DEAC (7-Diethylaminocoumarin-3-carboxylic acid), 7-Hydroxy-4-methylcoumarin-3, 7-Hydroxycoumarin-3-carboxylic acid (Pubchem SID 135727263), MCA (7-Methoxycoumarin-4-acetic acid) (Pubchem CID 342221), 7-Methoxycoumarin-3, Fluoresceine, AMF (4'-(Aminomethyl)fluorescein), 5-DTAF (5-(4,6-Dichlorotriazinyl)aminofluorescein), 6-DTAF (6-(4,6-Dichlorotriazinyl)aminofluorescein), 6-FAM (6-Carboxyfluorescein), 5(6)-FAM cadaverine (Fluorescein-5(6)-carboxamide cadaverine), 5-FAM cadaverine (Fluorescein-5-carboxamide cadaverine), 5(6)-FAM ethylenediamine (Fluorescein-5(6)-carboxamide ethylenediamine) , 5-FAM ethylenediamme (Fluorescein-5(6)-carboxamide ethylenediamine), 5-FITC (FITC Isomer I; fluorescein-5-isothiocyanate), 5-FITC cadaverin; Fluorescein-5-maleimide; 5-IAF (5-Iodoacetamidofluorescein), 6-JOE (6-Carboxy-4',5'-dichloro-2',7'-dimethoxyfluorescein), 5-CR110 (5-Carboxyrhodamine 110), 6-CR110 (6-Carboxyrhodamine 110), 5-CR6G (5-Carboxyrhodamine 6G); 6-CR6G (6-Carboxyrhodamine 6G), 5(6)-Caroxyrhodamine 6G cadaverine; 5(6)-Caroxyrhodamine 6G ethylenediamme, 5-ROX (5-Carboxy-X-rhodamine), 6-ROX (6-Carboxy-X-rhodamine); 5-TAMRA (5-Carboxytetramethylrhodamine), 6-TAMRA (6-Carboxytetramethylrhodamine), 5-TAMRA cadaverine, 6-TAMRA cadaverine, 5-TAMRA ethylenediamine (5-Carboxytetramethylrhodamine ethylenediamine), 6-TAMRA ethylenediamine (6-Carboxytetramethylrhodamine ethylenediamine) , 5-TMR C6 maleimide, 6-TMR C6 maleimide, TR C2 maleimide, TR cadaverine, 5-TRITC (Tetramethylrhodamine-5-(and-6)-isothiocyanate), 6-TRITC, R isomer (Tetramethylrhodamine-6-isothiocyanate), Dansyl cadaverine (5-Dimethylaminonaphthalene-1-(N-(5-aminopentyl))sulfonamide), EDANS C2 maleimide (5-((2-Aminoethyl)amino)naphthalene-1-sulfonic acid C2 maleimide), EDANS acid (5-((2-Aminoethyl)amino)naphthalene-1-sulfonic acid); fluorescamine (4-Phenylspiro-[furan-2(3*H*),1-phthalan]-3,3'-dion), NBD (4-Chlor-7-nitro-benzo-2-oxa-1,3-diazol), pyrromethene, Texas Red (1*H*,5*H*,11*H*,15*H*-Xantheno[2,3,4-*ij*:5,6,7-*i'j*']diquinolizin-18-ium, 9-[2(*or* 4)-(chlorosulfonyl)-4(or 2)-sulfophenyl]-2,3,6,7,12,13,16,17-octahydro-, inner salt), Cy5, Cy5 succinimidylester (3H-Indolium, 2-[5-[1-[6-[(2,5-dioxo-1-pyrrolidinyl)oxy]-6-oxohexyl]-1,3-dihydro-3,3-dimethyl-5-sulfo-2*H*-indol-2-ylidene]-1,3-pentadien-1-yl]-1-ethyl-3,3-dimethyl-5-sulfo-, inner salt), Cy5 sacridine orange, 2,7-dichlorofluorescein, eosin, rose bengal, 1,2-dihydroxyanthraquinone, 1,4-dihydroxyanthraquinone, 1,8-dihydroxyanthraquinone, 1,3,8-trihydroxy-6-ethylanthraquinone, 1,2,5,8-tetrahydroxyanthraquinone, 1-aminonaphthalene, 2-aminonaphthalen, indicyano green (ICG), IRDye800CW, Bodipy650-X, CF680R, 580CP-methocy, 610CP, SiR-methyl, ATTO 390, ATTO 425, ATTO 465, ATTO 488, ATTO 495, ATTO 514, ATTO 520, ATTO 532, ATTO Rho6G, ATTO 550, ATTO 565, ATTO Rho3B, ATTO Rho11, ATTO Rho12, ATTO Thio12, ATTO Rho101, ATTO 590, ATTO Rho13, ATTO 594, ATTO 610, ATTO 620, ATTO Rho14, ATTO 633, ATTO 647, ATTO 647N, ATTO 655, ATTO Oxa12, ATTO 665, ATTO 680, ATTO 700, ATTO 725, ATTO647N, STAR 600, STAR635 P, STAR RED, 580CP-methoxy and derivatives thereof.

In particular, building block A and/or building block B further comprises a fluorescein group, in particular, building block A and/or building block B comprise a labeled nucleotide, wherein the labeling group is a fluorescent group, more preferably fluorescein, and wherein the labeled nucleotide is most preferably a fluorescein-labeled deoxy-thymidin. Preferably, the fluorescein-labeled deoxy-thymidin has the structure:

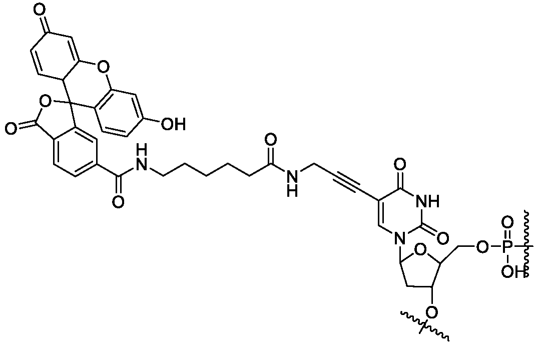

### Building block A comprises the Labeling Group

According to a first preferred embodiment of the invention thus, building block A further comprises the labeling group, preferably the fluorescent group, more preferably the fluorescein group. In particular, the building block A further comprises a single stranded polynucleotide segment, comprising a nucleotide with a base being substituted with a labeling group (labeled nucleotide), preferably a fluorescent group, more preferably wherein the fluorescent group is fluorescein. In particular, the labeled nucleotide is a fluorescein-labeled deoxy-thymidin as specified herein above.

Preferably, the building block A further comprises a linker group comprising ethoxyethyl groups.

Preferably, the building block A has the structure (A1)

FG^{a}-L²-(X)₁₋₅-(dT(Fluorescein))-(X)₃₋₁₀-L¹-(X)₁₋₅-(Pur) (A1)

wherein each X is independently selected from A, T, G, and C, wherein L1 and L2 are linker, and wherein dT(Fluorescein) is fluorescein-labeled deoxy-thymidin, as specified herein above.

Linker L¹ is a linking moiety. In general, there are no particular restrictions as to the chemical nature of the linking moiety L¹ with the proviso it provides suitable chemical properties for the building block A and its intended use. Thus, the term Linker L¹ refers any suitable chemical moiety linking the respective nucleotides X and typically includes moieties comprising groups selected ffrom the group consisting of alkoxyalkyl, alkyl, alkenyl, alkynyl, alkylaryl, arylalkyl, aryl, alkylarylalkyl, heteroaryl, alkylheteroaryl, alkylheteroalkyl, heteroarylalkyl, cycloalkyl. Preferably, L¹ comprises alkoxyalkyl groups, more preferably -[CH2-CH2-O]- groups. Further, the L¹ is preferably attached to the nucleotide via phosphate groups.

Preferably, L¹ is a linker having the structure -P(=O)(-OH)-O-[CH₂-CH₂-O]n_{L1}--P(=O)(-OH)-O-[CH₂-CH₂-O]m_{L1}- wherein n_{L1} and m_{L1} are integers in the range of from 2 to 15, preferably n_{L1} is 5-7, such as 6, and m_{L1} is 5-7, such as 6.

In particular, L¹ has the structure

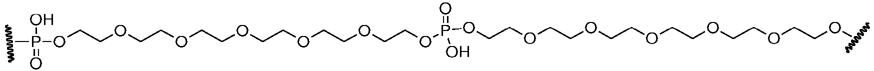

Linker L² is a linking moiety. In general, there are no particular restrictions as to the chemical nature of the linking moiety L² with the proviso it provides suitable chemical properties for the building block A and its intended use. Thus, the term Linker L² refers any suitable chemical moiety linking the respective nucleotides X and FG^{a} and typically includes moieties comprising groups selected from the group consisting of alkoxyalkyl, alkyl, alkenyl, alkynyl, alkylaryl, arylalkyl, aryl, alkylarylalkyl, heteroaryl, alkylheteroaryl, alkylheteroalkyl, heteroarylalkyl, cycloalkyl. Preferably, L² comprises alkoxyalkyl groups and alkyl groups.

In particular, L² has the structure:

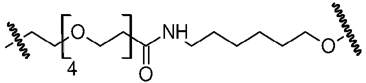

Preferably, the building block A has the structure (A2)

FG^{a}-L²-TC-(dT(Fluorescein))-CTC-L¹-CC-(Pur) (A2)

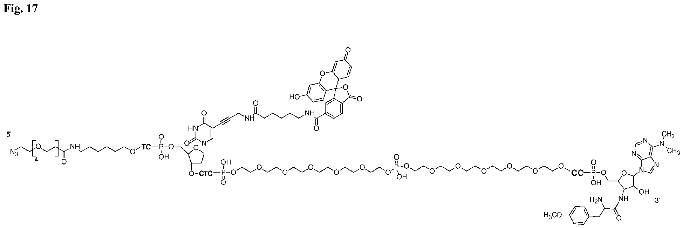

in particular, the structure (A3), as shown in Fig. 17, and below:

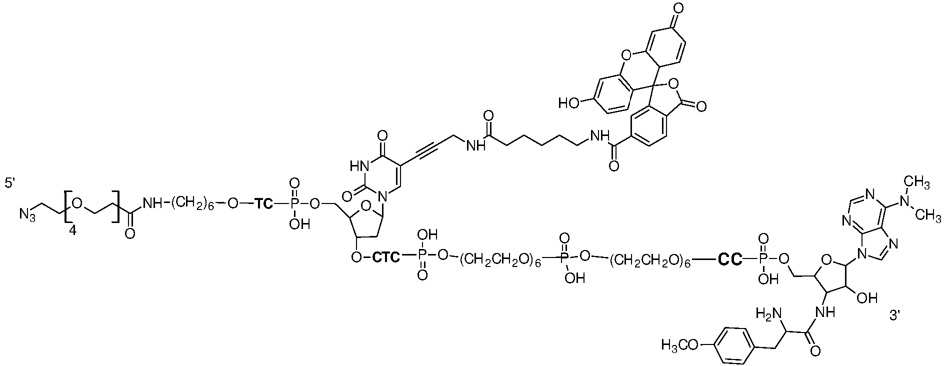

According to this first preferred embodiment of the invention, the building block B, the building block B has the structure (B1)

Z-(X)₂₋₅-rG-(X)₂₋₁₀-(CnVK)-(X)₅₋₁₅-X(L³-(FG^{b}))-(X)₃₋₅ (B1)

wherein each X is independently selected from A, T, G, and C, wherein rG is ribo-guanosine, and A, T, G, an C are deoxynucleotides and wherein L³ is a linker and CnVK is a cyanovinylcarbazol group, and wherein Z is dT-Biotin or a group having the structure:

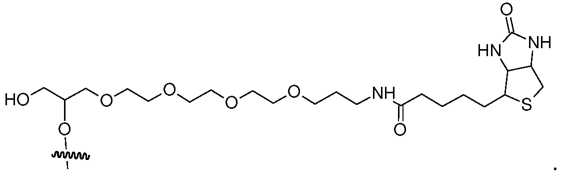

Linker L³ is a linking moiety. In general, there are no particular restrictions as to the chemical nature of the linking moiety L³ with the proviso it provides suitable chemical properties for the building block A and its intended use. Thus, the term Linker L³ refers any suitable chemical moiety linking the respective nucleotides X and typically includes moieties comprising groups selected from the group consisting of alkoxyalkyl, alkyl, alkenyl, alkynyl, alkylaryl, arylalkyl, aryl, alkylarylalkyl, heteroaryl, alkylheteroaryl, alkylheteroalkyl, heteroarylalkyl, cycloalkyl and alkyl groups.

X(L³-(FG^{b}) is a deoxynucleotide, such as A, T, G or C, being substituted with the moiety L³-FG^{b} at the base. Preferably, X(L³-(FG^{b}) has the structure:

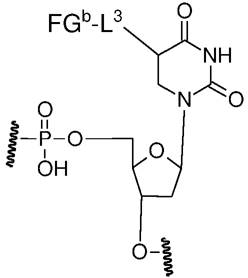

Preferably, L³ comprises alkoxyalkyl groups or alkyl groups. More preferably L3 has the structure: 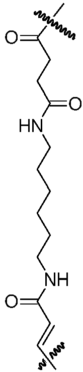 Preferably, (L³-(FG^{b}) has the structure: 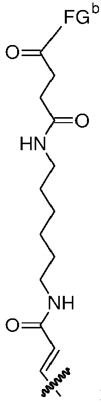 in particular

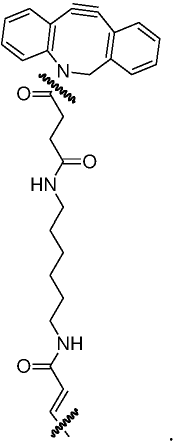

More preferably, the building block B has the structure (B2)
Z-AA-rG-AATTTCCA-(CnVK)-GCCGCCCCCCG-T(L³-(FG^{b}))-CCT (B2, SEQ ID NO:7)
wherein L³ is the linker, CnVK is a cyanovinylcarbazol group, rG is ribo-guanosine, and A, T, G, an C are deoxynucleotides, and wherein Z is dT-Biotin or a group having the structure: 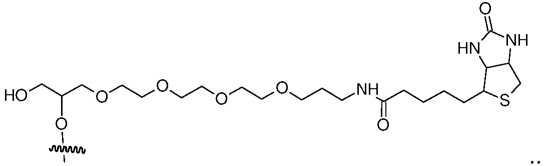 preferably dT(biotin).

In particular, the building block B has the structure (B3) describe above and as shown in Fig. 18 or the structure (B'3), as described above, and as shown in Fig. 18a.

Upon reaction of the building block A with the building block B, the Puromycin linker is formed.

As described above, upon reaction, the functional group FG^{a} and the functional group FG^{b} react with each other, thereby forming the linking group a'-b'.

In case, the building block A has the structure (A1), described above and below, and the building block B has the structure (B1) described above and below, preferably, the resulting Puromycin Linker (I) has the structure (Ia) 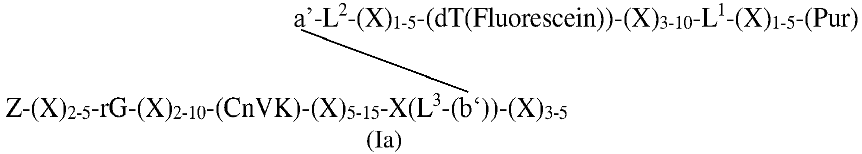 wherein each X is independently selected from A, T, rG, G, and C, wherein rG is ribo-guanosine, and A, T, G, an C are deoxynucleotides and wherein L³ is a linker and CnVK is a cyanovinylcarbazol group, and wherein Z is dT-Biotin or a linker-biotinyl group as described herein above, and wherein L1 and L2 are linkers, and wherein dT(Fluorescein) is fluorescein-labeled deoxy-thymidin and preferably having the structure as specified herein above and a'-b' is the linking group which is formed upon reaction of the functional group FG^{a} with the functional group FG^{b}, the linking group preferably comprising a triazole, more preferably wherein the linking group a'-b' is

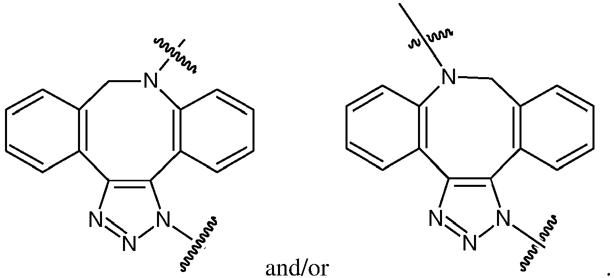

Preferably, the Puromycin Linker (I) has the structure (Iaa): 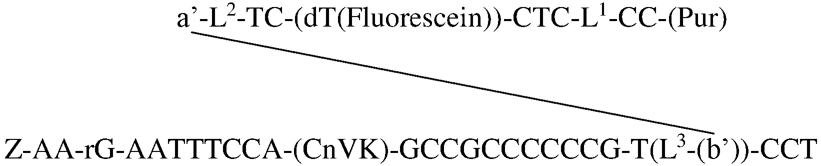 more preferably, the structure (Iaaa) and/or the structure (Iaaa*):

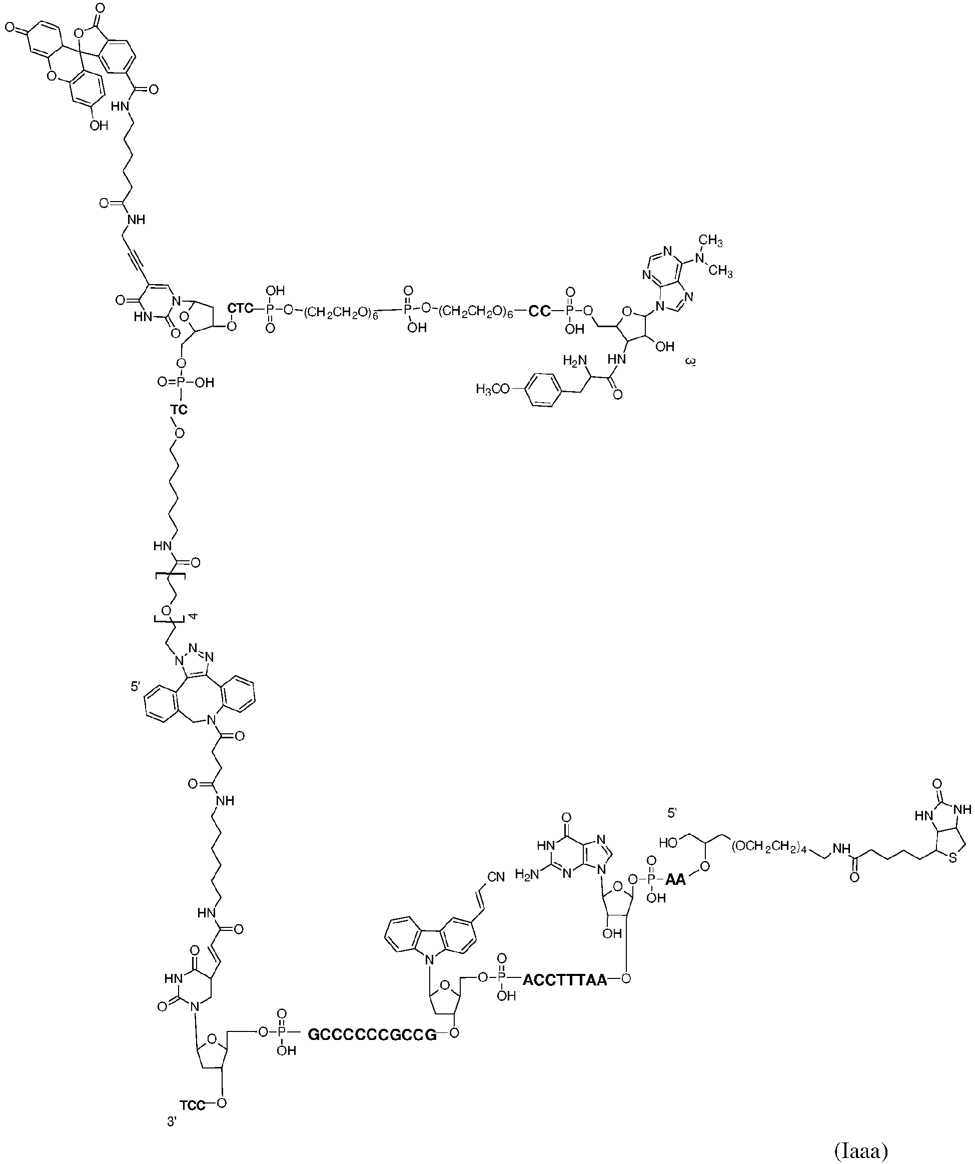

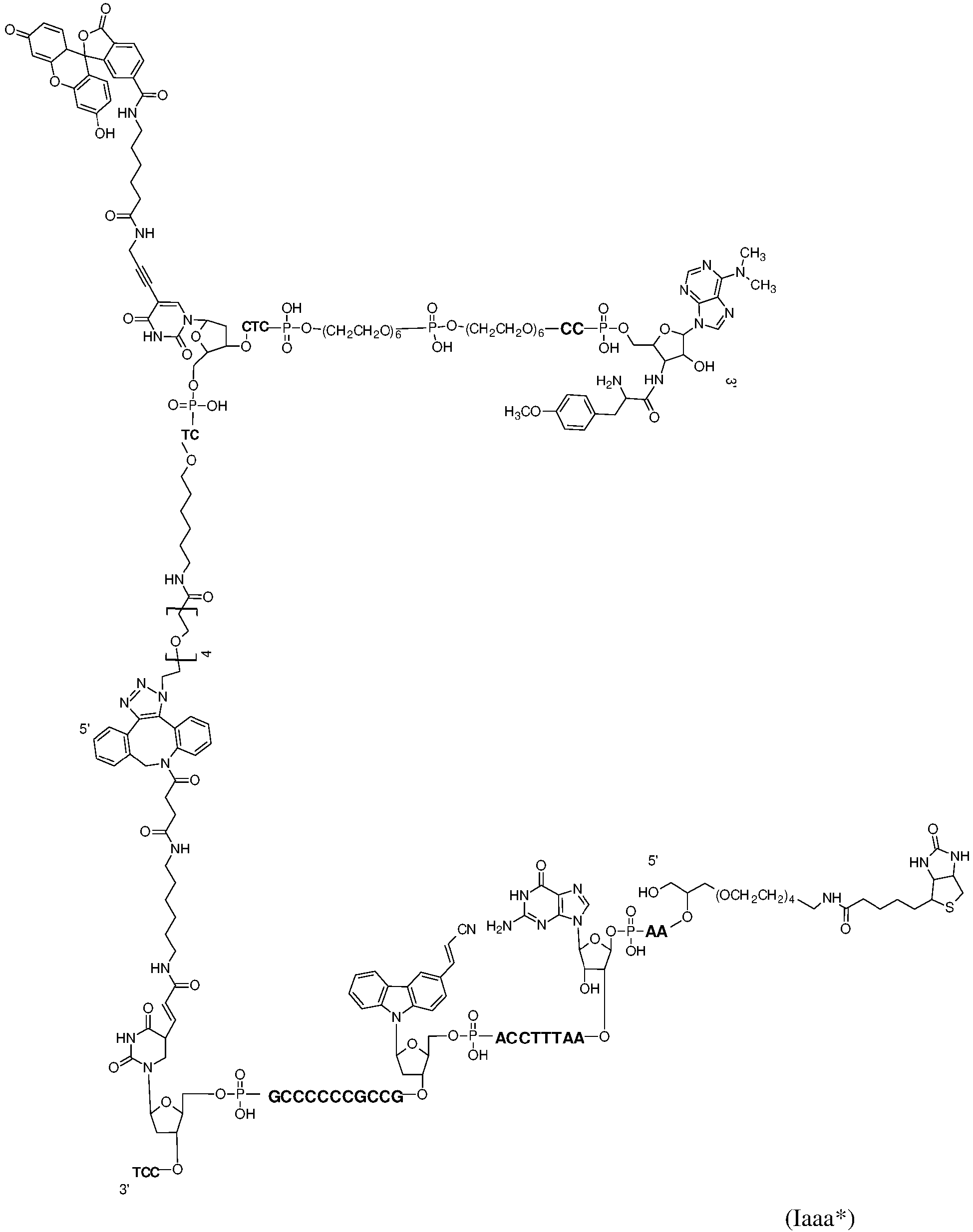

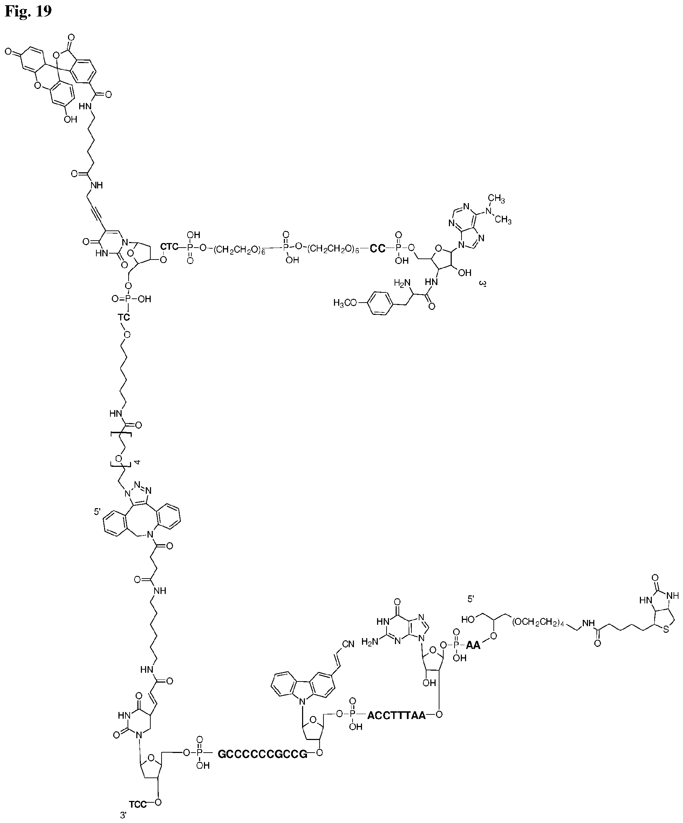

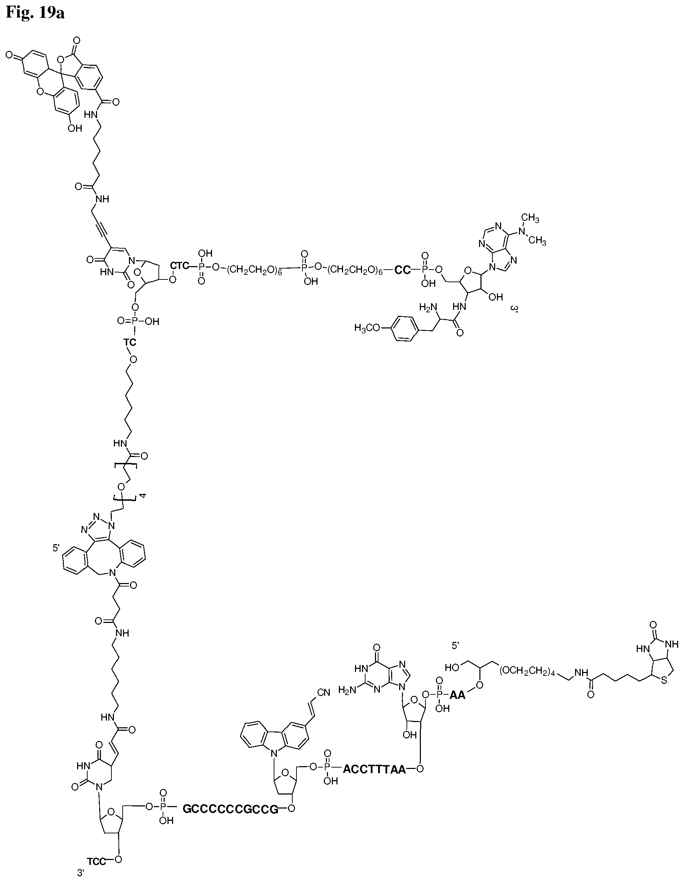

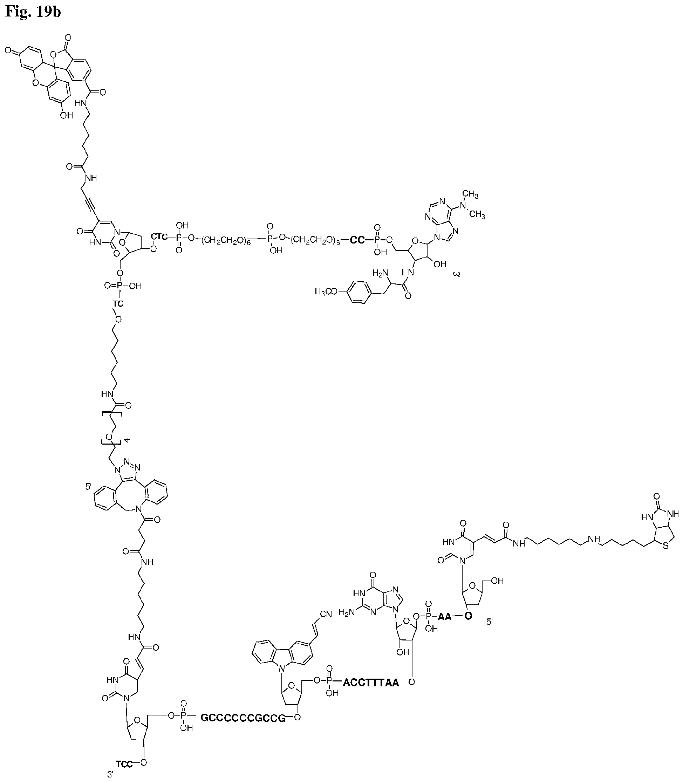

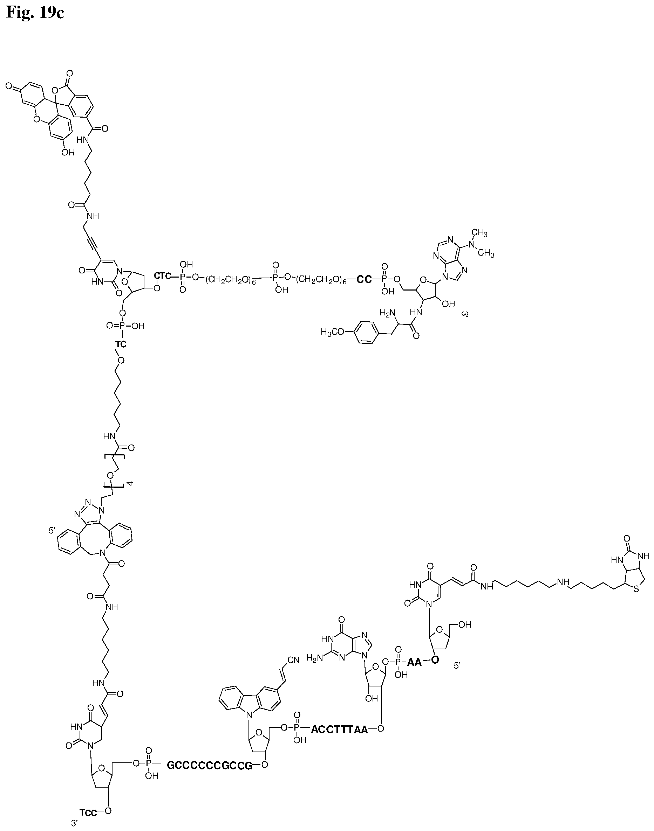

Preferably, the Puromycin Linker (I) has the structure (Iaaa) as shown in Fig. 19 and/or the Fig. (Iaaa*) Fig 19a.

It is thus to be understood that the Puromycin Linker (I) may be a mixture of (Iaaa) and (Iaaa*).

According to an alternative preferred embodiment, the structure (I'aaa) and/or the structure (I'aaa*):

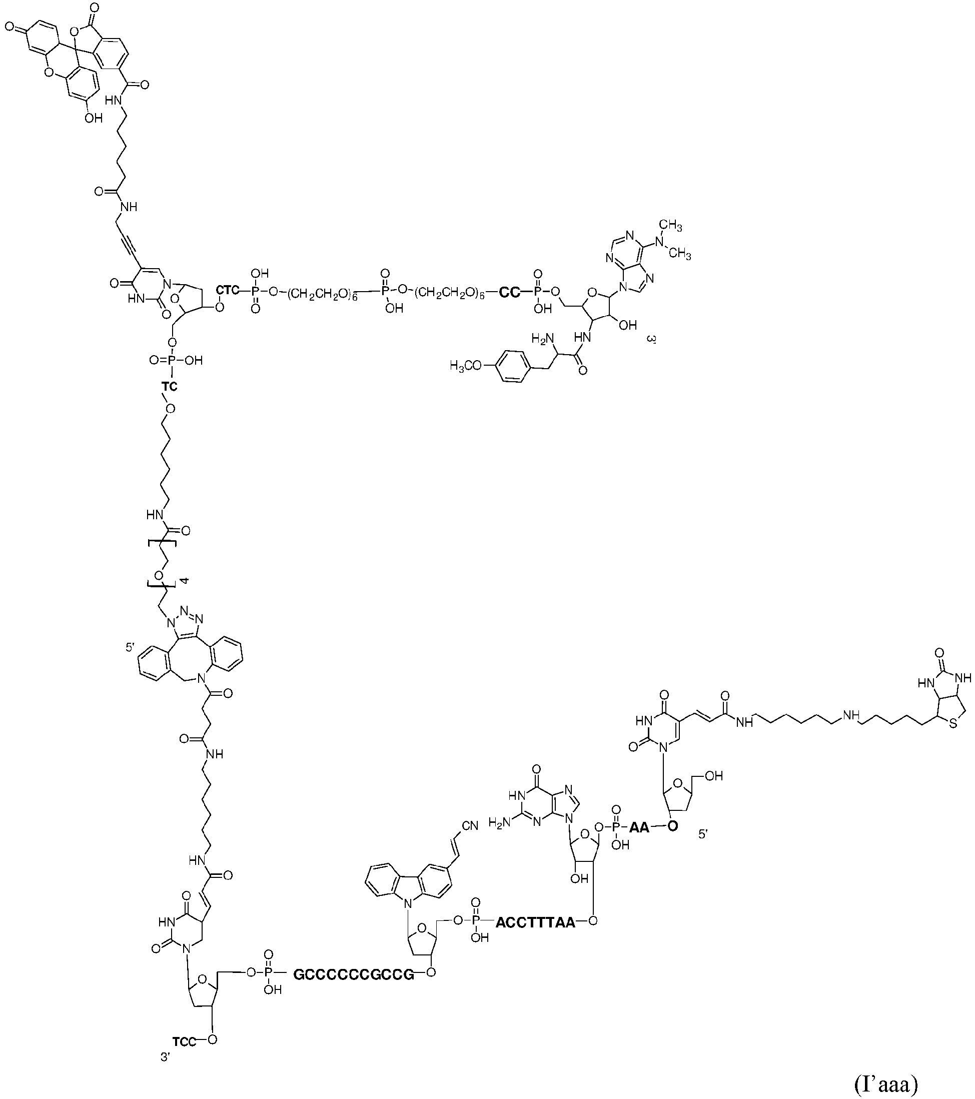

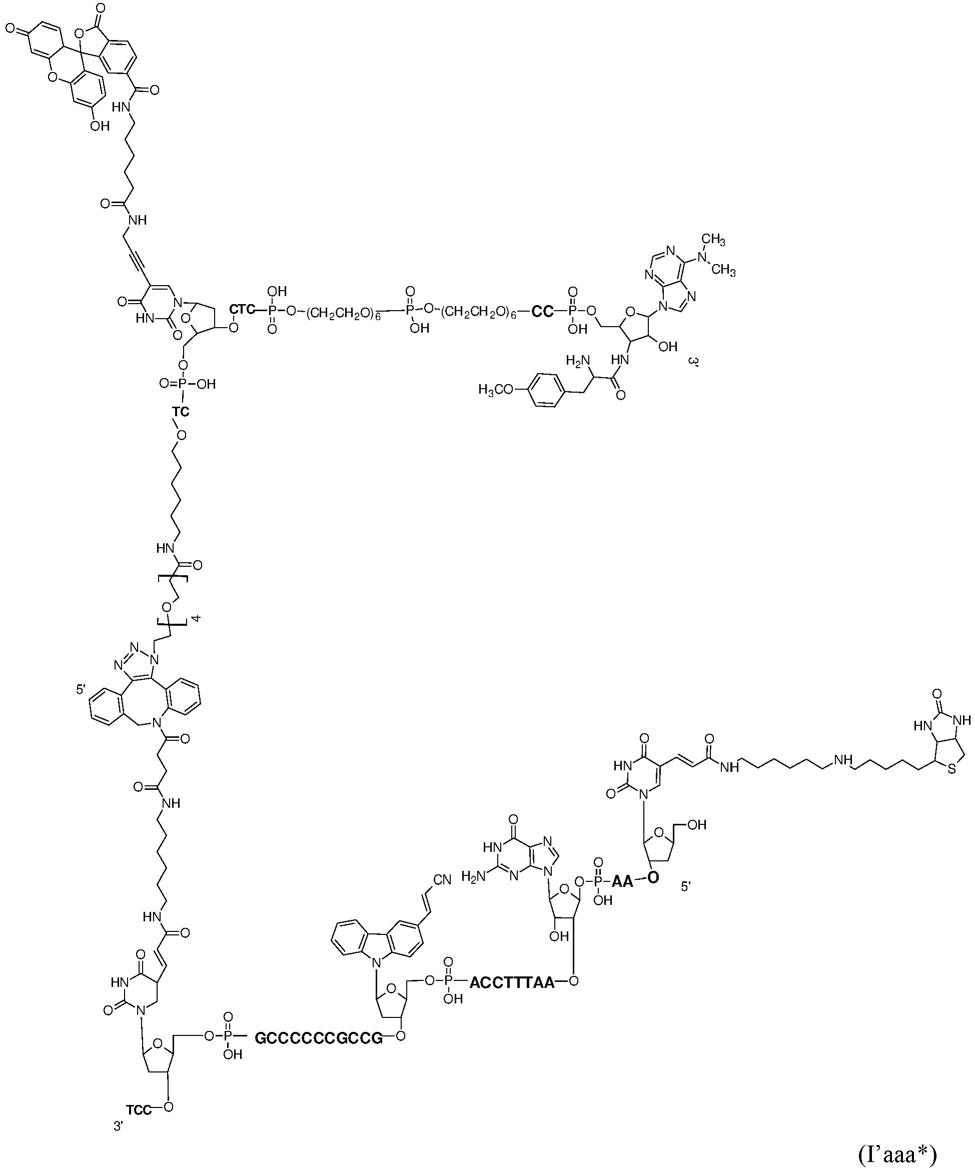

According to this embodiment, the Puromycin Linker (I) has preferably the structure (I'aaa) as shown in Fig. 19b and/or the (I'aaa*) as shown in Fig 19c.

It is thus to be understood that the Puromycin Linker (I) may be a mixture of (I'aaa) and (I'aaa*).

### Building block B comprises a Labeling Group

According to a second preferred embodiment of the invention building block B further comprises a labeling group, preferably the fluorescent group, more preferably the fluorescein group.

In particular, the building block B further comprises a single stranded polynucleotide segment, comprising a nucleotide with a base being substituted with a labeling group (labeled nucleotide), preferably a fluorescent group, more preferably wherein the fluorescent group is fluorescein. In particular, the labeled nucleotide is a fluorescein-labeled deoxy-thymidin and preferably Fluorescein(dT) as escribed above and below.

According to this second preferred embodiment, the building block B preferably has the structure

Z-(X)₂₋₅-rG-(X)₂₋₅-(dT(Fluorecein))-(X)₂₋₁₀-(CnVK)-(X)₅₋₁₅-X(L3-(FG^{b}))-(X)₃₋₅, (2B1)

wherein each X is independently selected from A, T, G, and C, wherein rG is ribo-guanosine, and A, T, G, an C are deoxynucleotides, and wherein L³ is a linker and CnVK is a cyanovinylcarbazol group.

Linker L³ is a linking moiety and has already been described above. As described above, further X(L³-(FG^{b}) is a deoxynucleotide, such as A, T, G or C, being substituted with the moiety L³-FG^{b} at the base. Preferably, X(L³-(FG^{b}) has the structure:

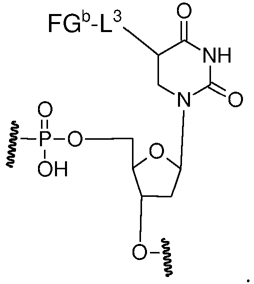

Preferably, L³ comprises alkoxyalkyl groups or alkyl groups. More preferably L3 has the structure:

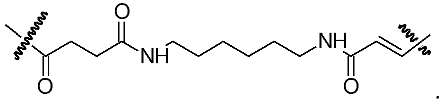

Preferably, (L³-(FG^{b}) has the structure: 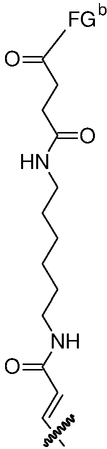 in particular

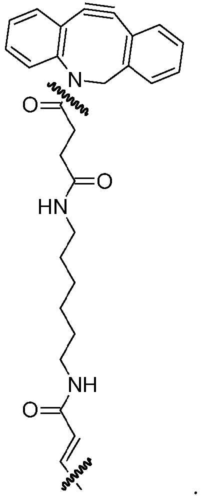

According to this second preferred embodiment, the building block B has the structure (2B2):

Z-AA-rG-AA-(dT(Fluorescein)-TTCCA-(CnVK)-GCCGCCCCCCG-T(L3-(FG^{b}))-CCT (2B2)

More preferably the structure (2B3), as also shown in Fig. 20: 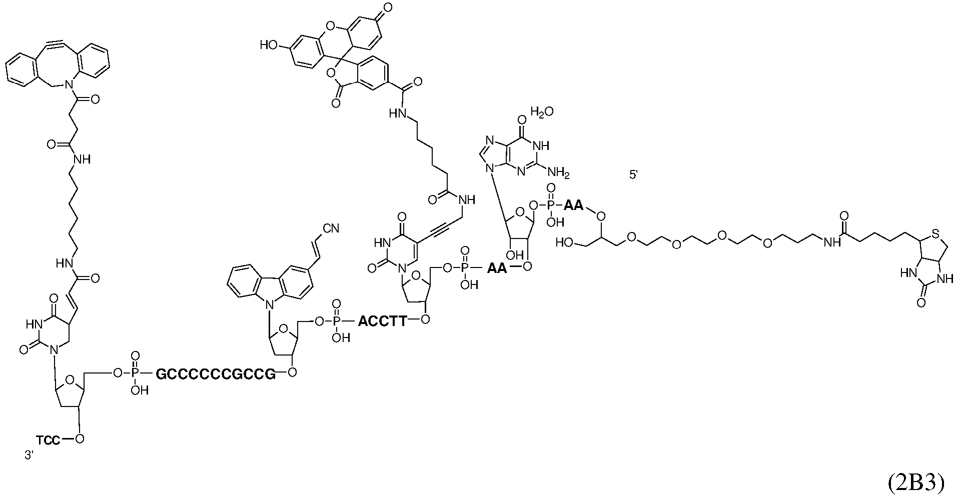 or the structure (2B'3),

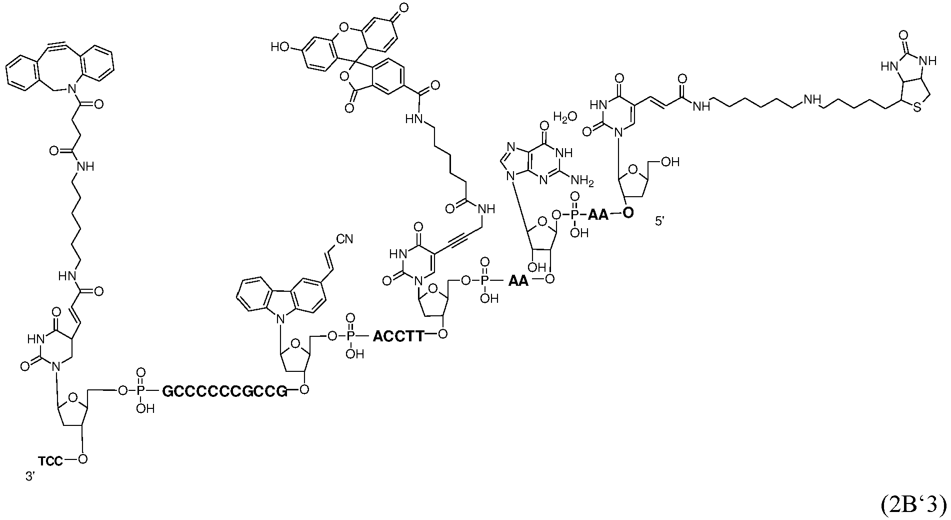

According to this second preferred embodiment, the building block A further preferably comprises a linker group comprising ethoxyethyl groups.

Preferably, the building block A has the structure (2A1):

FG^{a}-L²-(X)₄₋₁₅-L¹-(X)₁₋₅-(Pur) (2A1)

wherein each X is independently selected from A, T, G, and C, wherein L¹ and L² are linker.

Linker L¹ has already been described above in the context of the first preferred embodiment. Thus, there are in general, no particular restrictions as to the chemical nature of the linking moiety L¹ with the proviso it provides suitable chemical properties for the building block A and its intended use. Thus, the term Linker L¹ refers any suitable chemical moiety linking the respective nucleotides X and typically includes moieties comprising groups selected ffrom the group consisting of alkoxyalkyl, alkyl, alkenyl, alkynyl, alkylaryl, arylalkyl, aryl, alkylarylalkyl, heteroaryl, alkylheteroaryl, alkylheteroalkyl, heteroarylalkyl, cycloalkyl. Preferably, L¹ comprises alkoxyalkyl groups, more preferably -[CH2-CH2-O]- groups. Further, the L¹ is preferably attached to the nucleotide via phosphate groups. Preferably, L¹ is a linker having the structure -P(=O)(-OH)-O-[CH₂-CH₂-O]n_{L1}--P(=O)(-OH)-O-[CH₂-CH₂-O]m_{L1}-wherein n_{L1} and m_{L1} are integers in the range of from 2 to 15, preferably n_{L1} is 5-7, such as 6, and m_{L1} is 5-7, such as 6. In particular, L¹ has the structure

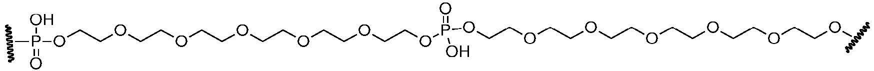

Linker L² and has already been described above in the context of the first preferred embodiment. In general, there are no particular restrictions as to the chemical nature of the linking moiety L² with the proviso it provides suitable chemical properties for the building block A and its intended use. Thus, the term Linker L² refers any suitable chemical moiety linking the respective nucleotides X and FG^{a} and typically includes moieties comprising groups selected from the group consisting of alkoxyalkyl, alkyl, alkenyl, alkynyl, alkylaryl, arylalkyl, aryl, alkylarylalkyl, heteroaryl, alkylheteroaryl, alkylheteroalkyl, heteroarylalkyl, cycloalkyl. Preferably, L² comprises alkoxyalkyl groups and alkyl groups. In particular, L² has the structure:

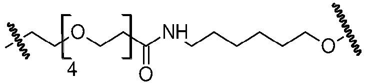

Preferably, the building block A has the structure (2A2)

FG^{a}-L²-TCTCTC-L¹-CC-(Pur) (2A2)

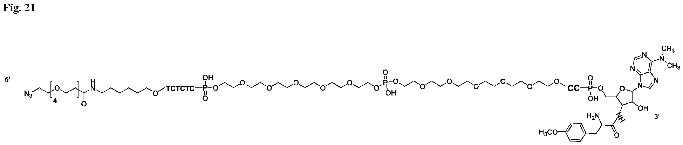

More preferably, the structure (2A3), as shown below and as shown in Fig. 21

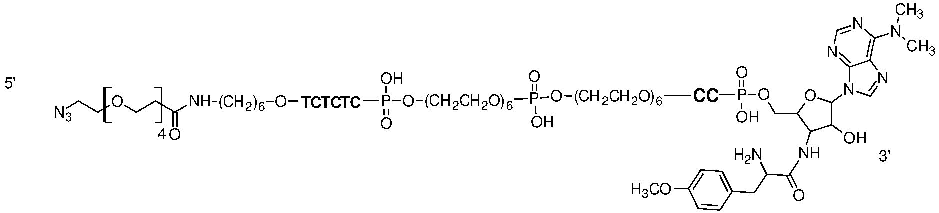

Upon reaction of the building block A with the building block B, the Puromycin linker is formed. As described above, upon reaction, the functional group FG^{a} and the functional group FG^{b} react with each other, thereby forming the linking group a'-b'. In case, the building block A has the structure (2A1), described above and below, and the building block B has the structure (2B1) described above and below, preferably, the resulting Puromycin Linker (I) has the structure (Ib) 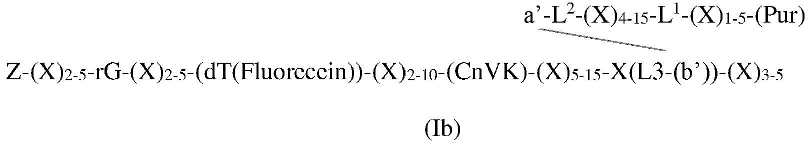 wherein each X is independently selected from A, T, G, and C, wherein L¹ and L² are linker, wherein rG is ribo-guanosine, wherein A, T, G, an C are deoxynucleotides, and wherein L³ is a linker and CnVK is a cyanovinylcarbazol group, and wherein Z is dT-Biotin or a group having the structure: 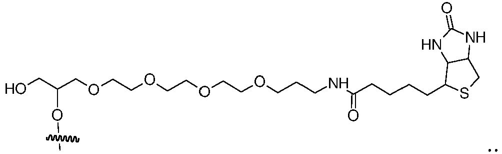 and wherein dT(Fluorescein) is as described above, and a'-b' is the linking group which is formed upon reaction of the functional group FG^{a} with the functional group FG^{b}, wherein the linking group a'-b' is

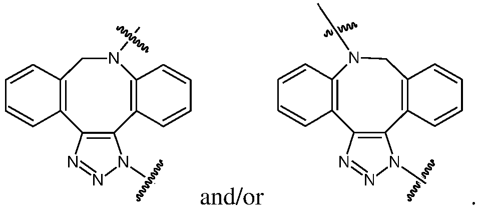

In case, the building block A has the structure (A2), described above and below, and the building block B has the structure (B2) described above and below, preferably, the resulting Puromycin Linker (I) has the structure (Ib)

Preferably, the Puromycin Linker (I) has the structure (Ibb): 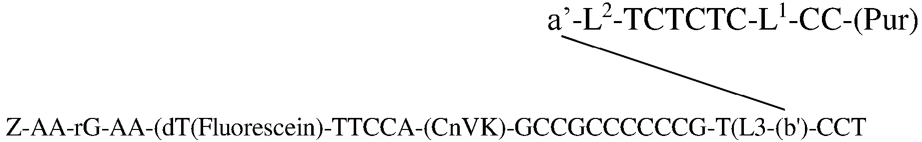 more preferably the structure (Ibbb) and/or (Ibbb*):

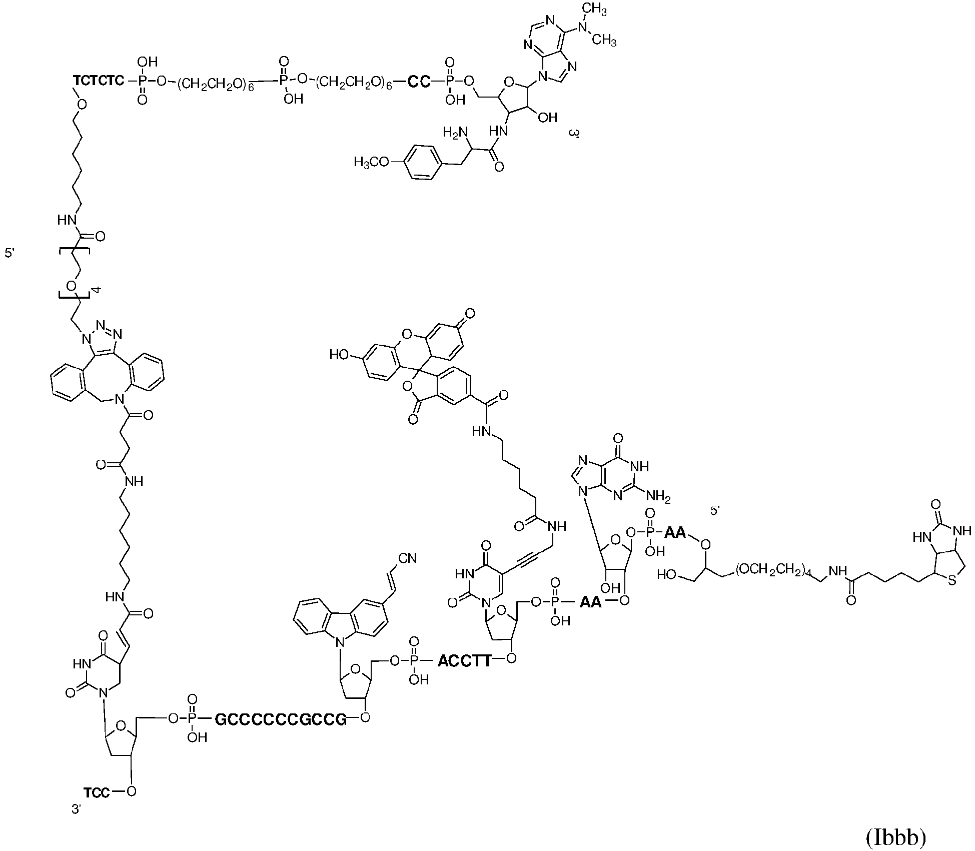

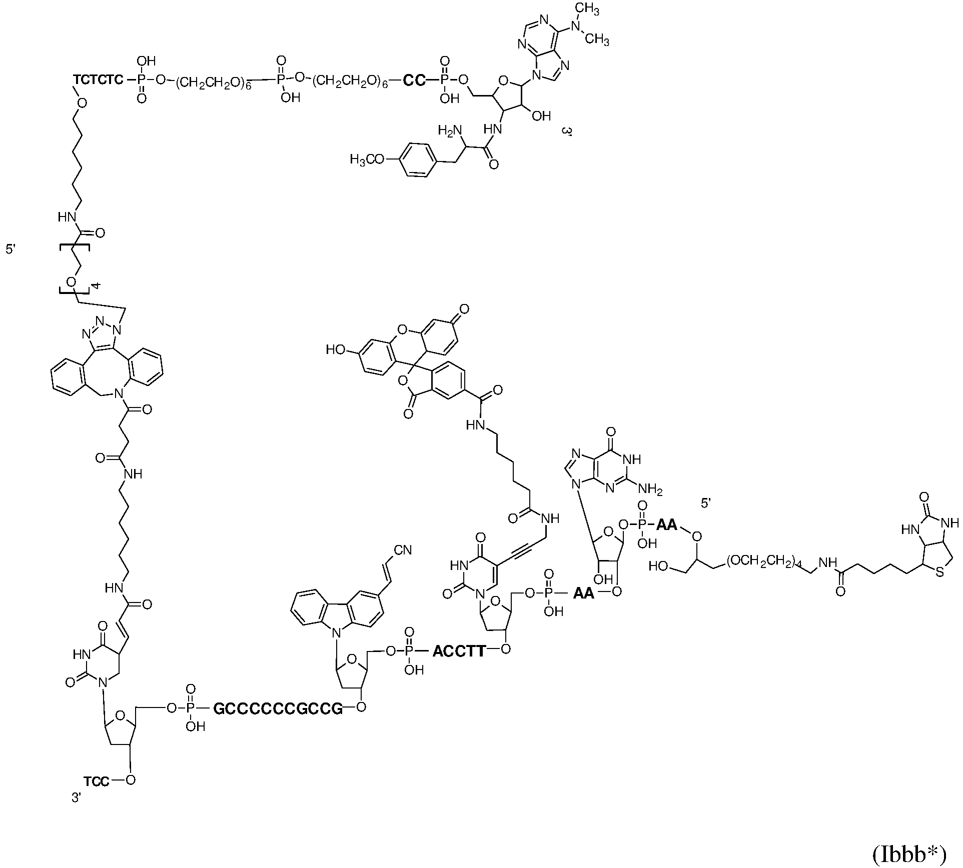

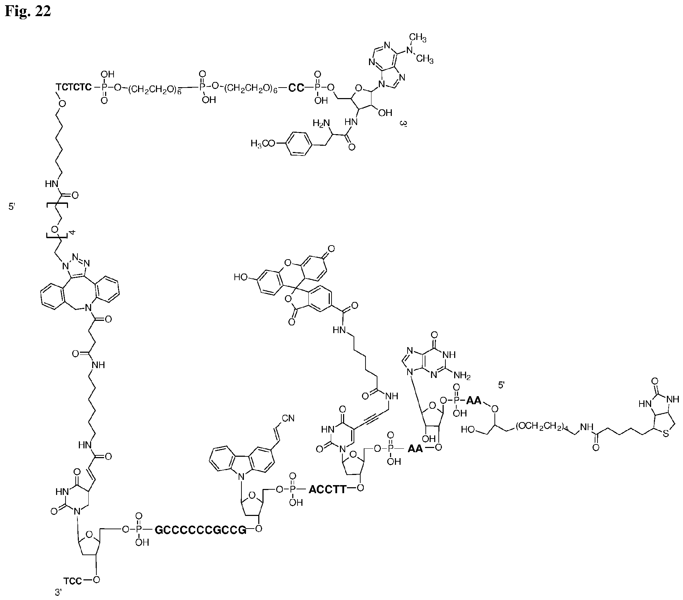

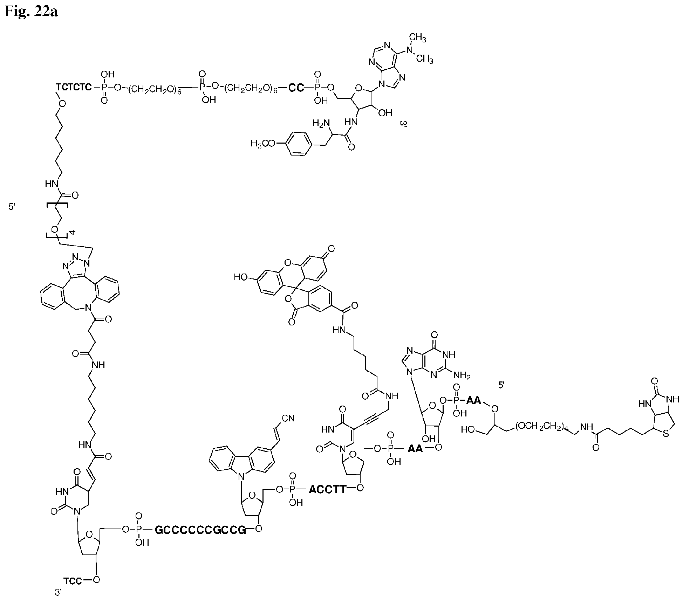

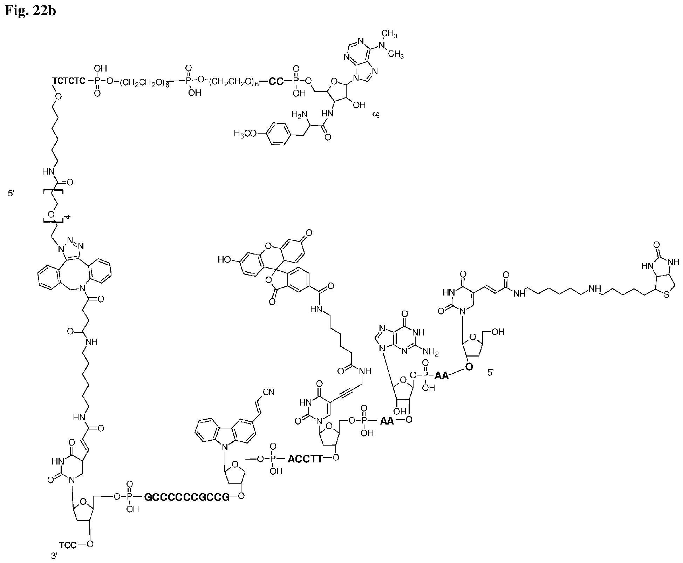

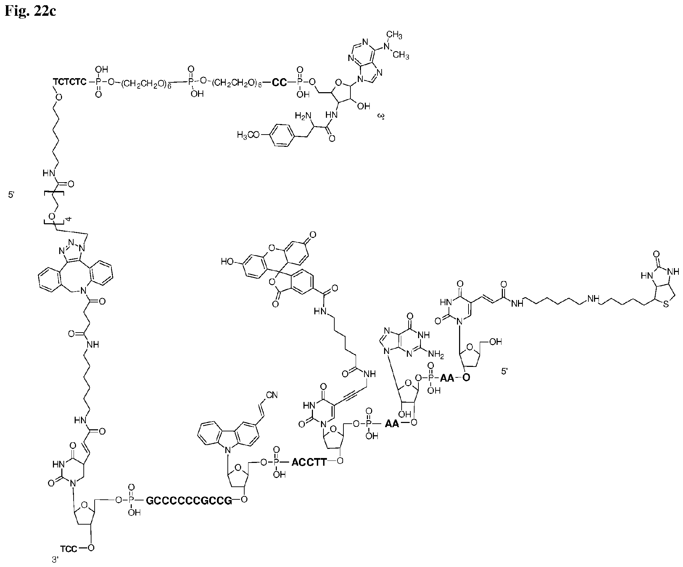

Preferably, the Puromycin Linker (I) has the structure (Ibbb) as shown in Fig. 22 and/or the Fig. (Ibbb*) Fig 22a.

It is thus to be understood that the Puromycin Linker (I) may be a mixture of (Ibbb) and (Ibbb*).

According to an alternative preferred embodiment, Puromycin Linker (I) has preferably the structure (I'bbb) and/or (I'bbb*):

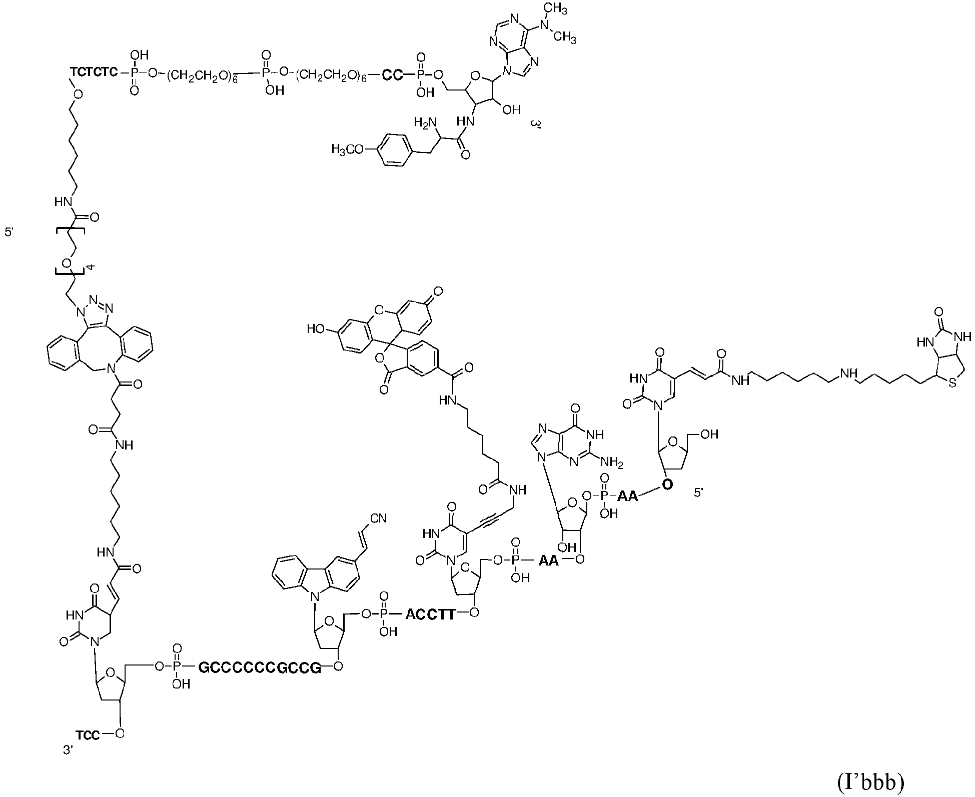

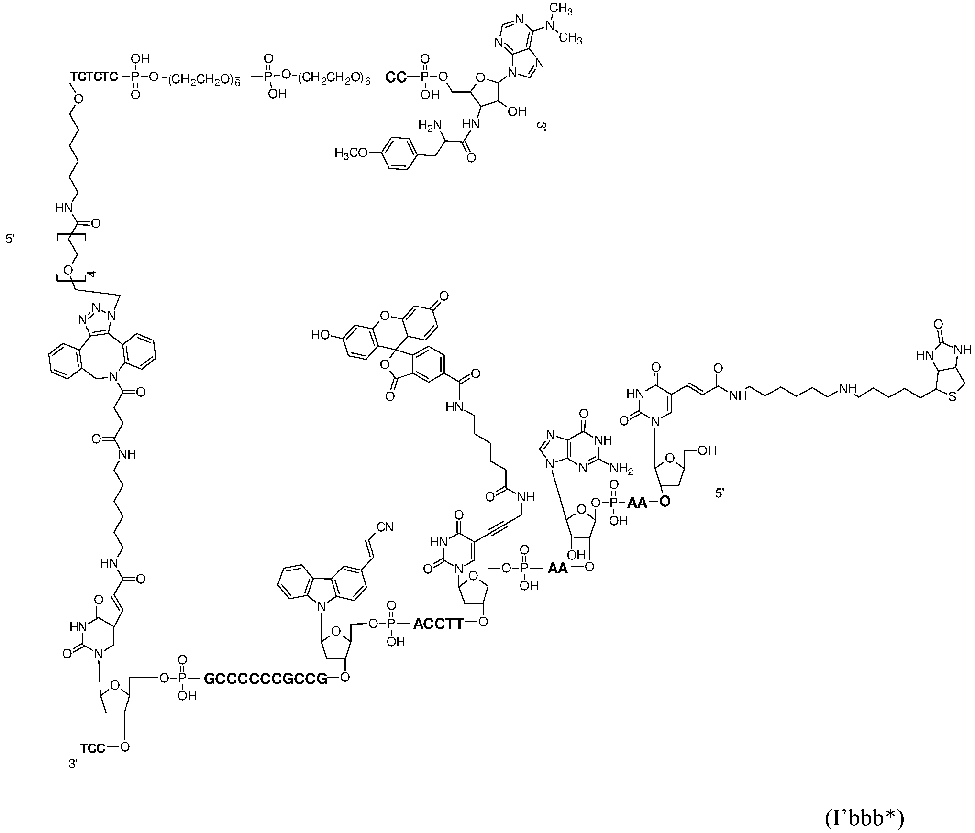

Preferably, the Puromycin Linker (I) has the structure (I'bbb) as shown in Fig. 22b and/or the Fig. (I'bbb*) Fig 22c.

It is thus to be understood that the Puromycin Linker (I) may be a mixture of (I'bbb) and (I'bbb*).

The definitions made above apply mutatis mutandis to the following. Additional definitions and explanations made further below also apply for all embodiments described in this specification mutatis mutandis.

The present invention also relates to a method for producing a covalent mRNA-Puromycin Linker conjugate, said method comprising the steps of the method (P) for preparing a puromycin linker as specified herein above and the further steps
(a) contacting the Puromycin Linker produced in the preceding steps with an mRNA;
(b) photocrosslinking said mRNA and said Puromycin Linker, and thereby
(c) producing a covalent mRNA-Puromcyin Linker conjugate.

The term "mRNA" is used herein in its usual meaning known to the skilled person. The mRNA as referred to herein comprises at least a coding sequence, preferably lacking a stop codon, and encoding a peptide sequence comprising at least five amino acids, preferably at least seven amino acids, more preferably at least 9 amino acids, and at least one nucleic acid sequence essentially reverse complementary to the single-stranded polynucleotide segment of building block B; thus, the mRNA encodes a peptide as specified and hybridizes to the puromycin linker under appropriate conditions known to the skilled person. Further structural elements and the specific structure of the mRNA is selected by the skilled person depending on the planned further use of the covalent mRNA-Puromycin Linker conjugate, in particular in case the covalent mRNA-Puromycin Linker conjugate is used in translation. Thus, in case the covalent mRNA-Puromycin Linker conjugate is used in a bacterial translation system, including cell-free translation systems derived from bacteria, the mRNA may in particular further comprise a 5'-untranslated region including a Shine-Dalgarno sequence and/or a 3' untranslated region. In case the covalent mRNA-Puromycin Linker conjugate is used in a eukaryotic translation system, including cell-free translation systems derived from eukaryotic cells, the mRNA may in particular comprise a 5'-untranslated region including at least one binding site(s) for translation initiation factors, a 3' untranslated region, and/or a poly-A tail.

As referred to herein, the terms mRNA and covalent mRNA-Puromycin Linker conjugate may relate to single molecular species of the respective items. The terms, however, preferably relate to a multitude of molecular species, wherein said molecular species may be identical, however more preferably differ in their nucleic acid sequence. Thus, the mRNA preferably is a pool of mRNAs, and/or the covalent mRNA-Puromycin Linker conjugate preferably is a pool of covalent mRNA-Puromycin Linker conjugates, wherein the molecular species preferably only differ in the sequence encoding a peptide comprised in the mRNA. In accordance, the mRNA preferably is a pool of mRNAs comprising the same nucleic acid sequenceessentially reverse complementary to the single-stranded polynucleotide segment of building block B and a plurality of non-identical coding sequences encoding a peptide as specified herein above. Thus, the mRNA preferably is a library of mRNAs hybridizing to the single-stranded polynucleotide segment of building block B and carrying a variety of coding sequences. Principal method for constructing such libraries, in particular for use with mRNA and cDNA display methods, are known in the art. The aforesaid applies mutatis mutandis to the mRNA comprised in the covalent mRNA-Puromycin Linker conjugate; in particular, also the covalent mRNA-Puromycin Linker conjugate may be a library as specified herein above.

Steps (a) to (c) are in principle known to the skilled person from protocols for mRNA or cDNA display methods known in the art, e.g. from Arai et al (2019), loc. cit. The method for producing an mRNA-Puromycin Linker conjugate is an in vitro method.

The aforesaid steps known from display methods known in the art typically do not require modification when used in the context of the instant method for producing a covalent mRNA-Puromycin Linker conjugate, except for an optional reduction of the amount of puromycin linker used. As the skilled person will understand in view of the description herein, synthesis yields in the method for producing a covalent mRNA-Puromycin Linker conjugate are much higher compared to known synthesis methods; in accordance, the product as obtained in synthesis is more pure and can be used in lower amounts to provide the same molar amount of puromycin linker and/or can be used without purification.

Preferably, contacting step (a) is performed immediately after said preparing a Puromycin Linker, i.e., more preferably is performed using the product as it is obtained in the method for preparing a Puromycin Linker, most preferably without purifying said Puromycin Linker before step (a).

The present invention also relates to a method for producing a display complex, the method comprising the steps of
(I) providing a covalent mRNA-Puromycin Linker conjugate;
(II) contacting the covalent mRNA-Puromcyin linker conjugate of step (I) with an in vitro translation composition to produce a display complex comprising a polypeptide encoded by said mRNA,
(III) optionally reverse transcribing said mRNA to a cDNA, and optionally
(IV) cyclisation of the polypeptide comprised in said display complex,
thereby preferably producing a display complex, preferably a cDNA display complex.

The term "polypeptide" is understood by the skilled person and preferably refers to a molecule consisting of a multitude of amino acids that are covalently linked to each other by peptide bonds. Polypeptides consisting of less than 20 amino acids covalently linked by peptide bonds may also be referred to as "peptides". Preferably, the polypeptide comprises of from 5 to 1000, more preferably of from 10 to 500, still more preferably of from 15 to 250, most preferably of from 20 to 200 amino acids.

Preferably, the polypeptide comprises at least one cysteine moiety, more preferably within 2, preferably within 3 amino acids, more preferably within 4, more preferably within 5 amino acids, from the N or C terminus, preferably the N terminus of the polypeptide. Preferably, the polypeptide comprises at least 2, preferably at least 3, in particular 3, cysteine moieties. Preferably between each cysteine present in the polypeptide, the polypeptide comprises at least 4, preferably at least 5, such 5 or 6, amino acid residues.

Preferably, the polypeptide comprises the sequence -Cys-(X^{aa1})ₓ₁-Cys-(X^{aa2})ₓ₂-Cys-, wherein x1 is an integer in the range of from 4 to 7, preferably 5 or 6, more preferably 5, and wherein x2 is an integer in the range of from 4 to 7, preferably 5 or 6, more preferably 6, and wherein X^{aa1} are amino acid residues, preferably natural amino acid residues, wherein each X^{aa1} residue may be the same or may be different, and wherein each X^{aa2} are amino acid residues, preferably natural amino acid residues, wherein each X^{aa2} residue may be the same or may be different. It is to be understood that the term natural amino acid residues refers to naturally-occurring amino acids incorporated in the polypeptide sequence and includes all naturally occurring amino acids.

Preferably, the polypeptide comprises a histidine-tag. A histidine-tag is denoted to mean an amino acid motif typically consisting of of at least six histidine residues, in particular 6 histidine residues.

Preferably, the polypeptide comprises the sequence -Cys-(X^{aa1})ₓ₁-Cys-(X^{aa2})ₓ₂-Cys-(X^{aa3})ₓ₃-(His)₆, wherein x3 is an integer in the range of from 2 to 10, preferably 3 or 8, preferably 3 or 4, more preferably 3, and wherein X^{aa3} are amino acid residues, preferably natural amino acid residues, wherein each X^{aa3} residue may be the same or may be different, preferably (X^{aa3})x₃ is Gly-Ser-Gly.

More preferably, the polypeptide comprises the sequence

(X^{aa4})ₓ₄-Cys-(X^{aa1})ₓ₁-Cys-(X^{aa2})ₓ₂-Cys-(X^{aa3})ₓ₃-(His)₆

wherein x4 is an integer in the range of from 2 to 10, preferably 3 or 8, preferably 3 or 4, more preferably 4, and wherein X^{aa4} are amino acid residues, preferably natural amino acid residues, wherein each X^{aa4} residue may be the same or may be different, preferably (X^{aa4})ₓ₄ is Met-Gly-Gly-Ala.

According to a further preferred embodiment, the polypeptide comprises the sequence

Met-Gly-Gly-Ala -Cys-(X^{aa1})ₓ₁-Cys-(X^{aa2})ₓ₂-Cys-(X^{aa3})ₓ₃

More preferably, the polypeptide comprises the sequence

Met-Gly-Gly-Ala-Cys-(X^{aa1})ₓ₁-Cys-(X^{aa2})ₓ₂-CysGly-Ser-Gly-(His)₆, such as preferably the sequence
MGGACYSVVCHVWMICGSGHHHHHH (SEQ ID NO: 9).

E.g., the polypeptide comprises or consist of the sequence
Met-Gly-Gly-Ala-Cys-(X^{aa1})ₓ₁-Cys-(X^{aa2})ₓ₂-CysGly-Ser-Gly-(His)₆-Gly
such as preferably the sequence
MGGACYSVVCHVWMICGSGHHHHHHG (SEQ ID NO: 11).

According to a further preferred embodiment, the polypeptide comprises the sequence, in particular consists of the sequence
Met-Gly-Gly-Ala-Cys-(X^{aa1})ₓ₁-Cys-(X^{aa2})ₓ₂-CysGly-Ser-Gly-(His)₆-Gly-Lys-Leu-Gly-Gly,

More preferably, the polypeptide comprises the sequence, in particular consists of
MGGACYSVVCHVWMICGSGHHHHHHGKLGG (SEQ ID NO: 10).

Also preferably, the polypeptide was cyclized, i.e. has undergone a cyclization reaction as specified herein below. Preferably, the polypeptide has undergone a cyclization reaction.

Preferably the polypeptide is reacted with a cyclization agent, preferably with TBMB (1,3,5-Tris(brommethylbenzol)). Preferably, in this cyclisation reaction at least 2, preferably three cysteine thiol groups react with TBMB thereby forming a cyclic peptide.

Preferably, each cysteine thiol group comprised in -Cys-(X^{aa1})ₓ₁-Cys-(X^{aa2})ₓ₂-Cys- is being reacted with TBMB whereby each thiol groups forms a covalent bond with a methylene groups (thus a bond -S-CH₂-) and wherein the bromide group is a leaving group. Thus, preferably, the following structure is formed: 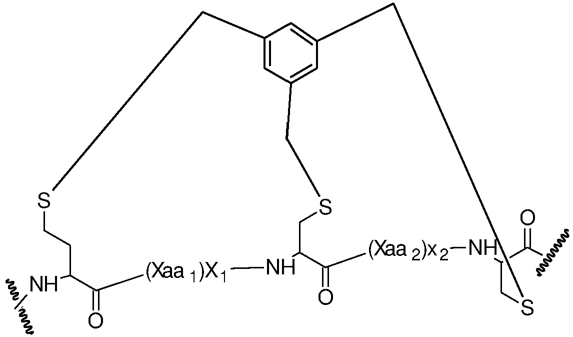 in particular,

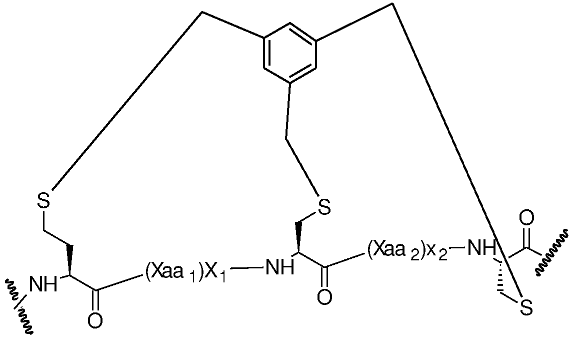

In particular, the cyclic polypeptide comprises, preferably consists of, the structure

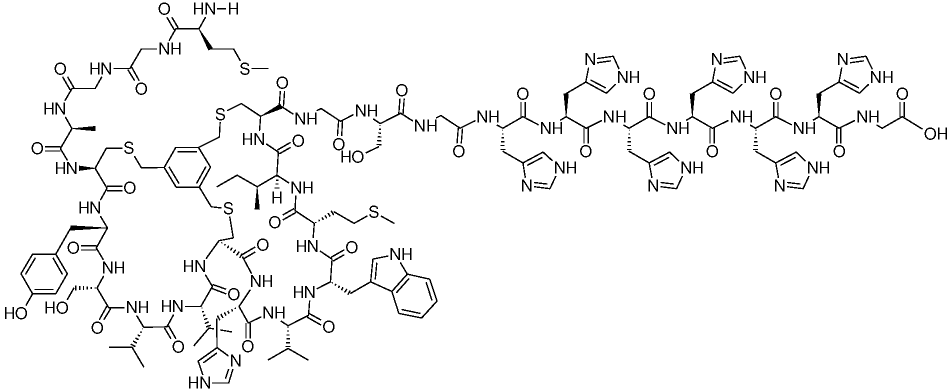

Preferably, the polypeptide is covalently bound to a covalent mRNA-Puromcyin linker conjugate as specified elsewhere herein, preferably via its C terminus.

Steps (I) to (III) are in principle known to the skilled person from protocols for mRNA or cDNA display methods known in the art, e.g. from Arai et al (2019), loc. cit. The method for producing a display complex is an in vitro method, i.e. is not performed on the human or animal body. The method may, however, in principle be performed on isolated cells, e.g. on cultured cells. More preferably, the method is an in vitro method performed using in vitro translation, i.e. more preferably is performed in a cell-free system; corresponding methods are known in the art. Also steps (I) to (III) typically do not require modification of known protocols, except for an optional reduction of the amount of puromycin linker used and an option to use the unpurified synthesis product, as specified herein above. Preferably, however, the covalent mRNA-Puromycin Linker conjugate is provided according to the present invention, i.e. preferably step (I) comprises steps (a) to (c) of the method for producing a covalent mRNA-Puromycin Linker conjugate described herein above.

As the skilled person understands in view of the description herein, step (II) contacting the covalent mRNA-Puromcyin linker conjugate of step (I) with an in vitro translation composition causes the mRNA comprised in the covalent mRNA-Puromcyin linker conjugate to be translated into a polypeptide by said in vitro translation system. Thus, after step (II), the covalent mRNA-Puromcyin linker conjugate comprises a polypeptide translated from the mRNA comprised in the covalent mRNA-Puromcyin linker conjugate. As is also understood by the skilled person, the puromycin moiety in the covalent mRNA-Puromcyin linker conjugate enters the ribosome during translation and becomes covalently attached to the nascent polypeptide. In consequence, the polypeptide is covalently attached to the covalent mRNA-Puromcyin linker conjugate. Thus, in step (II), a display complex as specified herein below is produced from the covalent mRNA-Puromcyin linker conjugate.

Optional step (IV) comprises cyclisation of peptides comprised in said display complex as described above. If optional step (IV) shall be performed, the polypeptide comprised in the display complex preferably comprises at least one cysteine, more preferably at least two, more preferably at least three, in particular three cysteine moieties, preferably as specified herein above. To perform step (IV), the display complex produced in step (d) or (e) preferably is incubated with TBMB.

Preferably, in the reaction a molar ratio of the polypeptide comprised in the display complex to TBMB in the range of from 2:1 to 1:10⁶. Thus, preferably a large excess of TBMB is used.

Preferably, the polypeptide comprised in the display complex and TBMB are reacted with each other for a time in the range of from 1 min to 48 h, preferably for a time in the range of from 5 min to 24 h, more preferably for a time in the range of from 30 min to 12 h, more preferably for a time in the range of from 45 min to 2 h, more preferably for around 1h.

Preferably, the polypeptide comprised in the display complex and TBMB are reacted with each other at a temperature in the range of from 0° C to 50 °C, preferably in the range of from 10°C to 45 °C, more preferably in the range of from 25 °C to 40 °C, preferably in the range of from 35°C to 40 °C, more preferably around 37 °C.

Preferably, the reaction is carried out in a suitable buffer, preferably in NH4HCO3 buffer. The pH is preferably in the range of from 7 to 9, more preferably 8.

Preferably, the display complex is immobilized on a solid support, in particular a bead, preferably a magnetic bead, during the reaction with TBMB.

Preferably the reaction is carried out under stirring.

The present invention also relates to a display complex obtained by the method for producing a display complex as described herein above.

The term "display complex" is understood by the skilled person to relate to a chemical molecule comprising at least an mRNA, building block B, building block A, and a peptide comprising an amino acid sequence encoded by the aforesaid mRNA, wherein all of the aforesaid elements are covalently connected and wherein the covalent connection between building blocks A and B is via a linking group as specified herein above. Thus, the display complex allows to screen for properties of the aforesaid peptide and at the same time includes the encoding mRNA, making it possible to allocate a property of the peptide to an encoding nucleic acid sequence. In view of the above, the display complex preferably comprises a cylic polypeptide.

In view of the description herein, the skilled person understands that the display complex as referred to herein comprises a puromycin linker with a chemical structure different from those known in the art; in particular, the puromycin linker as specified herein comprises a linking group as specified herein above. Moreover, since the puromycin linker can be produced at higher purity, a population of display complexes produced as described herein will comprise less non-functional puromycin linker fragments, in particular less display complexes lacking a puromycin moiety and thus lacking the peptide encoded by the respective mRNA.

The present invention also relates to a protein array obtained by immobilizing a multitude of display complexes as specified herein above on a substrate, preferably in a spatially ordered manner.

The term "protein array" is used herein in its meaning known to the skilled person and preferably includes each and every composition of matter comprising at least two display complexes in a spatially allocated manner. As the skilled person understands in view of the above, display complexes as referred to herein preferably are non-identical only with regards to the coding sequence they comprise and, as a consequence, the encoded peptide they comprise. Preferably, the spatial allocation is a two-dimensional arrangement of non-identical display complexes, preferably allowing identification of at least one display complex from its position within the array. As the skilled person will understand, it may not be necessary to allocate each molecular species of display complex to a specific position; rather, display complexes may be grouped. To that end, any grouping deemed appropriate by the skilled person may be used; e.g. display complexes comprising a pre-determined amino acid, e.g. alanine, as the N-terminal amino acid, but differing in the following amino acids, or peptides having a similar hydrophobicity or charge, or peptides of the same or similar lengths, may be allocated to the same position(s). Also preferably, each molecular species of display complex is allocated to a specific position in the array.

Preferably, the display complexes are immobilized on a solid support in the protein array. Appropriate solid supports are selected by the skilled person without further ado; preferably, the solid support is a glass slide, a plastic slide, or a bead, wherein a plurality of beads may e.g. be color-coded. Preferably, immobilization is performed via group Z of building block B, i.e. preferably by binding of the biotinyl-residue to streptavidin bound to the solid support. Also preferably, immobilization is reversible to release at least the mRNA and/or a cDNA produced therefrom from the solid support. Such release may e.g. be accomplished by cleaving building block B at a riboguanine (rG) by Rnase T1.

The present invention further relates to a method for identifying a binding compound binding to a compound of interest via cDNA display, comprising the steps of the method for preparing a Puromycin Linker as specified herein, the steps of the method for producing a covalent mRNA-Puromycin Linker conjugate as specified herein, and/or the steps of the method for producing a display complex as specified herein.

Steps required for a method for identifying a binding compound binding to a compound of interest via cDNA display are in principle known to the skilled person, and include in particular panning the display complex to the compound of interest, isolating complexes comprising the compound of interest and the display complex, identifying and/or amplifying polynucleotides (mRNA or cDNA) encoding the polypeptides comprised in the isolated complexes. Said steps may be repeated as deemed appropriate by the skilled person.

The term "compound of interest", as used herein, may relate to any composition of matter for which it may be desirable to identify a binding compound. Preferably, the compound of interest is a chemical compound, more preferably of a single molecular species. Preferably, the compound of interest is a compound of scientific and/or economical interest, preferably of medical, chemical, and/or biological relevance. Preferably, the compound of interest is a small molecule chemical compound, i.e. a compound with a molecular mass of at most 1000 u (1 kDa). Also preferably, the compound of interest is a chemical compound metabolized by a body of a subject, in particular of a human subject, or is a compound administered to a subject in order to induce a change in the subject's metabolism. More preferably, the compound of interest is a macromolecule, i.e. a compound with a molecular mass of more than 1000 u (i.e. more than 1 kDa). Even more preferably, the compound of interest is a biological macromolecule, in particular a polypeptide, a polynucleotide, a polysaccharide, or a fragment of any of the aforesaid. Most preferably, the compound of interest is a polypeptide.

As used herein, the term "binding compound" relates to any chemical molecule comprising or consisting of a (poly)peptide having the property of binding to a compound of interest as specified herein above. As the skilled person will understand in view of the description herein, the binding compound preferably comprises or consists of a peptide encoded by the mRNA as specified herein. The binding compound is identified via a display method comprising the steps of the method for producing a display complex as specified herein, which produces at least one display complex which comprises a peptide which can be tested for binding to a compound of interest. Said testing may be performed using an array as described herein above; testing may, however, also be performed by bulk methods using the compound of interest as a bait. Thus, the method identifying a binding compound preferably comprises a further step of determining binding of a binding complex as specified herein to said compound of interest. Optionally, the method may comprise the steps of the method for preparing a Puromycin Linker as specified herein and/or optionally the steps of the method for producing a covalent mRNA-Puromycin Linker conjugate as specified herein.

Summarizing the findings of the present invention, the following embodiments are preferred:
1. Method (P) for preparing a Puromycin Linker (I) for producing a complex of an mRNA and a polypeptide encoded by said mRNA, the method comprising reacting
   - a building block A, said building block comprising a Puromycin group (Pur) and a functional group FG^{a}, via said functional group FG^{a}, wherein the functional group FG^{a} is -N₃.
   - with a building block B comprising a functional group FG^{b} via said functional group FG^{b}, wherein the functional group FG^{b} is 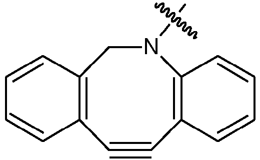
      thereby forming a covalent linkage between A and B,
      wherein B further comprises and a single-stranded polynucleotide segment essentially complementary to a sequence on the 3'-terminal side of the mRNA over a stretch of at least 3, preferably at least 12 nucleotides, more preferably at least 15, more preferably at least 22 nucleotides, wherein the functional group FG^{a} and the functional group FG^{b} are linked a strain-promoted azide-alkyne cycloaddition.
2. The method according to embodiment 1, wherein the building block B further comprises a biotin group Z, preferably wherein Z is dT-Biotin or a group having the structure: 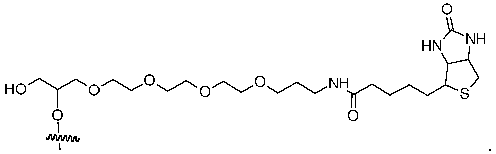
3. The method according to embodiment 1 or 2, wherein the puromycin group (Pur) has the structure 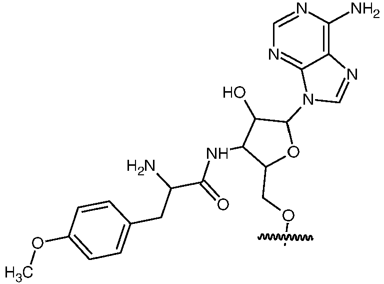
4. The method according to any one of embodiments 1 to 3, wherein the reacting is carried out in the absence of a catalyst.
5. The method according to any one of embodiments 1 to 4, wherein upon reaction of the functional group FG^{a} with the functional group FG^{b}, a linking group a'-b' is formed, the linking group a'-b' having the structure 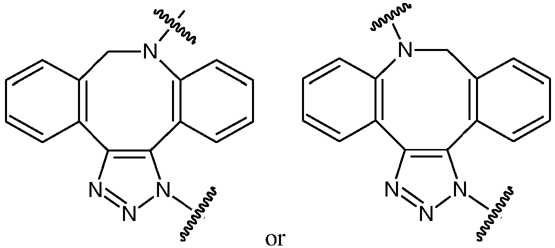
6. The method according to any one of embodiments 1 to 5, wherein in the reaction the molar ratio of building block A to building block B is > 1 : 1.
7. The method according to any one of embodiments 1 to 6, wherein in the reaction the molar ratio of building block A to building block B is of from 1.1 : 1 to 5:1, preferably of from 1.5 :1 to 3:1, more preferably around 2:1.
8. The method according to any one of embodiments 1 to 7, wherein in the reaction the molar ratio of building block A to building block B is of from 1.1 : 1 to 5:1, preferably of from 1.5 :1 to 3:1, more preferably around 2:1.
9. The method according to any one of embodiments 1 to 8, wherein building block A and B are reacted with each other for a time in the range of from 5 min to 24h, preferably of from 10 min to 10 h, more preferably of from 2 h to 5 h, more preferably around 3 h.
10. The method according to any one of embodiments 1 to 9, wherein the Puromycin Linker (I) has the structure (I*) 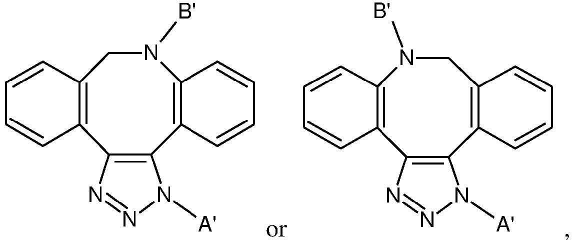 wherein A' is the remainder of building block A and B' is the remainder of building block B.
11. The method according to any one of embodiments 1 to 10, wherein building block A further comprises a labeling group, preferably a fluorescent group, more preferably a fluorescein group.
12. The method according to any one of embodiments 1 to 11, wherein building block A further comprises a linker group comprising ethoxyethyl groups, more preferably a spacer L¹ comprising the group [-CH₂CH₂-O-]ₙ with n = 10-15.
13. The method according to any one of embodiments 1 to 12, wherein building block A further comprises a single stranded polynucleotide segment, comprising a nucleotide with a base being substituted with a labeling group (labeled nucleotide), preferably a fluorescent group, more preferably wherein the fluorescent group is fluorescein.
14. The method according to embodiment 13, wherein the labeled nucleotide is a fluorescein-labeled deoxy-thymidin and preferably has the structure: 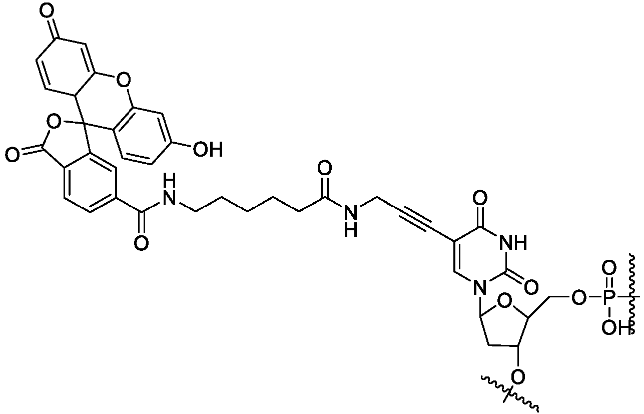
15. The method according to any one of embodiments 1 to 14, wherein building block B further comprises a reverse transcription primer region at the 3'terminal end, preferably a CCT region.
16. The method according to any one of embodiments 1 to 15, wherein building block B further comprises a single-stranded polynucleotide segment comprising a cleavage site, said cleavage site preferably comprising riboguanine (rG), more preferably wherein said cleavage site is an Rnase T1 recognition site.
17. The method according to any one of embodiments 1 to 16, wherein building block B further comprises a cyanovinylcarbazol group (CnVK), preferably a group having the structure 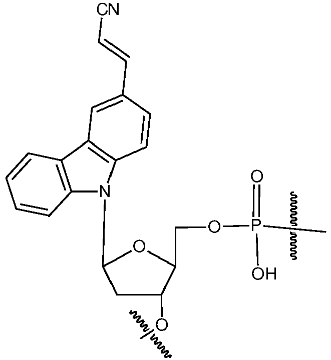
18. The method according to any one of embodiments 1 to 17, wherein building block A has the structure FG^{a}-L²-(X)₁₋₅-(dT(Fluorescein))-(X)₃₋₁₀-L¹-(X)₁₋₅-(Pur), wherein each X is independently selected from A, T, G, and C, wherein L1 and L2 are linker, and wherein dT(Fluorescein) is fluorescein-labeled deoxy-thymidin and preferably has the structure: 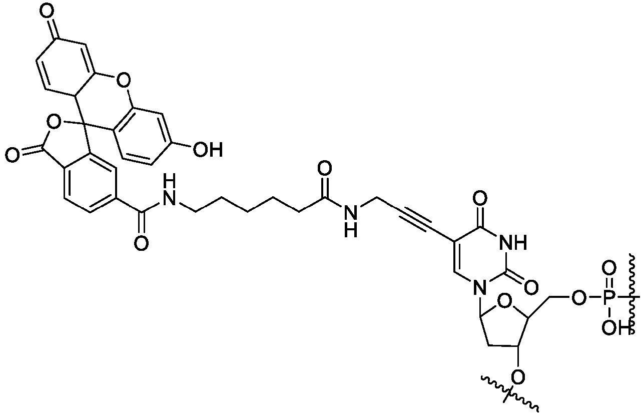
19. The method according to embodiment 18, wherein building block A has the structure FG^{a}-L²-TC-(dT(Fluorescein))-CTC-L¹-CC-(Pur).
20. The method according to embodiment 19, wherein building block A has the structure 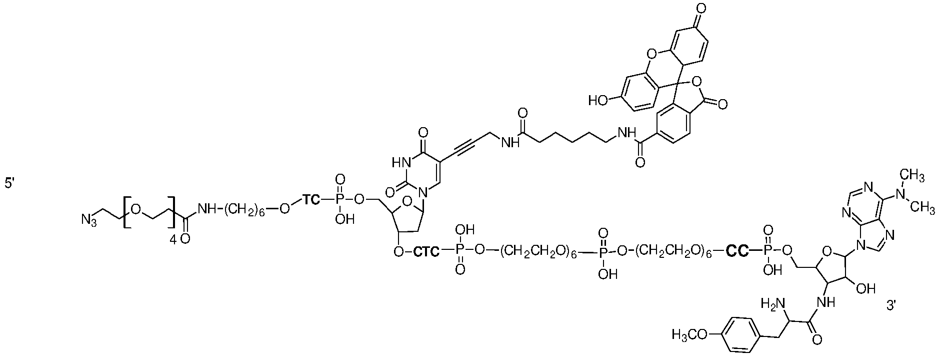
21. The method according to any one of embodiments 1 to 20, wherein building block B has the structure Z-(X)₂₋₅-rG-(X)₂₋₁₀-(CnVK)-(X)₅₋₁₅-X(L³-(FG^{b}))-(X)₃₋₅, wherein each X is independently selected from A, T, G, and C, wherein rG is ribo-guanosine, and A, T, G, an C are deoxynucleotides and wherein L³ is a linker and CnVK is a cyanovinylcarbazol group, and wherein Z is dT-Biotin or a group having the structure: 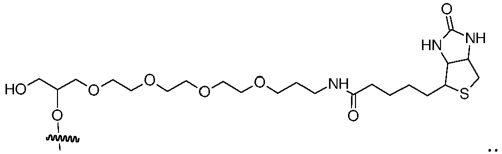
22. The method according to any one of embodiments 1 to 20, wherein building block B has the structure Z-AA-rG-AATTTCCA-(CnVK)-GCCGCCCCCCG-T(L³-(FG^{b}))-CCT, wherein L³ is a linker, CnVK is a cyanovinylcarbazol group, rG is ribo-guanosine, and A, T, G, an C are deoxynucleotides, and wherein Z is dT-Biotin or a group having the structure: 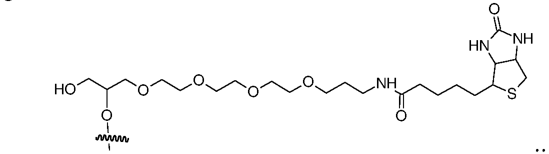 preferably dT(biotin).
23. The method according to any one of embodiments 1 to 20, wherein building block B has the structure 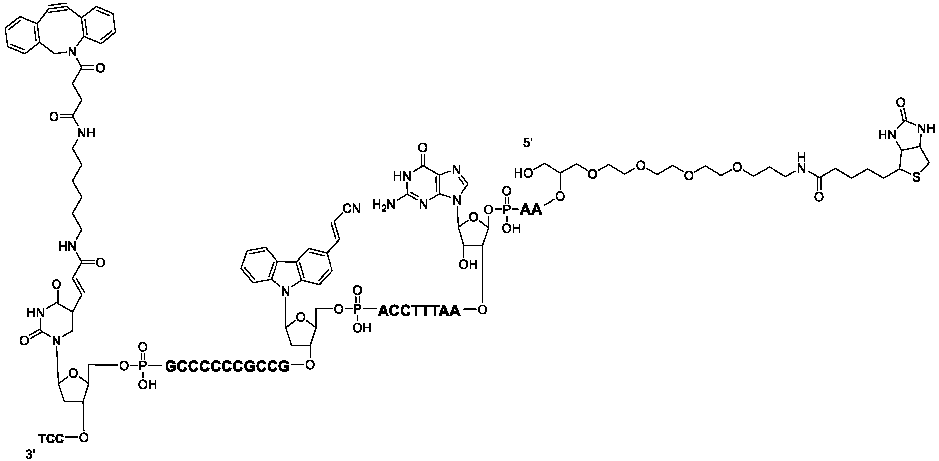
24. The method according to any one of embodiments 1 to 23, wherein the Puromycin Linker (I) has the structure (Ia) 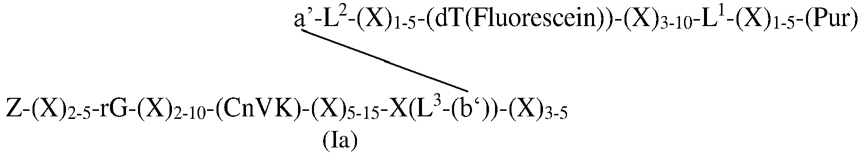
   wherein each X is independently selected from A, T, rG, G, and C, wherein rG is ribo-guanosine, and A, T, G, an C are deoxynucleotides and wherein L³ is a linker and CnVK is a cyanovinylcarbazol group, and wherein Z is dT-Biotin or a group having the structure: 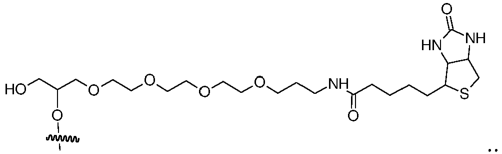
   and wherein L1 and L2 are linker, and wherein dT(Fluorescein) is fluorescein-labeled deoxy-thymidin and preferably having the structure: 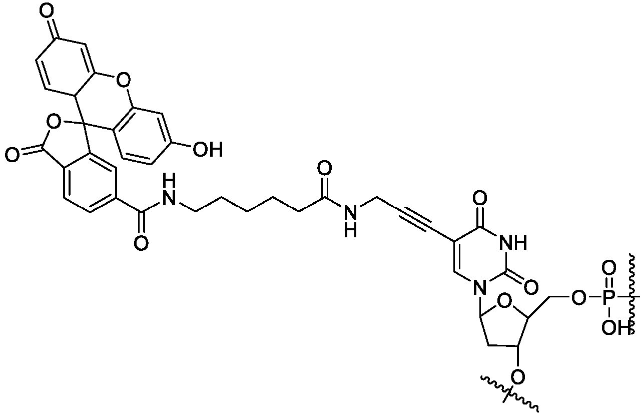
   and a'-b' is the linking group which is formed upon reaction of the functional group FG^{a} with the functional group FG^{b}.
25. The method according to embodiment 23, wherein the Puromycin Linker (I) has the structure (Iaa): 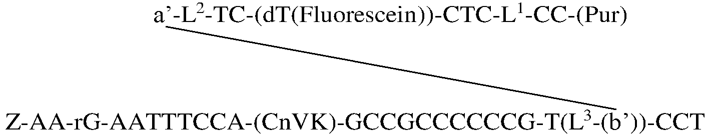
26. The method according to any one of embodiments 1 to 23, wherein the Puromycin Linker (I) has the structure (Iaaa) and/or (Iaaa*).
27. The method according to any one of embodiments 1 to 26, wherein building block B further comprises a labeling group, preferably a fluorescent group, more preferably a fluorescein group.
28. The method according to any one of embodiments 27, wherein building block B further comprises a single stranded polynucleotide segment, comprising a nucleotide with a base being substituted with a labeling group (labeled nucleotide), preferably a fluorescent group, more preferably wherein the fluorescent group is fluorescein.
29. The method according to embodiment 27 or 28, wherein the labeled nucleotide is a fluorescein-labeled deoxy-thymidin and preferably has the structure: 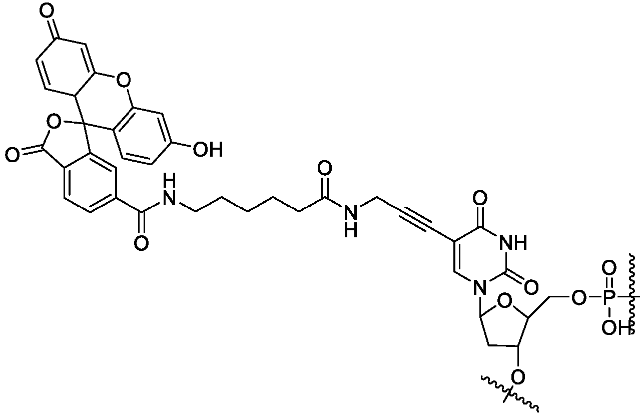
30. The method according to any one of embodiments 27 to 29, wherein building block B further comprises a reverse transcription primer region at the 3'terminal end, preferably a CCT region.
31. The method according to any of embodiments 27 to 30, wherein building block B further comprises a single-stranded polynucleotide segment comprising a cleavage site, said cleavage site preferably comprising riboguanine (rG), more preferably wherein said cleavage site is an Rnase T1 recognition site.
32. The method according to any one of embodiments 27 to 20, wherein building block B further comprises a cyanovinylcarbazol group (CnVK), preferably a group having the structure 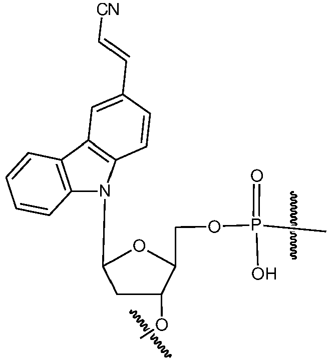
33. The method according to any one of embodiments 27 to 32, wherein building block B has the structure Z-(X)₂₋₅-rG-(X)₂₋₅-(dT(Fluorecein)-(X)₂₋₁₀-(CnVK)-(X)₅₋₁₅-X(L3-(FG^{b}))-(X)₃₋₅, wherein each X is independently selected from A, T, G, and C, wherein rG is ribo-guanosine, and A, T, G, an C are deoxynucleotides, and wherein L³ is a linker and CnVK is a cyanovinylcarbazol group.
34. The method according to any one of embodiments 27 to 33, wherein building block B has the structure Z-AA-rG-AA-(dT(Fluorescein)-TTCCA-(CnVK)-GCCGCCCCCCG-T(L3-(FG^{b}))-CCT, wherein L³ is a linker, CnVK is a cyanovinylcarbazol group, rG is ribo-guanosine, and A, T, G, an C are deoxynucleotides, and Z is wherein Z is dT-Biotin or a group having the structure: 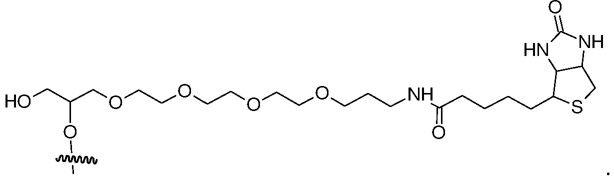
35. The method according to any one of embodiments 27 to 34, wherein building block B has the structure 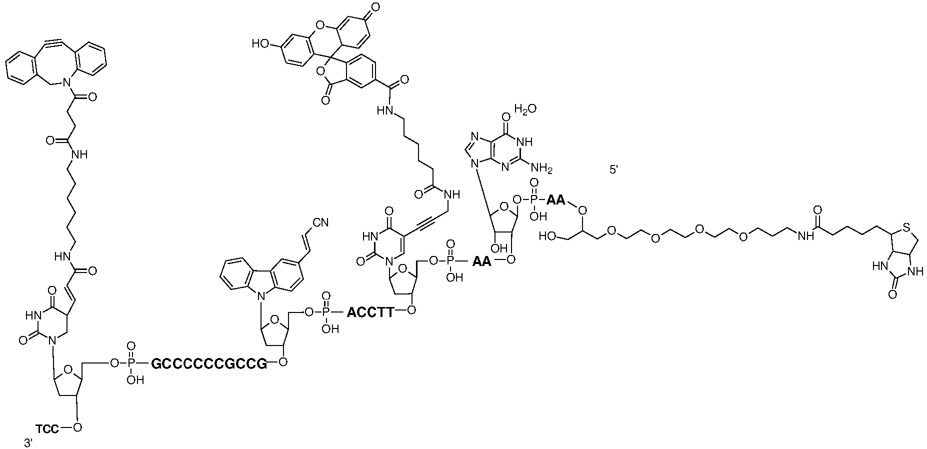
36. The method according to any one of embodiments 27 to 35, wherein building block A further comprises a linker group comprising ethoxyethyl groups, more preferably a spacer L¹ comprising the group [-CH₂CH₂-O-]ₙ with n = 10-15.
37. The method according to any one of embodiments 27 to 36, wherein building block A has the structure FG^{a}-L²-(X)₄₋₁₅-L¹-(X)₁₋₅-(Pur), wherein each X is independently selected from A, T, G, and C, wherein L¹ and L² are linker.
38. The method according to embodiment 37, wherein building block A has the structure FG^{a}-L²-TCTCTC-L¹-CC-(Pur).
39. The method according to embodiment 38, wherein building block A has the structure 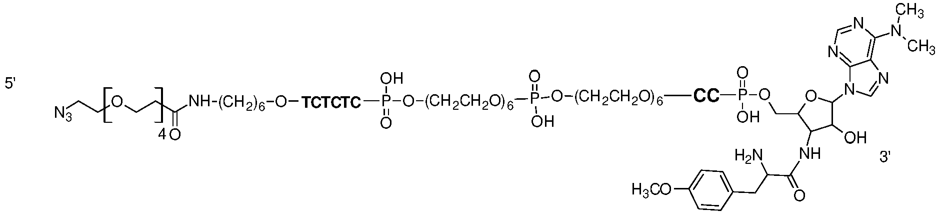
40. The method according to any one of embodiments 27 to 29, wherein the Puromycin Linker (I) has the structure (Ib): 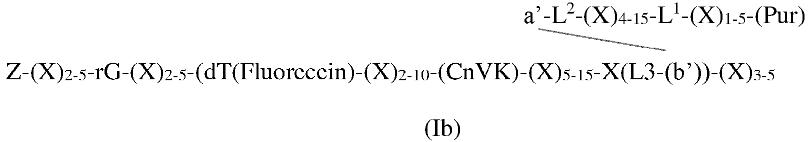
   wherein each X is independently selected from A, T, G, and C, wherein L¹ and L² are linker, wherein rG is ribo-guanosine, wherein A, T, G, an C are deoxynucleotides, and wherein L³ is a linker and CnVK is a cyanovinylcarbazol group, and wherein Z is dT-Biotin or a group having the structure: 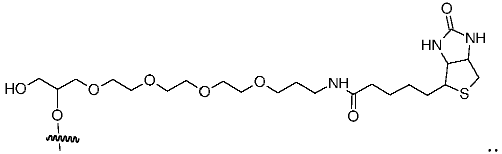
   and wherein dT(Fluorescein) is fluorescein-labeled deoxy-thymidin and preferably having the structure: 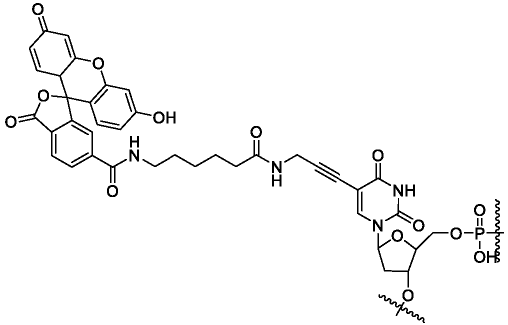
   and a'-b' is the linking group which is formed upon reaction of the functional group FG^{a} with the functional group FG^{b}, the linking group preferably comprising a triazole, more preferably wherein the linking group a'-b' is 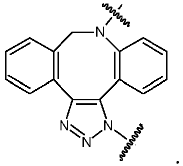
41. The method according to embodiments 40, wherein the Puromycin Linker (I) has the structure (Ibb): 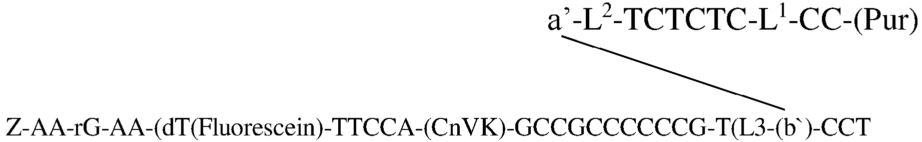
42. The method according to embodiments 41, wherein the Puromycin Linker (I) has the structure (Ibbb) and/or the structure (Ibbb*).
43. A Puromycin Linker for producing a complex of mRNA and a polypeptide encoded by that mRNA obtained or obtainable by the method according to of any one of embodiments 1 to 42.
44. The Puromycin Linker according to embodiment 43, having the structure (Iaaa) or (Ibbb).
45. Puromycin Linker for producing a complex of an mRNA and a polypeptide encoded by said mRNA comprising
   a building block A comprising a Puromycin group and a building block B comprising a single-stranded polynucleotide segment essentially complementary to a sequence on the 3'-terminal side of the mRNA over a stretch of at least 3, preferably at least 12 nucleotides, more preferably at least 15, more preferably at least 22 nucleotides.
   wherein (A) and (B) are linked via the functional moiety a'-b', the linking group a'-b' having the structure 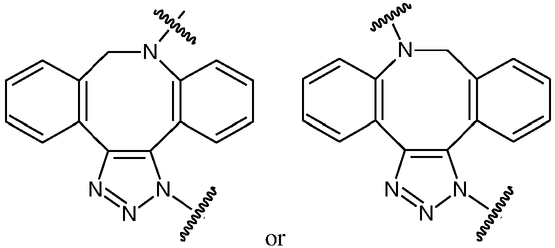
46. Puromycin Linker according to embodiment 45 having the structure (Ia) 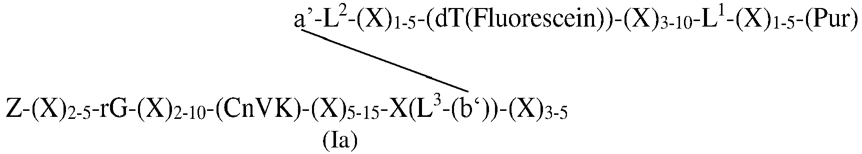
   wherein Pur is a puromycin group, each X is independently selected from A, T, rG, G, and C, wherein rG is ribo-guanosine, and A, T, G, an C are deoxynucleotides and wherein L³ is a linker and CnVK is a cyanovinylcarbazol group, and wherein Z is dT-Biotin or a group having the structure: 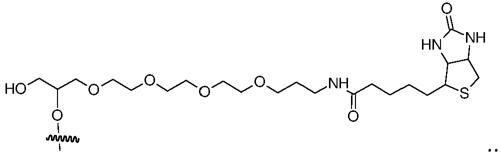
   and wherein L1 and L2 are linker, and wherein dT(Fluorescein) is fluorescein-labeled deoxy-thymidin and preferably having the structure: 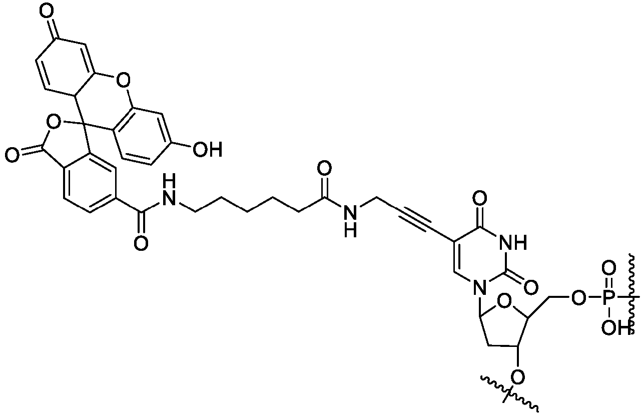
   the linking group a'-b' having the structure 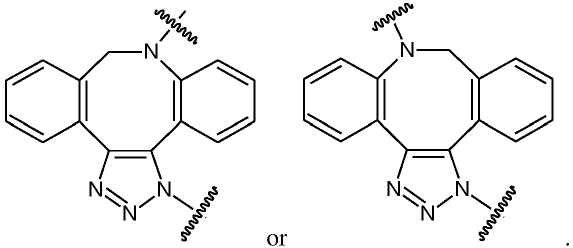
47. Puromycin Linker according to embodiment 45 having the structure (Ib): 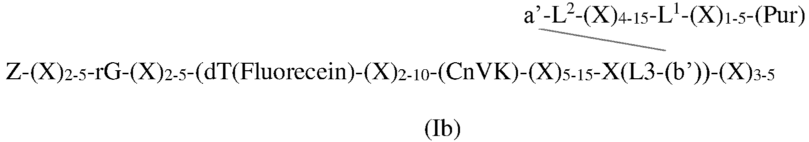
   wherein Pur is a puromycin group, and wherein each X is independently selected from A, T, G, and C, wherein L¹ and L² are linker, wherein rG is ribo-guanosine, wherein A, T, G, an C are deoxynucleotides, and wherein L³ is a linker and CnVK is a cyanovinylcarbazol group, and wherein Z is dT-Biotin or a group having the structure: 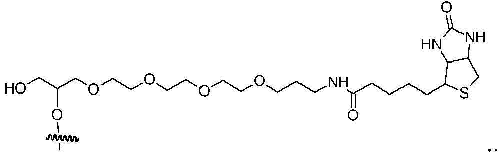
   and wherein dT(Fluorescein) is fluorescein-labeled deoxy-thymidin and preferably having the structure : 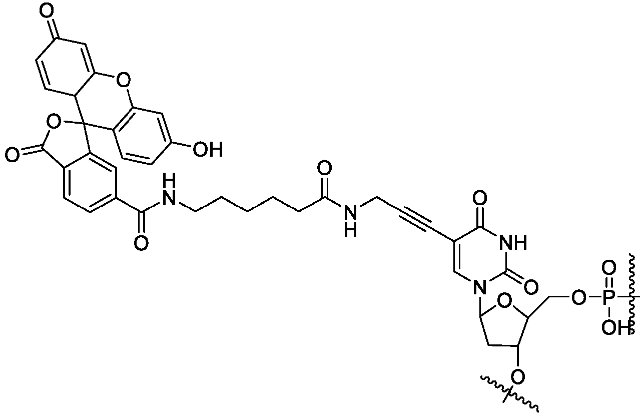
   the linking group a'-b' having the structure 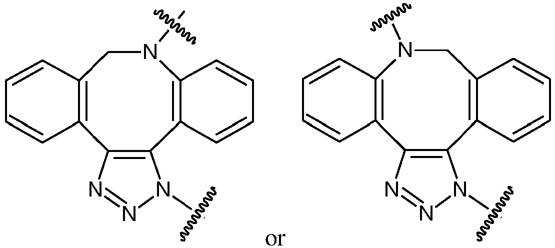
48. A building block A, having the structure FG^{a}-L²-(X)₁₋₅-(dT(Fluorescein))-(X)₃₋₁₀-L¹-(X)₁₋₅-(Pur), wherein Pur is a puromycin group, FG^{a} is an azide group, each X is independently selected from A, T, G, and C, wherein L¹ and L² are linker, and wherein dT(Fluorescein) is fluorescein-labeled deoxy-thymidin and preferably has the structure: 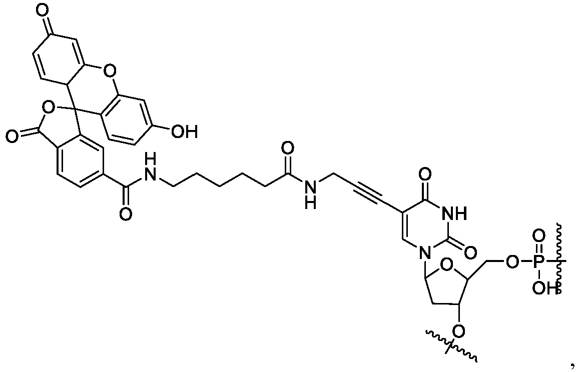 preferably, the structure FG^{a}-L²-TC-(dT(Fluorescein))-CTC-L¹-CC-(Pur), more preferably the structure 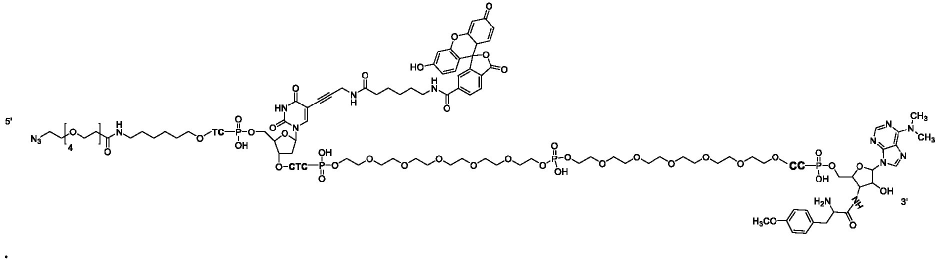
49. A building block A, having the structure FG^{a}-L²-(X)₄₋₁₅-L¹-(X)₁₋₅-(Pur), wherein Pur is a puromycin group, FG^{a} is an azide, wherein each X is independently selected from A, T, G, and C, wherein L¹ and L² are linker, preferably the structure FG^{a}-L²-TCTCTC-L¹-CC-(Pur), more preferably the structure 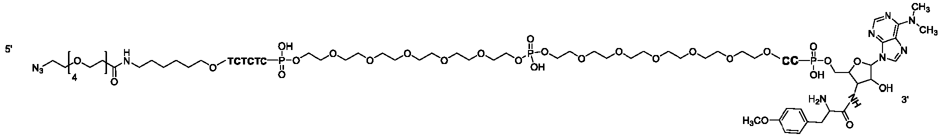
50. A building block B, having the structure

   Z-(X)₂₋₅-rG-(X)₂₋₁₀-(CnVK)-(X)₅₋₁₅-X(L³-(FG^{b}))-(X)₃₋₅,

   - wherein FG^{b} is 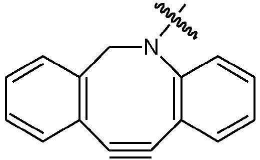
      and wherein each X is independently selected from A, T, G, and C, wherein rG is ribo-guanosine, and A, T, G, an C are deoxynucleotides and wherein L³ is a linker and CnVK is a cyanovinylcarbazol group, and wherein Z is dT-Biotin or a group having the structure: 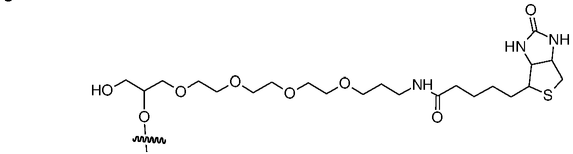
      preferably the structure Z-AA-rG-AATTTCCA-(CnVK)-GCCGCCCCCCG-T(L³-(FG^{b}))-CCT, wherein L³ is a linker, CnVK is a cyanovinylcarbazol group, rG is ribo-guanosine, and A, T, G, an C are deoxynucleotides, and wherein Z is dT-Biotin or a group having the structure: 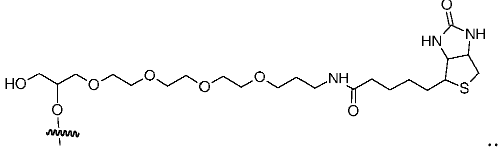
      preferably dT(biotin).
      wherein building block B has the structure 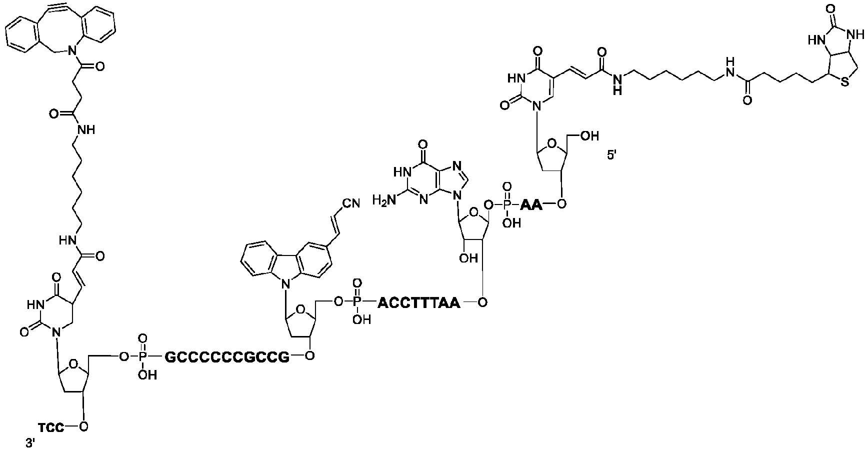
51. A building block B, having the structure

   Z-(X)₂₋₅-rG-(X)₂₋₅-(dT(Fluorecein)-(X)₂₋₁₀-(CnVK)-(X)₅₋₁₅-X(L3-(FG^{b}))-(X)₃₋₅

   - wherein FG^{b} 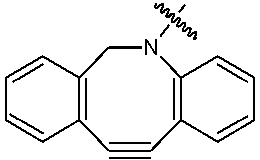
      and wherein each X is independently selected from A, T, G, and C, wherein rG is ribo-guanosine, and A, T, G, an C are deoxynucleotides, and wherein L³ is a linker and CnVK is a cyanovinylcarbazol group,
      more preferably the building block B has the structure Z-AA-rG-AA-(dT(Fluorescein)-TTCCA-(CnVK)-GCCGCCCCCCG-T(L3-(FG^{b}))-CCT,
      wherein L³ is a linker, CnVK is a cyanovinylcarbazol group, rG is ribo-guanosine, and A, T, G, an C are deoxynucleotides, and Z is wherein Z is dT-Biotin or a group having the structure: 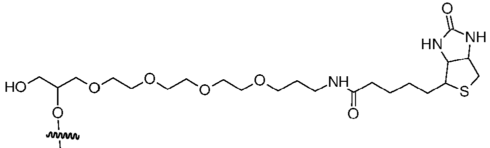
      in particular, the building block B has the structure 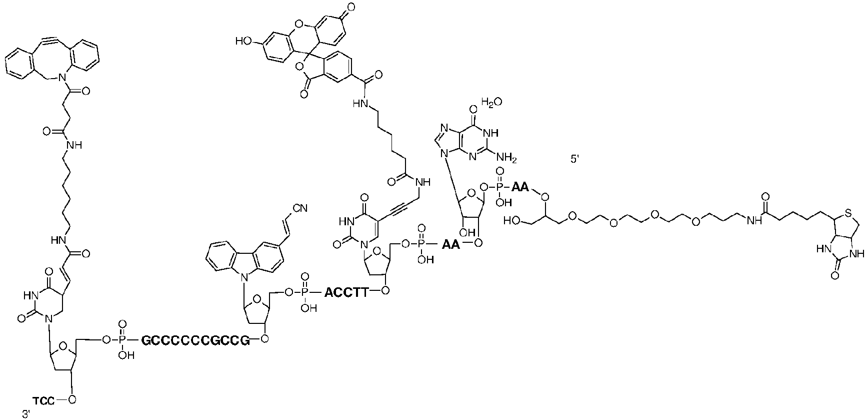
52. A method for producing a covalent mRNA-Puromycin Linker conjugate, said method comprising the steps of any one of embodiments 1 to 42 and the further steps
   (a) contacting the Puromycin Linker produced in the preceding steps with an mRNA;
   (b) photocrosslinking said mRNA and said Puromycin Linker, and thereby
   (c) producing a covalent mRNA-Puromcyin Linker conjugate.
53. A method for producing a covalent mRNA-Puromycin Linker conjugate, said method comprising the steps
   (a) contacting the Puromycin Linker(I) produced in the preceding steps with an mRNA;
   (b) photocrosslinking said mRNA and said Puromycin Linker, and thereby
   (c) producing a covalent mRNA-Puromcyin Linker conjugate.
   wherein the Puromycin Linker (I) according to step (a) is prepared by method (P) as described in any one of embodiments 1 to 42.
54. The method of embodiment 52 or 53, wherein said contacting step (a) is performed immediately after said preparing a Puromycin Linker, preferably without purifying the prepared Puromycin Linker.
55. The method of embodiment 54, wherein performing immediately is performing contacting step (a) with the product as it is obtained in the method for preparing a Puromycin Linker.
56. The method of embodiment 53 or 54, wherein performing immediately comprises avoiding purifying said Puromycin Linker before step (a).
57. A method for producing a display complex, the method comprising
   (I) providing a covalent mRNA-Puromycin Linker conjugate, preferably by the steps of the method for producing a covalent mRNA-Puromycin Linker conjugate of any one of embodiments 52 to 56,
   (II) contacting the covalent mRNA-Puromcyin linker conjugate of step (I) with an in vitro translation composition to produce a display complex comprising a polypeptide encoded by said mRNA,
   (III) optionally reverse transcribing said mRNA to a cDNA, and optionally
   (IV) cyclisation of the polypeptide comprised in said display complex.
58. A display complex obtained by the method according to the method of embodiment 57.
59. A protein array obtained by immobilizing a multitude of display complexes according to embodiment 57 or 58 on a substrate, preferably in a spatially ordered manner.
60. A method for identifying a binding compound binding to a compound of interest via cDNA display, comprising the steps of the method for preparing a Puromycin Linker according to any one of embodiments 1 to 42, the steps of the method for producing a covalent mRNA-Puromycin Linker conjugate of embodiments 52 to 56, and/or the steps of the method for producing a display complex of embodiment 57.
61. The subject matter of any one of embodiments 57 to 60, wherein said polypeptide is a cyclic polypeptide.

### FIGURES

**Fig. 1****:** FAM-puromycin segment
**Fig. 2****:** Biotin Segment with dT-Biotin

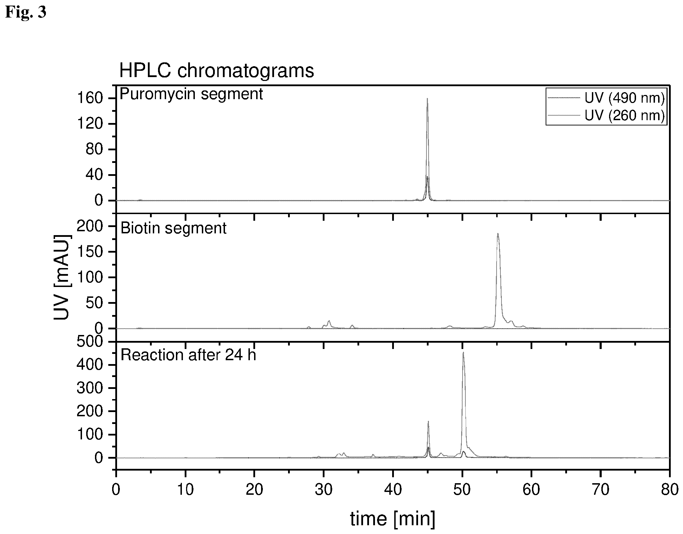

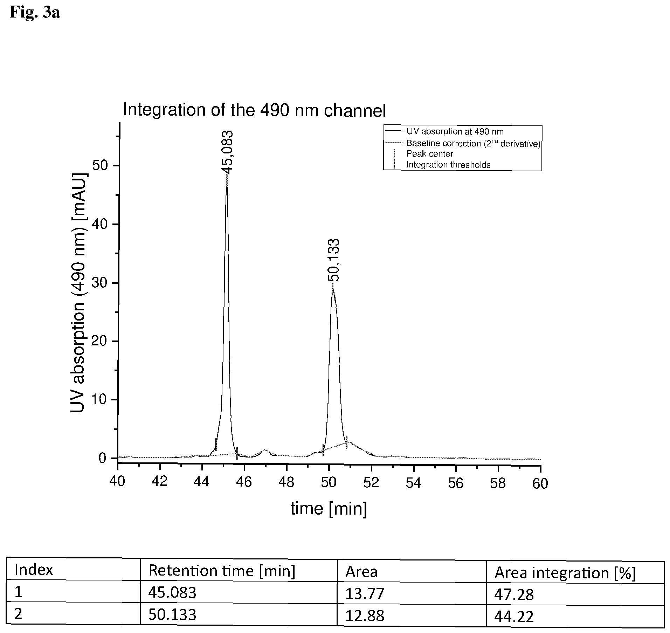

**Fig. 3****:** HPLC-chromatograms of the FAM puroomycin segment and the biotin segment with dT biotin as well as the chromatogram after 24 h of SPPAC reaction. All runs had the same conditions: "Machery Nagel EC 250/4.6 Nulceodur 300-5 C18ec" column (250 mm length, 4.6 mm thickness) driven with 1 mL min⁻¹ flow rate. A 0.1 M solution of triethylamine acetate (TEAA) in H₂O was used as solvent A and acetonitrile as solvent B. For elution a gradient from 90 - 70% A was driven over 80 min (0.25 percent per minute). The HPLC chromatograms after 24 h of SPAAC reaction shows that the Biotin segment's peak (around 55 min elution time) completely disappears. Puromycin segment was in twofold exceed, so there is still about 50% of starting material detectable..
**Fig. 3a****:** HPLC record with integration over the 490 nm channel. The peak at 45 min is the unreacted exceed of FAM labeled puromycin segment, the peak at 50 min is the puromycin linker.
**Fig. 4****:** Biotin segment with TEG biotin.

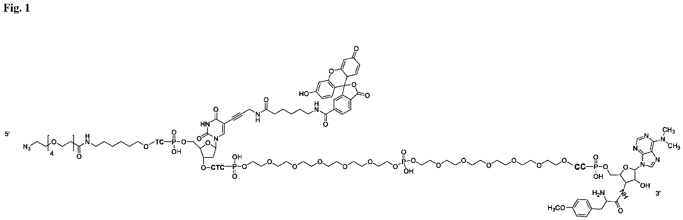

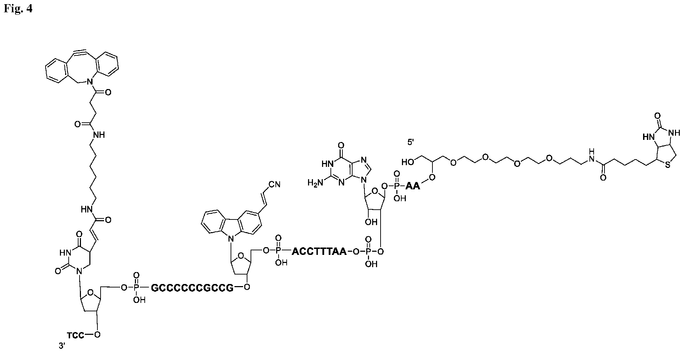

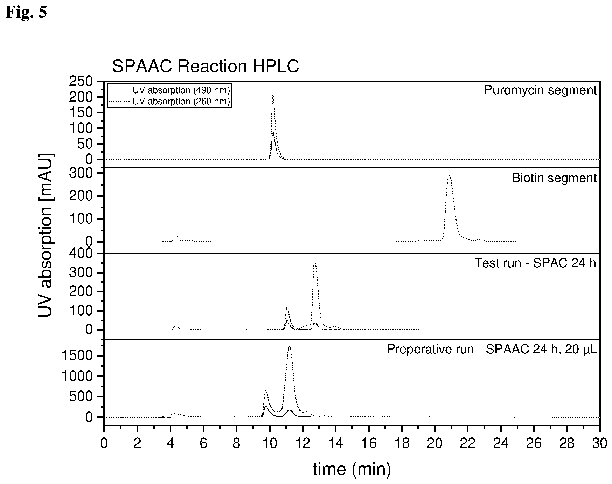

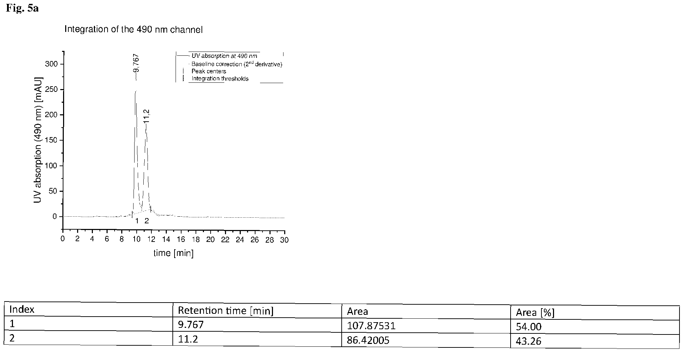

**Fig. 5****:** Analytic and preparative HPLC of the SPAAC reaction with the biotin and puromycin building blocks according to figures 4 and 1. As described above, the puromycin building block is used in the reaction in twofold excees. There is a peak shift between the analytical 2 µL and the preparative 20 µL run. The HPLC chromatograms were recorded at two channels, a 490 nm channel to detect the puromycin building block's and the puromycin linker's FAM's signal, as well as an 260 nm channel for general DNA detection. The 490 nm signal is used for yield estimation. The HPLC was conducted using a "Machery Nagel EC 250/4.6 Nulceodur 300-5 Cl8ec" column (250 mm length, 4.6 mm thickness) driven with 1 mL min-1 flow rate. A 0.1 M solution of triethylamine acetate (TEAA) in H₂O was used as solvent A and acetonitrile as solvent B. For elution a gradient from 84% - 70% (A) was driven over 30 min.
**Fig. 5a****:** HPLC record with integrated 490 nm channel. The peak at 9.7 min is the unreacted exceed of FAM labeled puromycin segment, peak at 11.2 min is the puromycin linker.
**Fig. 6****:** Puromycin segment without fluorescence label.
**Fig. 7****:** Biotin segment with TEG biotin and FAM fluorescence label.
**Fig. 8a****:** HPLC record with integrated 490 nm channel. The product peak (3) was integrated to about 90%.
**Fig. 9a****:** MALDI-TOF spectra of the puromycin segment, the biotin segment and the product related HPLC peak. All spectra were recorded in a 1:1 mixture of HCCA and 2,5-DHB.
**Fig. 9b****:** Isomers from SPAAC reaction.
**Fig. 10****:** Puromycin segment with 5' Thiol modifier (see Fig. 10)
**Fig. 11****:** Biotin segment with dT-C6-amino modifier (see Fig. 11)
**Fig. 12:** Figure 12: Lineup between the starting materials, the biotin segment after modification with EMCS and run 3 (which has the best baseline overlay between the 260 nm and 490 nm channel) of the Michael addition.
**Fig. 13****:** Expected molecule
**Fig. 14****:** Obtained Molecule
**Fig. 15** : ESI-MS measurement of the biotin segment after reaction with EMCS.
**Fig. 16****:** MALDI-TOF spectrum of the 5.7 min HPLC peak of the Michael addition. Recorded from 2,5-Dihydroxybenzoeic acid
**Fig. 17****:** Structure A3
**Fig. 18****:** Structure B3
**Fig. 18a****:** Structure B'3
**Fig. 19****:** Structure of Puromycin linker (Iaaa)
**Fig. 19a****:** Structure of Puromycin linker (Iaaa*)
**Fig. 19****:** Structure of Puromycin linker (I'aaa)
**Fig. 19a****:** Structure of Puromycin linker (I'aaa*)
**Fig. 20****:** Structure 2B3
**Fig. 20a****:** Structure 2B'3
**Fig. 21****:** Structure 2A3
**Fig. 22****:** Structure of Puromycin linker (Ibbb)
**Fig. 22a****:** Structure of Puromycin linker (Ibbb*)
**Fig. 22b****:** Structure of Puromycin linker (I'bbb)
**Fig. 22c****:** Structure of Puromycin linker (I'bbb*)

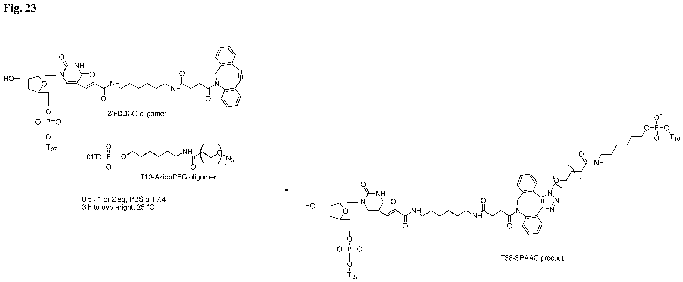

**Fig. 23****:** Reaction scheme of 3+2 cycloaddition of T28-DBCO oligomer (T28 including 5' C6-amino modifier and dibenzocyclooctine modification) with T10-AzidoPEG (3' modification with C6-amino modifier and PEG4 azide) forming a tetrazine linking both starting molecules.

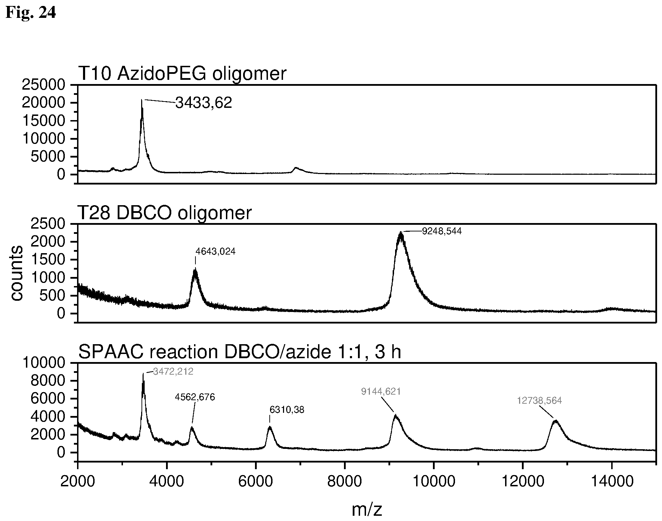

**Fig. 24****:** MALDI-TOF spectra of example 4.4.1 showing the T10 AzidoPEG oligomer, T28 DBCO oligomer and the 3 h reaction control measurement. Single charged species are labeled in red.

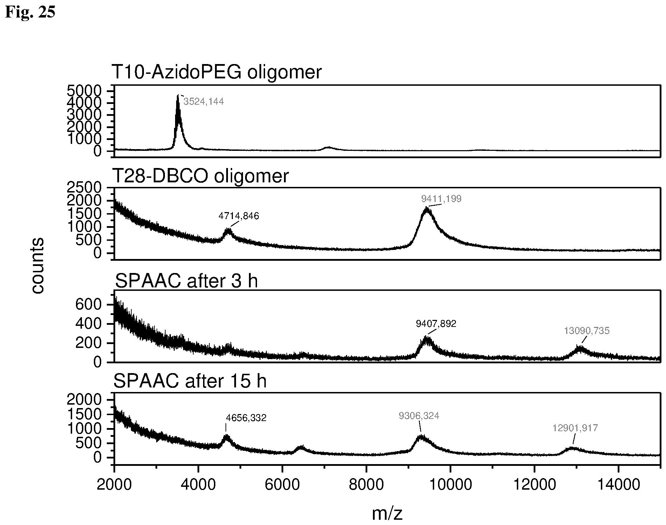

**Fig. 25****:** MALDI-TOF spectra of example 4.4.2 showing the T10-AzidoPEG oligomer and T28-DBCO oligomer and the reaction control measurements after 3 h and over-night after 15 h. 1 µL probe with 1 µL 2,5 dihydroxybenzoeic acid in 50% (v/v) acetonitrile in H₂O matrix solution. 15 h reaction time shows less noisy signals, because a new matrix solution was used. The spectrum of T28-DBCO oligomer shows two peaks. One at approx. 9500 Da and one around 4750 Da. The 4750 Da peak is a double charged species of the T28-DBCO oligomer that appears at the half mass to charge ratio as the single charged one. The reaction product appears in single charge at 13,000 Da and in double charge around 6500 Da.

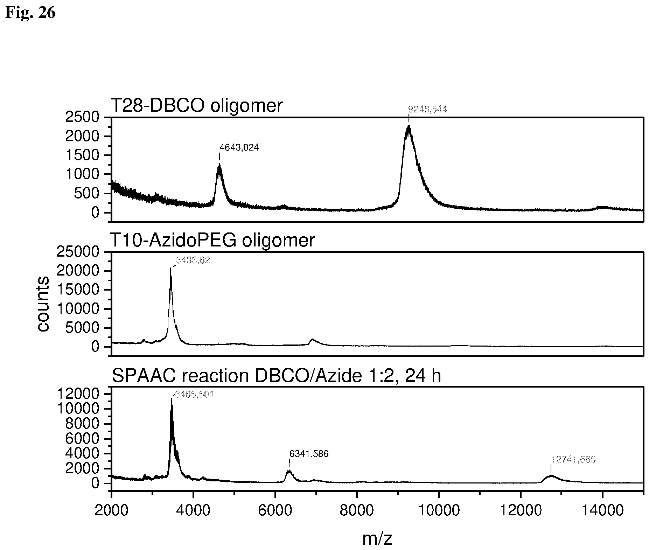

**Fig. 26****:** MALDI-TOF spectra of example 4.4.3 showing the T28-DBCO, T10 azido oligomer and the reaction control after 24 h. The T28-DBCO oligomer completely disappeared in the reaction control.

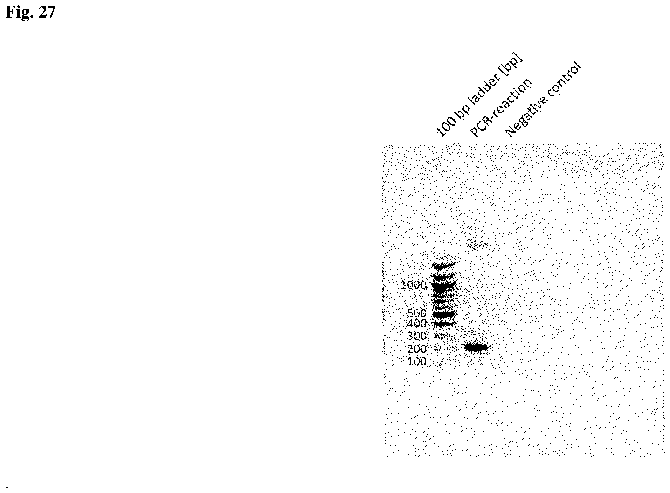

**Fig. 27****:** 1.5% agarose gel electrophoresis sybr safe PCR of working example 5.
**Figure 18****:** 10% SDS PAGE with 8 M Urea of working example 5.

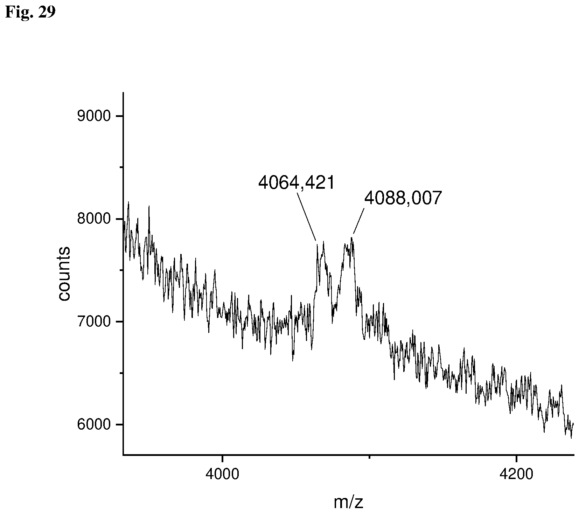

**Figure 29****:** MALDI-TOF recorded from HCCA-matrix of working example 5. The 4046 peak matches with the cyclic peptide with the sequence "MGGACYSVVCHVMWICGSGHHHHHHG-puromycin-cytosine-cytosine"

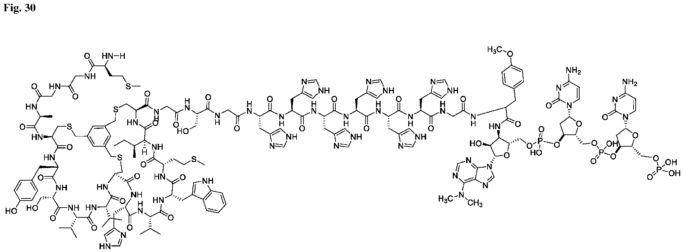

**Fig. 30****:** Structure of cyclic peptide with the sequence "MGGACYSVVCHVMWICGSGHHHHHHG-puromycin-cytosine-cytosine (SEQ ID NO: 14).

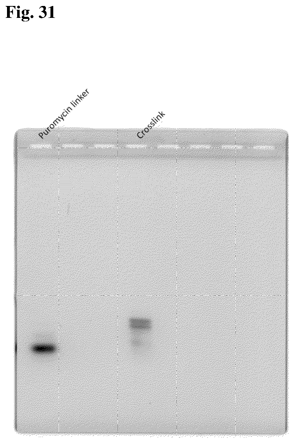

**Fig. 31****:** 1.5% agarose gel electrophoresis of puromycin linker (PuL) and PuL-mRNA crosslink.

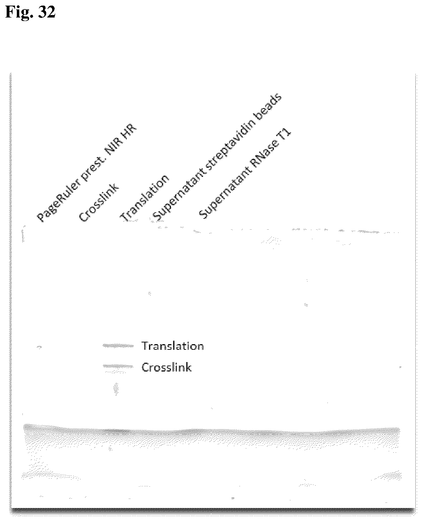

**Fig. 32****:** : 8M urea 10% SDS - page of the translation.

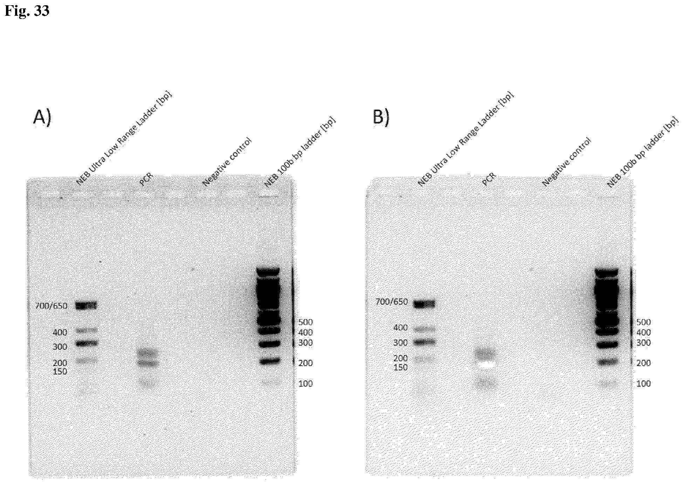

**Fig. 33****:** 2% agarose gel electrophoresis of the PCR. Markers are NEB Ultra Low Range Ladder and NEB 100 bp ladder.

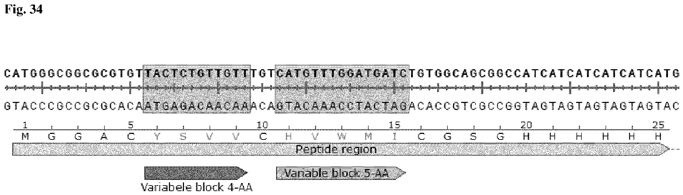

**Fig. 34****:** Sequence of HQ0318 (SEQ ID NO: 18) and peptide sequence encoded (SEQ ID NO:19).

### EXAMPLES

### A: Synthesis of 5'- Azid (Azido-PEG4)-tctctc55cc-Puromycin-3'

### 5= Spacer 18 (see table below)

The "puromycin segment without fluorescence label" was obtained by standard phosphoramidite solid phase synthesis, e.g. using the building blocks shown below.

| **Trivial name** | **Nomenclature** | **Manufacturer** | **#cat.** |
|---|---|---|---|
| Puromycin-CPG | 5'-Dimethoxytrityl-N-trifluoroacetyl-puromycin,2'-succinoyl-long chain alkylamino-CPG | Glen Research | 20-4040 |
| Spacer Phosphorami dite **18** | 18-O-Dimethoxytritylhexaethyleneglycol,1-[(2-cyanoethyl)-(N,N-diisopropyl)]-phosphoramidite | Glen Research | 10-1918 |
| 5'-Amino-Modifier C5-TFA | 5-(Trifluoroacetylamino)-pentyl-(2-cyanoethyl)-(N,N-diisopropyl)-phosphoramidite | Nucleosyn | 174224 |
| Azido-PEG4 NHS-Ester | 3-(Azidotetra(ethyleneoxy))propionic acid succinimidyl ester, NHS-Peg4-Azide, Polyethylene glycol | Broadpharm | BP-20518 |
| DMT-dA(bz) Phosphorami dite | N6-Benzoyl-5'-O-(4,4'-dimethoxytrityl)-2'-deoxyadenosine-3'-O-[O-(2-cyanoethyl)-N,N'-diisopropylphosphoramidite] | Merck | A1110 30 |
| DMT-dG(dmf) Phosphorami dite | N2-Dimethylformamidine-5'-O-(4,4'-dimethoxytrityl)-2'-deoxyguanosine-3'-O-[O-(2-cyanoethyl)-N,N'-diisopropylphosphoramidite] | Merck | G1150 30 |
| DMT-dC(ac) Phosphorami dite | N4-Acetyl-5'-O-(4,4'-dimethoxytrityl)-2'-deoxycytidine-3'-O-[O-(2-cyanoethyl)-N,N'-diisopropylphosphoramidite] | Merck | C11303 0 |
| DMT-dT Phosphorami dite | 5'-O-(4,4'-dimethoxytrityl)-2'-deoxythymidine-3'-O-[O-(2-cyanoethyl)-N,N'-diisopropylphosphoramidite] | Merck | T11103 0 |

In a first step, starting with Puromycin-CPG the modified oligonucleotide 5' - AminoC5-tctctc55cc-Puromycin-3' (5= Spacer 18) was obtained by phosphoramidite solid phase synthesis following standard synthesis cycles and procedures:
The following specific conditions were used:
   - Detritylation (3% trichloroacetic acid in dichloromethane)
   - Coupling (0.3 M 5-(Ethylthio)-1H-tetrazole in acetonitrile/0.1 M phosphoramidite in acetonitrile)
   - Capping (Cap A: 10% acetic anhydride in tetrahydrofurane /lutidine, Cap B: 16% N-methylimidazole in tetrahydrofurane)
   - Oxidation (0.02 M iodine in tetrahydrofurane / water / pyridine )

For spacer phosphoramidite 18 and 5'-amino-modifier-C5-TFA phosphoramidite a prolonged coupling time of 600 seconds was applied.

Deprotection and cleavage from the solid support was carried out in concentrated aqueous ammonia solution at 60 °C overnight. After evaporation of the ammonia solution, the oligonucleotide was dissolved in water and isolated by standard ethanol precipitation.

In a second step the obtained oligonucleotide was reacted with azido-PEG4-NHS-ester:
The 5'-amino-oligonucleotide was dissolved in sodium bicarbonate buffer pH 8.3 (10 OD260 in 200 µl) and a solution of azido-PEG4-NHS-ester (10 equ.) in dimethylformamide (200 µl) was added, mixed well and left overnight.

After ethanol precipitation the azide labeled oligonucleotide was purified by HPLC (RP-18 column, triethylammonium acetate/acetonitrile gradient).

### B: Synthesis of 5'-Biotin-TEG-aa6aa7ttcca5gccgccccccg8cct-3' (SEQ ID NO:15)

*5= CNVK*
*6= rG*
*7= Fluorescein-dT*
*8= DBCO-C6-dT*

The biotin segment with fluorescence label was obtained by standard phosphoramidite solid phase synthesis, e.g. using the building blocks shown below.

| **Trivial name** | **Nomenclature** | **Manufacturer** | **#cat.** |
|---|---|---|---|
| BiotinTEG phosphoram idite | 1-Dimethoxytrityloxy-3-O-(N-biotinyl-3-aminopropyl)-triethyleneglycolyl-glyceryl-2-O-(2-cyanoethyl)-(N,N-diisopropyl)-phosphoramidite | Glen Research | 10-1955 |
| Fluorescein -dT Phosphora midite | 5'-Dimethoxytrityloxy-5-[N-((3',6'-dipivaloylfluoresceinyl)-aminohexyl)-3-acrylimido]-2'-deoxyUridine-3'-[(2-cyanoethyl)-(N,N-diisopropyl)]-phosphoramidite | Nucleosyn | 182624 |
| Amino-Modifier C6 dT | 5'-Dimethoxytrityl-5-[N-(trifluoroacetylaminohexyl)-3-acrylimido]-2'-deoxyUridine,3'-[(2-cyanoethyl)-(N,N-diisopropyl)]-phosphoramidite | Nucleosyn | 175624 |
| 3-Cyanovinyl carbazole Phosphora midite (CNVK) | 5'-O-(4,4'-Dimethoxytrityl)-1'-(3-cyanovinylcarbazol-9-yl)-2'-deoxy-β-D-ribofuranosyl- 3'-[(2-cyanoethyl)-(N,N-diisopropyl)]-phosphoramidite | Glen Research | 10-4960 |
| iPr-Pac-G-CE Phosphora midite | 5'-Dimethoxytrityl-N-p-isopropylphenoxyacetyl-Guanosine,2'-O-TBDMS-3'-[(2-cyanoethyl)-(N,N-diisopropyl)] -phosphoramidite | Glen Research | 10-3021 |
| DBCO-C6 NHS-Ester | 1-{[6-(11,12-didehydrodibenzo[b,f]azocin-5(6H)-yl)-6-oxohexanoyl]oxy}-2,5-pyrrolidinedione | Broadpharm | BP-22447 |
| Pac-dA-CE Phosphora midite | 5'-Dimethoxytrityl-N-phenoxyacetyl-2'-deoxyAdenosine,3'-[(2-cyanoethyl)-(N,N-diisopropyl)]-phosphoramidite | Glen Research | 10-1601 |
| iPr-Pac-dG-CE | 5'-Dimethoxytrityl-N-p-isopropyl-phenoxyacetyl-2'-deoxyGuanosine,3'-[(2-cyanoethyl)-(N,N-diisopropyl)] -phosphoramidite | Glen Research | 10-1621 |
| Phosphora midite | | | |
| DMT-dC(ac) Phosphora midite | N4-Acetyl-5'-O-(4,4'-dimethoxytrityl)-2'-deoxycytidine-3'-O-[O-(2-cyanoethyl)-N,N'-diisopropylphosphoramidite] | Merck | C 113030 |
| DMT-dT Phosphora midite | 5'-O-(4,4'-dimethoxytrityl)-2'-deoxythymidine-3'-O-[O-(2-cyanoethyl)-N,N'-diisopropylphosphoramidite] | Merck | T111030 |

The modified oligonucleotide 5'-biotin-TEG-aa6aa7ttcca5gccgccccccg8cct-3' (5= CNVK, 6= rG, 7= fluorescein-dT, 8= amino-modifier-C6-dT, SEQ ID NO:15) was obtained by phosphoramidite solid phase synthesis following standard synthesis cycles and procedures:
The following specific conditions were used:
- Detritylation: (3% Trichloroacetic acid in Dichloromethane)
- Coupling (0.3 M 5-(ethylthio)-1H-tetrazole in acetonitrile/0.1 M phosphoramidite in acetonitrile)
- Capping (Cap A: 5% phenoxyacetic anhydride in tetrahydrofurane/pyridine, Cap B: 16% N-methylimidazole in tetrahydrofurane)
- Oxidation (0.02 M iodine in tetrahydrofurane/water/pyridine )

For the internal modifications CNVK, rG, fluorescein-dT, amino-modifier-C6-dT and the 5'-modification biotin-TEG a prolonged coupling time of 600 seconds was applied. The synthesis was carried out as a DMT-ON synthesis (the final DMT group was not automatically cleaved on the DNA-synthesizer).

Deprotection and cleavage from the solid support was carried out in 0.05M potassium carbonate in methanol for 4 hours at room temperature. After evaporation, the oligonucleotide was dissolved in a mixture of 1-methyl-2-pyrrolidinone/triethylamine/triethylamine trihydrofluoride 2/1/1.5 (v/v/v) and incubated at 60 °C for 2 hours to cleave the TBDMS protecting group from the rG building block.

After cooling, the solution was neutralized with 1.4 M ammonium bicarbonate solution and directly applied to a RP-18 HPLC column for purification (triethylammonium acetate/acetonitrile gradient). The purified oligonucleotide was detritylated (10% acetic acid, 45 min, room temperature) and isolated by ethanol precipitation.

In a second step the obtained oligonucleotide was reacted with DBCO-C6-NHS-ester:
The oligonucleotide with internal amino-modifier C6-dT was dissolved in sodium bicarbonate buffer pH 8.3 (10 OD260 in 200 µl) and a solution of DBCO-C6-NHS-ester (10 equ.) in dimethylformamide (200 µl) was added, mixed well and left overnight.

After standard ethanol precipitation the DBCO labeled oligonucleotide was purified by HPLC (RP-18 column, triethylammonium acetate/acetonitrile gradient).

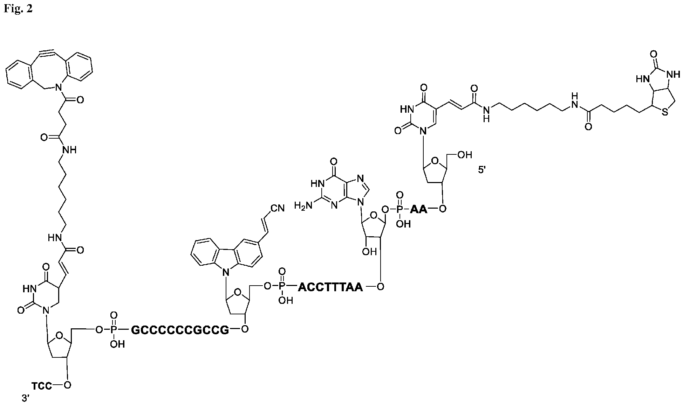

### 1. Strain-promoted azide-alkyne cycloaddition between FAM-puromycin segment (see Fig. 1) and dT-Biotin (see Fig. 2)

### 1.1 Synthesis of FAM puromycin segment

The FAM puromycin segment can be obtained by phosphoramidite solid phase synthesis, e.g. using the building blocks shown below and in accordance with the synthetic details of examples A and B above.

| **Trivial name** | **Nomenclature** | **Manufacturer** | **#cat.** |
|---|---|---|---|
| Spacer Phosphoramidite 18 | 18-O-Dimethoxytritylhexaethyleneglycol,1-[(2-cyanoethyl)-(N,N-diisopropyl)]-phosphoramidite | Glen Research | 125607-09-2 |
| Fluorescein-dT Phosphoramidite (FAM dT) | 5'-Dimethoxytrityloxy-5-[N-((3',6'-dipivaloylfluoresceinyl)-aminohexyl)-3-acrylimido]-2'-deoxyUridine-3'-[(2-cyanoethyl)-(N,N-diisopropyl)]-phosphoramidite | Glen Research | 289712-99-8 |
| Puromycin-CPG | 5'-Dimethoxytrityl-N-trifluoroacetyl-puromycin,2'-succinoyl-long chain alkylamino-CPG | Glen Research | |
| 5'-Amino-Modifier C6 | 6-(4-Monomethoxytritylamino)hexyl-(2-cyanoethyl)-(N,N-diisopropyl)-phosphoramidite | Glen Research | 114616-27-2 |
| Azido-dPEG^{®}₄-NHS ester | 2,5-dioxopyrrolidin-1-yl 1-azido-3,6,9,12-tetraoxapentadecan-15-oate | Sigma Aldrich | QBD10501 |
| dA-CE Phosphoramidite | 5'-Dimethoxytrityl-N-benzoyl-2'-deoxyAdenosine,3'-[(2-cyanoethyl)-(N,N-diisopropyl)]-phosphoramidite | Glen Research | 98796-53-3 |
| dG-CE Phosphoramidite | 5'-Dimethoxytrityl-N-isobutyryl-2'-deoxyGuanosine,3'-[(2-cyanoethyl)-(N,N-diisopropyl)]-phosphoramidite | Glen Research | 93183-15-4 |
| dC-CE Phosphoramidite | 5'-Dimethoxytrityl-N-benzoyl-2'-deoxyCytidine,3'-[(2-cyanoethyl)-(N,N-diisopropyl)]-phosphoramidite | Glen Research | 102212-98-6 |
| dT-CE Phosphoramidite | 5'-Dimethoxytrityl-2'-deoxyThymidine,3'-[(2-cyanoethyl)-(N,N-diisopropyl)]-phosphoramidite | Glen Research | 98796-51-1 |

### 1.2 Synthesis of Biotin segment with dT biotin

The Biotin segment can be obtained by phosphoramidite solid phase synthesis, e.g. using the building blocks shown below, and in accordance with the synthetic details of examples A and B above.

| **Trivial name** | **Nomenclature** | **Manufacturer** | **#cat.** |
|---|---|---|---|
| Biotin-dT Phosphoramidite | 5'-Dimethoxytrityloxy-5-[N-((4-t-butylbenzoyl)-biotinyl)-aminohexyl)-3-acrylimido]-2'-deoxyUridine-3'-[(2-cyanoethyl)-(N,N-diisopropyl)]-phosphoramidite | Glen Research | 198080-40-9 |
| Fmoc Amino-Modifier C6 dT | 5'-Dimethoxytrityl-5-[N-((9-fluorenylmethoxycarbonyl)-aminohexyl)-3-acrylimido]-2'-deoxyUridine,3'-[(2-cyanoethyl)-(N,N-diisopropyl)]-phosphoramidite | Glen Research | 2087458-62-4 |
| Ac-G-CE Phosphoramidite | 5'-Dimethoxytrityl-N-acetyl-Guanosine,2'-O-TBDMS-3'-[(2-cyanoethyl)-(N,N-diisopropyl)]-phosphoramidite | Glen Research | 944138-03-8 |
| 3-Cyanovinylcarbazole Phosphoramidite (^{CNV}K) | 5'-O-(4,4'-Dimethoxytrityl)-1'-(3-cyanovinylcarbazol-9-yl)-2'-deoxy-β-D-ribofuranosyl- 3'-[(2-cyanoethyl)-(N,N-diisopropyl)]-phosphoramidite | Glen Research | |
| Dibenzocyclooctin-*N-*hydroxysuccinimidylester | | Sigma Aldrich | 761524 |
| dA-CE Phosphoramidite | 5'-Dimethoxytrityl-N-benzoyl-2'-deoxyAdenosine,3'-[(2-cyanoethyl)-(N,N-diisopropyl)]-phosphoramidite | Glen Research | 98796-53-3 |
| dG-CE Phosphoramidite | 5'-Dimethoxytrityl-N-isobutyryl-2'-deoxyGuanosine,3'-[(2-cyanoethyl)-(N,N-diisopropyl)]-phosphoramidite | Glen Research | 93183-15-4 |
| dC-CE Phosphoramidite | 5'-Dimethoxytrityl-N-benzoyl-2'-deoxyCytidine,3'-[(2-cyanoethyl)-(N,N-diisopropyl)]-phosphoramidite | Glen Research | 102212-98-6 |
| dT-CE Phosphoramidite | 5'-Dimethoxytrityl-2'-deoxvThvmidine,3'-[(2-cyanoethyl)-(N,N-diisopropyl)]-phosphoramidite | Glen Research | 98796-51-1 |

### 1.3 Cycloaddition between FAM puromycin segment and Biotin segment with dT biotin

The 30 nmol biotin-segment with dT biotin (280.5 µg) were dissolved in 60 µL nuclease free H₂O. As well, the 34 nmol FAM puromycin-segment (155.6 µg) were dissolved in 69 µL nuclease free H₂O. Resulting from the dissolution steps, both ssDNA macromolecules dwelled in 500 µM solution of H₂O. For a first reaction, half of the probes were merged in a 2:1 ratio (FAM puromycin to biotin-segment with dT biotin). Therefore, 15 µL biotin-segment with dT biotin (500 µM) were pipetted into 30 µL FAM puromycin-segment (500 µM) and incubated first for 3 h at 23 °C and 350 rpm, than up to 24 h at 4 °C and 350 rpm.

After 24 h of incubation, about 10 µL of the reaction volume were injected into HPLC for analysis and purification. The HPLC was conducted using a "Machery Nagel EC 250/4.6 Nulceodur 300-5 C18ec" column (250 mm length, 4.6 mm thickness) driven with 1 mL min⁻¹ flow rate. A 0.1 M solution of triethylamine acetate (TEAA) in H₂O was used as solvent A and acetonitrile as solvent B. For elution a gradient from 90 - 70% A was driven over 80 min (0.25 percent per minute).

The HPLC of the reaction is shown in Fig. 3 and Fig. 3a.

### 2. Strain-promoted azide-alkyne cycloaddition between FAM-puromycin segment (see Fig. 1 and example 1.1) and TEG-Biotin (see Fig. 4)

### 2.1 Synthesis of TEG-Biotin segment

The TEG-Biotin segment can be obtained by phosphoramidite solid phase synthesis, e.g. using the building blocks shown below, and in accordance with the synthetic details of examples A and B above.

| **Trivial name** | **Nomenclature** | **Manufacturer** | **#cat.** |
|---|---|---|---|
| BiotinTEG Phosphoramidite | 1-Dimethoxytrityloxy-3-O-(N-biotinyl-3-aminopropyl)-triethyleneglycolyl-glyceryl-2-O-(2-cyanoethyl)-(N,N-diisopropyl)-phosphoramidite | Glen Research | 198080-44-3 |
| Fmoc Amino-Modifier C6 dT | 5'-Dimethoxytrityl-5-[N-((9-fluorenylmethoxycarbonyl)-aminohexyl)-3-acrylimido]-2'-deoxyUridine,3'-[(2-cyanoethyl)-(N,N-diisopropyl)]-phosphoramidite | Glen Research | 2087458-62-4 |
| Ac-G-CE Phosphoramidite | 5'-Dimethoxytrityl-N-acetyl-Guanosine,2'-O-TBDMS-3'-[(2-cyanoethyl)-(N,N-diisopropyl)]-phosphoramidite | Glen Research | 944138-03-8 |
| 3-Cyanovinylcarbazole Phosphoramidite (^{CNV}K) | 5'-O-(4,4'-Dimethoxytrityl)-1'-(3-cyanovinylcarbazol-9-yl)-2'-deoxy-β-D-ribofuranosyl- 3'-[(2-cyanoethyl)-(N,N-diisopropyl)]-phosphoramidite | Glen Research | |
| Dibenzocyclooctin-*N-*hydroxysuccinimidylester | | Sigma Aldrich | 761524 |
| dA-CE Phosphoramidite | 5'-Dimethoxytrityl-N-benzoyl-2'-deoxyAdenosine,3'-[(2-cyanoethyl)-(N,N-diisopropyl)]-phosphoramidite | Glen Research | 98796-53-3 |
| dG-CE Phosphoramidite | 5'-Dimethoxytrityl-N-isobutyryl-2'-deoxyGuanosine,3'-[(2-cyanoethyl)-(N,N-diisopropyl)]-phosphoramidite | Glen Research | 93183-15-4 |
| dC-CE Phosphoramidite | 5'-Dimethoxytrityl-N-benzoyl-2'-deoxyCytidine,3'-[(2-cyanoethyl)-(N,N-diisopropyl)]-phosphoramidite | Glen Research | 102212-98-6 |
| dT-CE Phosphoramidite | 5'-Dimethoxytrityl-2'-deoxyThymidine,3'-[(2-cyanoethyl)-(N,N-diisopropyl)]-phosphoramidite | Glen Research | 98796-51-1 |

### 2.2 Cycloaddition between FAM puromycin segment and TEG Biotin segment

The 369.4 µg (82.4 nmol) FAM puromycin segment (see 1.1.) were dissolved in 164 µL nuclease free ddH₂O to resolve in a concentration of 500 µM. The 230.8 µg (25 nmol) dT biotin segment were dissolved in 50 µL nuclease free ddH₂O to resolve in 500 µM concentration. For SPAAC reaction, 20 µL TEG
biotin segment [500 µM] and 40 µL FAM puromycin segment [500 µM] were mixed and incubated for 24 h at 8 °C (light exclosure). Reaction control was performed after 24 h by HPLC. To identify the reaction starting materials in the reaction control, 2 µL each (puromycin and biotin segment) were diluted in 8 µL ddH₂O to 100 µM, before they were measured in HPLC as reference. At the beginning of every purification, a blanc (not shown in protocol data) and an analytical run (2 µL reaction volume in 8 µL ddH₂O) were conducted.
The HPLC chromatogram is shown in Fig. 5. For the HPLC experiments, the following conditions were maintained. Solvent A: 0.1 M TEAA in H₂O, so
lvent B: Acetonitrile. Gradient: 87% - 80% A over 30 min at 1 mL min⁻¹. Column: Machery Nagel NUCLEODUR 300-5 C18 ec, 5 µm, 250x4.6 mm.

The Analytical run in Fig. 5 shows 2 peaks - one at 11 and another at 13 min. the 11 min peaks matches the puromycin segment reference, while the 13 min peak is the product. The biotin segment's peak at 21 min does not appear neither in the analytical, nor in the preparative run. The preparative run shows a "pack shift" of the puromycin segment and the Puromycin-linker (product) related peak of about 2 min. The void signal of the column at 4 min stays unaffected from the shift. This intends that the shift is caused by the solvent gradient (blanc run and 10 void volumes of equilibration before and column flush with 100% solvent B after each run exclude the possibility of ghost peaks) and is a systematic mistake with the HPLC itself as origin.

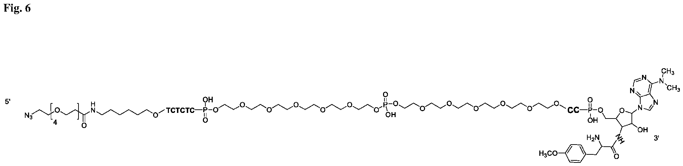

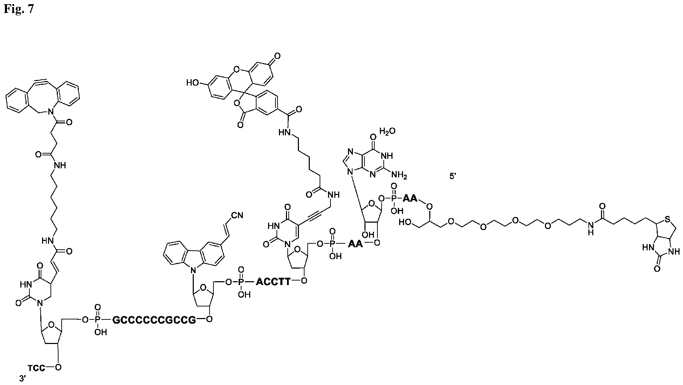

### 3. Strain-promoted azide-alkyne cycloaddition between Puromycin segment without fluorescence label (see Fig. 6) and Biotin segment with TEG-Biotin and FAM label (see Fig. 7)

### 3.1 Synthesis of puromycin segment without fluorescence label

The Puromycin segment can be obtained by phosphoramidite solid phase synthesis, e.g. using the building blocks shown below, and in accordance with the synthetic details of examples A and B above.

| **Trivial name** | **Nomenclature** | **Manufacturer** | **#cat.** |
|---|---|---|---|
| Spacer Phosphoramidite 18 | 18-O-Dimethoxytritylhexaethyleneglycol,1-[(2-cyanoethyl)-(N,N-diisopropyl)]-phosphoramidite | Glen Research | 125607-09-2 |
| Puromycin-CPG | 5'-Dimethoxytrityl-N-trifluoroacetyl-puromycin,2'-succinoyl-long chain alkylamino-CPG | Glen Research | |
| 5'-Amino-Modifier C6 | 6-(4-Monomethoxytritylamino)hexyl-(2-cyanoethyl)-(N,N-diisopropyl)-phosphoramidite | Glen Research | 114616-27-2 |
| Azido-dPEG^{®}₄-NHS ester | 2,5-dioxopyrrolidin-1-yl 1-azido-3,6,9,12-tetraoxapentadecan-15-oate | Sigma Aldrich | QBD10501 |
| dA-CE Phosphoramidite | 5'-Dimethoxytrityl-N-benzoyl-2'-deoxyAdenosine,3'-[(2-cyanoethyl)-(N,N-diisopropyl)]-phosphoramidite | Glen Research | 98796-53-3 |
| dG-CE Phosphoramidite | 5'-Dimethoxytrityl-N-isobutyryl-2'-deoxyGuanosine,3'-[(2-cyanoethyl)-(N,N-diisopropyl)]-phosphoramidite | Glen Research | 93183-15-4 |
| dC-CE Phosphoramidite | 5'-Dimethoxytrityl-N-benzoyl-2'-deoxyCytidine,3'-[(2-cyanoethyl)-(N,N-diisopropyl)]-phosphoramidite | Glen Research | 102212-98-6 |
| dT-CE Phosphoramidite | 5'-Dimethoxytrityl-2'-deoxyThymidine,3'-[(2-cyanoethyl)-(N,N-diisopropyl)]-phosphoramidite | Glen Research | 98796-51-1 |

### 3.2 Synthesis of Biotin segment with TEG Biotin and FAM label

The Puromycin segment with FAM label can be obtained by phosphoramidite solid phase synthesis, e.g. using the building blocks shown below, and in accordance with the synthetic details of examples A and B above.

| **Trivial name** | **Nomenclature** | **Manufacturer** | **#cat.** |
|---|---|---|---|
| BiotinTEG Phosphoramidite | 1-Dimethoxytrityloxy-3-O-(N-biotinyl-3-aminopropyl)-triethyleneglycolyl-glyceryl-2-O-(2-cyanoethyl)-(N,N-diisopropyl)-phosphoramidite | Glen Research | 198080-44-3 |
| Fmoc Amino-Modifier C6 dT | 5'-Dimethoxytrityl-5-[N-((9-fluorenylmethoxycarbonyl)-aminohexyl)-3-acrylimido]-2'-deoxyUridine,3'-[(2-cyanoethyl)-(N,N-diisopropyl)]-phosphoramidite | Glen Research | 2087458-62-4 |
| Ac-G-CE Phosphoramidite | 5'-Dimethoxytrityl-N-acetyl-Guanosine,2'-O-TBDMS-3'-[(2-cyanoethyl)-(N,N-diisopropyl)]-phosphoramidite | Glen Research | 944138-03-8 |
| 3-Cyanovinylcarbazole Phosphoramidite (^{CNV}K) | 5'-O-(4,4'-Dimethoxytrityl)-1'-(3-cyanovinylcarbazol-9-yl)-2'-deoxy-β-D-ribofuranosyl- 3'-[(2-cyanoethyl)-(N,N-diisopropyl)]-phosphoramidite | Glen Research | |
| Fluorescein-dT Phosphoramidite (FAM dT) | 5'-Dimethoxytrityloxy-5-[N-((3',6'-dipivaloylfluoresceinyl)-aminohexyl)-3-acrylimido]-2'-deoxyUridine-3'-[(2-cyanoethyl)-(N,N-diisopropyl)]-phosphoramidite | Glen Research | 289712-99-8 |
| Dibenzocyclooctin-N-hydroxysuccinimidylester | | Sigma Aldrich | 761524 |
| dA-CE Phosphoramidite | 5'-Dimethoxytrityl-N-benzoyl-2'-deoxyAdenosine,3'-[(2-cyanoethyl)-(N,N-diisopropyl)]-phosphoramidite | Glen Research | 98796-53-3 |
| dG-CE Phosphoramidite | 5'-Dimethoxytrityl-N-isobutyryl-2'-deoxyGuanosine,3'-[(2-cyanoethyl)-(N,N-diisopropyl)]-phosphoramidite | Glen Research | 93183-15-4 |
| dC-CE Phosphoramidite | 5'-Dimethoxytrityl-N-benzoyl-2'-deoxyCytidine,3'-[(2-cyanoethyl)-(N,N-diisopropyl)]-phosphoramidite | Glen Research | 102212-98-6 |
| dT-CE Phosphoramidite | 5'-Dimethoxytrityl-2'-deoxyThymidine,3'-[(2-cyanoethyl)-(N,N-diisopropyl)]-phosphoramidite | Glen Research | 98796-51-1 |

### 3.3. Cycloaddition between puromycin segment without fluorescence label and Biotin segment with TEG Biotin and FAM label Data and solvent volume of the Pu-Linker subsegments.

**Table: Data and solvent volume of the Pu-Linker subsegments.**

| **Segment** | **m [µg]** | **n [nmol]** | **M [g mol⁻¹]** | **V (H₂O) [µL]** | **c [µM]** |
|---|---|---|---|---|---|
| Puromycin-S. | 1588 | 400 | 3970 | 800 | 500 |
| Biotin-S. | 1461 | 150 | 9746 | 300 | 500 |

100 µL biotin segment[500 µM] and 200 µL puromycin segment [500 µM] were mixed and incubated for 3 h at 37 °C and 15 rpm (over and under rotation.

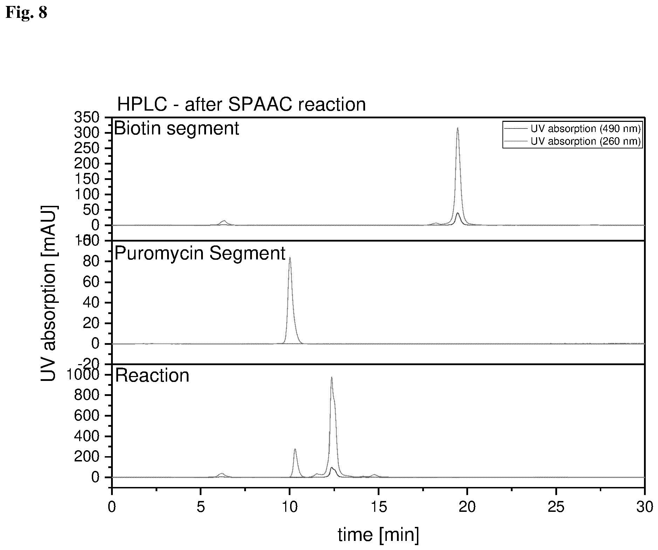

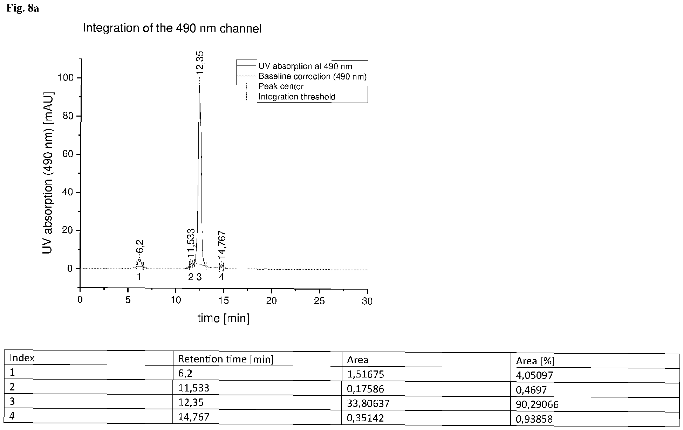

For HPLC, a Machery-Nagel "NUCLEODUR 300-5 C18 ec, 5 µm, 250x4.6 mm" HPLC column was used at a flow rate of 1 mL min⁻¹. Solvent A: 0.1 M TEAA in H₂O, solvent B: acetonitrile. The gradient from 84% to 70% 0.1 M TEAA was driven over 30 min. For the analytical run, as well as for the starting materials, 2 µL substance were diluted in 18 µL H₂O. The preparative runs were conducted using 20 µL pure substance. The HPLC results are given in Fig. 8 and 8a.

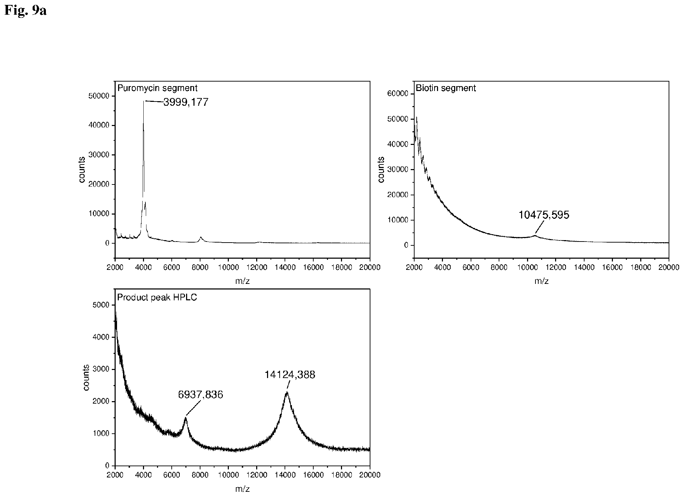

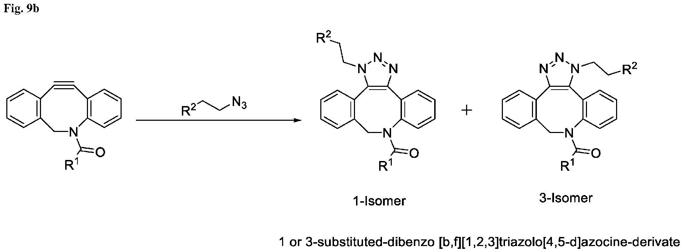

MALDI-TOF spectra of the product related HPCL peak and the puromycin and biotin segment were recorded (see Fig. 9) The single peak appearing with a maximum at a m/z value 14124 shows that there are no byproducts in the HPLC product fraction. The peak appearing at a m/z value of 6937 is the [m+2H]²⁺ species of the puromycin linker and therefore is a secondary peak of the peak at 14124. According to the HPLC record and integration of the 490 nm channel the reaction conversion is 99%.

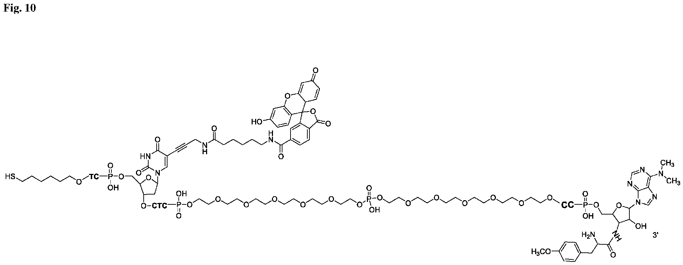

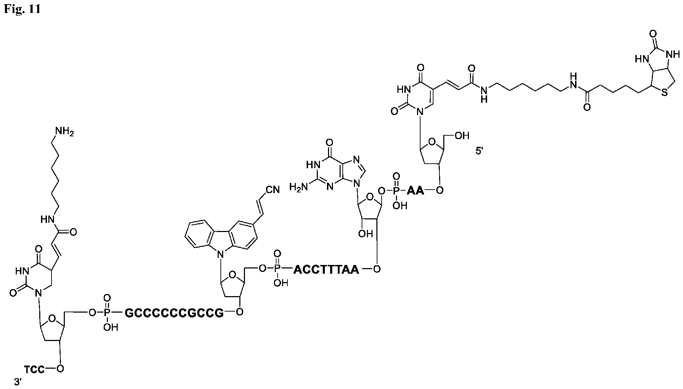

### 4. Comparative example: Michael-Addition between Puromycin segment with 5' Thiol modifier (see Fig. 10) and Biotin segment with dT-C6-amino modifier (see Fig. 11)

**Table: Concentration of the Pu-linker subsegments before reaction.**

| **Segment** | **n [nmol]** | **m [µL]** | **V [µL]** | **c [mM]** |
|---|---|---|---|---|
| Puromycin | 14.8 | 62.4 | 14 | 1 |
| Biotin | 12.5 | 113.3 | 25 | 0.5 |

### 4.1 Synthesis of Puromycin segment with 5' Thiol modifier

The Puromycin segment with Thiol modifier can be obtained by phosphoramidite solid phase synthesis, e.g. using the building blocks shown below, and in accordance with the synthetic details of examples A and B above.

| **Trivial name** | **Nomenclature** | **Manufacturer** | **#cat.** |
|---|---|---|---|
| Spacer Phosphoramidite 18 | 18-O-Dimethoxytritylhexaethyleneglycol,1-[(2-cyanoethyl)-(N,N-diisopropyl)]-phosphoramidite | Glen Research | 125607-09-2 |
| Fluorescein-dT Phosphoramidite (FAM dT) | 5'-Dimethoxytrityloxy-5-[N-((3',6'-dipivaloylfluoresceinyl)-aminohexyl)-3-acrylimido]-2'-deoxyUridine-3'-[(2-cyanoethyl)-(N,N-diisopropyl)]-phosphoramidite | Glen Research | 289712-99-8 |
| Puromycin-CPG | 5'-Dimethoxytrityl-N-trifluoroacetyl-puromycin,2'-succinoyl-long chain alkylamino-CPG | Glen Research | |
| 5'-Thiol-Modifier C6 | S-Trityl-6-mercaptohexyl-1-[(2-cyanoethyl)-(N,N-diisopropyl)]-phosphoramidite | Glen Research | 116919-17-6 |
| dA-CE Phosphoramidite | 5'-Dimethoxytrityl-N-benzoyl-2'-deoxyAdenosine,3'-[(2-cyanoethyl)-(N,N-diisopropyl)]-phosphoramidite | Glen Research | 98796-53-3 |
| dG-CE Phosphoramidite | 5'-Dimethoxytrityl-N-isobutyryl-2'-deoxyGuanosine,3'-[(2-cyanoethyl)-(N,N-diisopropyl)]-phosphoramidite | Glen Research | 93183-15-4 |
| dC-CE Phosphoramidite | 5'-Dimethoxytrityl-N-benzoyl-2'-deoxyCytidine,3'-[(2-cyanoethyl)-(N,N-diisopropyl)]-phosphoramidite | Glen Research | 102212-98-6 |
| dT-CE Phosphoramidite | 5'-Dimethoxytrityl-2'-deoxyThymidine,3'-[(2-cyanoethyl)-(N,N-diisopropyl)]-phosphoramidite | Glen Research | 98796-51-1 |

### 4.2 Synthesis of Biotin segment with dT-C6-amino modifier

The Biotin segment with can be obtained by phosphoramidite solid phase synthesis, e.g. using the building blocks shown below, and in accordance with the synthetic details of examples A and B above..

| **Trivial name** | **Nomenclature** | **Manufacturer** | **#cat.** |
|---|---|---|---|
| Biotin-dT Phosphoramidite | 5'-Dimethoxytrityloxy-5-[N-((4-t-butylbenzoyl)-biotinyl)-aminohexyl)-3-acrylimido]-2'- | Glen Research | 198080-40-9 |
| | deoxyUridine-3'-[(2-cyanoethyl)-(N,N-diisopropyl)]-phosphoramidite | | |
| Fmoc Amino-Modifier C6 dT | 5'-Dimethoxytrityl-5-[N-((9-fluorenylmethoxycarbonyl)-aminohexyl)-3-acrylimido]-2'-deoxyUridine,3'-[(2-cyanoethyl)-(N,N-diisopropyl)]-phosphoramidite | Glen Research | 2087458-62-4 |
| Ac-G-CE Phosphoramidite | 5'-Dimethoxytrityl-N-acetyl-Guanosine,2'-O-TBDMS-3'-[(2-cyanoethyl)-(N,N-diisopropyl)]-phosphoramidite | Glen Research | 944138-03-8 |
| 3-Cyanovinylcarbazole Phosphoramidite (^{CNV}K) | 5'-O-(4,4'-Dimethoxytrityl)-1'-(3-cyanovinylcarbazol-9-yl)-2'-deoxy-β-D-ribofuranosyl- 3'-[(2-cyanoethyl)-(N,N-diisopropyl)]-phosphoramidite | Glen Research | |
| dA-CE Phosphoramidite | 5'-Dimethoxytrityl-N-benzoyl-2'-deoxyAdenosine,3'-[(2-cyanoethyl)-(N,N-diisopropyl)]-phosphoramidite | Glen Research | 98796-53-3 |
| dG-CE Phosphoramidite | 5'-Dimethoxytrityl-N-isobutyryl-2'-deoxyGuanosine,3'-[(2-cyanoethyl)-(N,N-diisopropyl)]-phosphoramidite | Glen Research | 93183-15-4 |
| dC-CE Phosphoramidite | 5'-Dimethoxytrityl-N-benzoyl-2'-deoxyCytidine,3'-[(2-cyanoethyl)-(N,N-diisopropyl)]-phosphoramidite | Glen Research | 102212-98-6 |
| dT-CE Phosphoramidite | 5'-Dimethoxytrityl-2'-deoxyThymidine,3'-[(2-cyanoethyl)-(N,N-diisopropyl)]-phosphoramidite | Glen Research | 98796-51-1 |

### 4.3. Michael-Addition between Puromycin segment with 5' Thiol modifier (see Fig. 10) and Biotin segment with dT-C6-amino modifier

10 µL biotin segment were solved in 50 µL phosphate buffer [200 mM, pH 7.2]. Meanwhile, 2 mg EMCS were solved in 65 µL DMF and 10 µL of the solution were added to the biotin segment. The mixture was incubated for 30 min at 37 °C. For ethanol precipitation 7 µL (0.1 volume) sodium acetate (NaCH₃COO, 3 M) and 140 µL -80 °C ethanol (>95%) were added. The solution was incubated for 2 h at -80 °C. Thereafter, the precipitation was centrifuged for 20 min at 15 000 rcf and 4 °C. The supernatant was discarded and the pellet was dissolved in 55 µL phosphate buffer [200 mM, pH 7.2]. 5 µL were taken for HPLC and the remaining 50 µL were mixed with 10 µL of the puromycin segment. The reaction was incubated over-night at 4 °C and 15 rpm over and under rotation.

For HPLC, a Machery-Nagel "NUCLEODUR 300-5 C18 ec, 5 µm, 250x4.6 mm" HPLC column was used at a flow rate of 1 mL min⁻¹. Solvent A: 0.1 M TEAA in H₂O, solvent B: acetonitrile. The gradient from 84% to 70% 0.1 M TEAA was driven over 30 min. For the analytical run, as well as for the starting materials, 2 µL substance were diluted in 18 µL H₂O. the preparative runs were conducted using 20 µL pure substance.

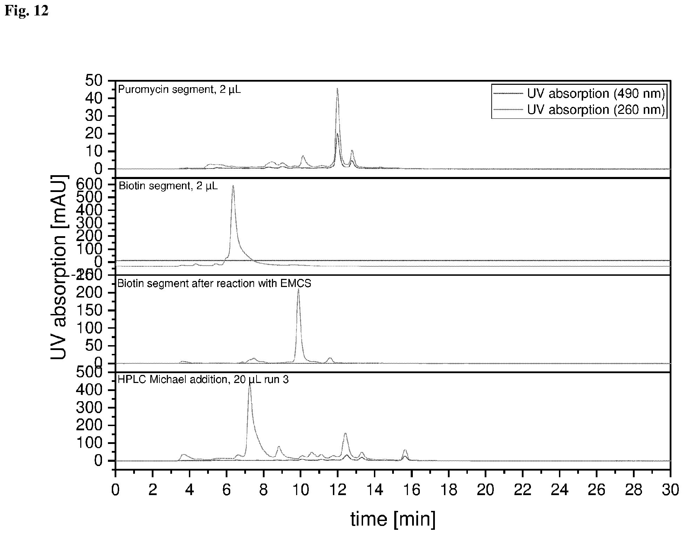

Fig. 12shows the lineup between the Michael addition and its starting materials. Most interesting is the shift of the biotin segment's peak. Appearing at 6.35 min in the starting material HPLC it shifts to 9.88 min after the reaction with EMCS. This shift intends that the reaction was successful. The Reaction control took place after the ethanol precipitation. During the Michael addition reaction, the biotin segment's peak shifts backwards to 7.2 min and is the most intensive peak within the reaction. The new peak arising at 15.7 min is supposed to be product related. The 5.7 min fractions of all runs were lyophilized. Nevertheless, MALDI-TOF measurements found no evidence of a reaction product.

The MALDI-TOF spectrum was recorded using 2,5-Dihydroxybenzoeic acid as matrix. The matrix was prepared as saturated solution in 1:1 H₂O/Acetonirile. On the MALDI-target 1 µL probe (approx. 0.8 pmol) was mixed with 1 µL matrix solution. The signal in the MALDI-TOF spectrum strongly indicates a relation between the puromycin segment (starting material) and the 15.7 min peak in HPLC. Also the fluoresceine signal (490 nm channel in HPLC) suggests a relation to the puromycin segment. But by MALDI-TOF it can't be the desired reaction product. The appearing peak matches more to the puromycin segment.

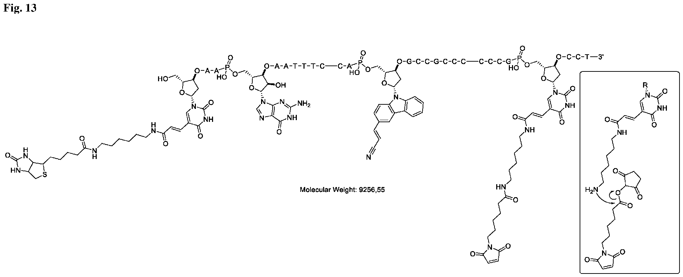

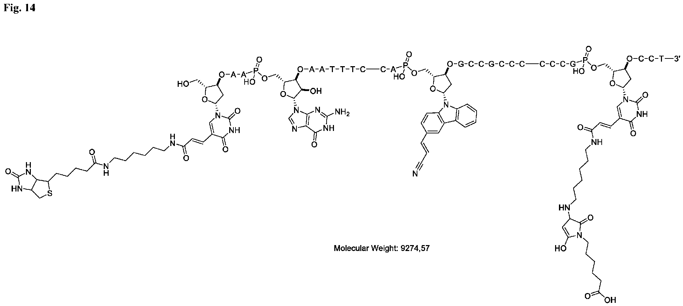

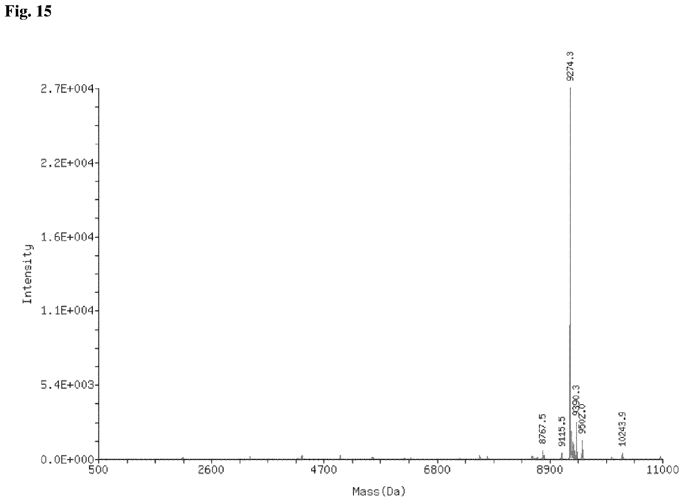

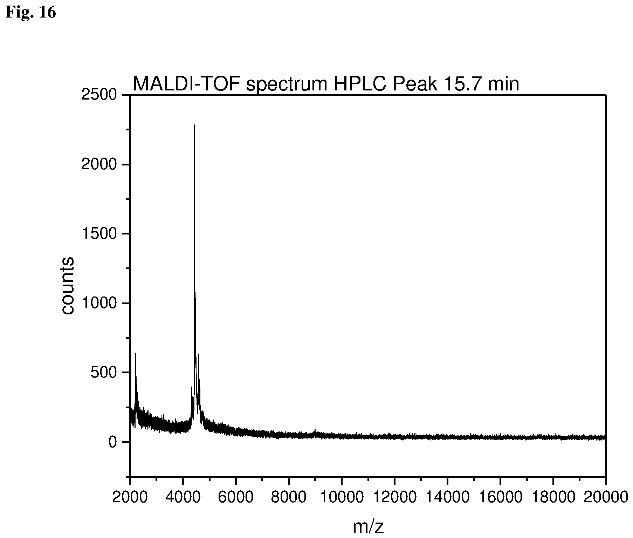

The MALDI-TOF spectrum showed that the only new HPLC peak arising at 15.7 min after reaction having both 260 nm and 490 nm signals, is not the desired product (Fig. 13), but an undesired product having the formula shown in Fig. 14 and the ESI-MS spectrum shown in Fig. 15. The MALDI-TOF spectrum is shown in Fig. 16

The EMCS reacted in a Michael reaction with the biotin segment (maleimide to primary amine of the C6-aminomodifier). The hydrolyzation fragile NHS-ester site hydrolyzed to a carbon acid that makes the whole molecule inert to reactions with the puromycin segment.

### 4.4 Click Chemistry according to the invention: Testings of different ratios of DBCO oligomer : Azide oligomer in the strain promoted azide alkine coupling reaction

In these testings, the experiment was conducted with two model substrates, a T28-DBCO oligomer and a T10 azidoPEG, the structures of both being shown in Fig. 23.

### 4.4.1 Reaction Ratio of Azide to DBCO 1:1

### Reaction preparation

16.9 µmol DBCO modified T28 oligomer were dissolved in 33.8 µL H₂O. 100 nmol AzidoPEG T10 oligomer were dissolved in 200 µL H₂O. Therefore, both oligos resulted in 500 µM solutions. For reaction control MALDI-TOF spectra of both oligomer solutions were recorded. In this case the SPAAC reaction was conducted in a 1:1 ratio (DBCO to Azido) what was realized by using 16µL of the 500 µM DBCO oligomer solution (8 nmol) and 16 µL of the 500 µM Azido-PEG oligomer (8 nmol) solution which resulted in 32 µL reaction volume. The reaction was incubated at 25 °C and 350 rpm for 3 h before MALDI-TOF reaction control measurements were conducted.

### Results

For MALDI-TOF investigations 0.5 µL probe were pipetted onto the MALDI target and subsequently merged with 1 µL saturated 2,5-Dihydroxybenzoeic acid (2,5-DHB) in Water/Acetonitrile solution (50% v/v).

### Table: Molecular Weights

| **Oligo** | **Mass [Da]** |
|---|---|
| T10 azidoPEG | 3407 |
| T28 DBCO | 8896 |
| T38 SPAAC product | 12303 |

The resulting spectra are shown in Fig. 24.

The MALDI-TOF spectra of the SPAAC reaction show a new peak around 12700 m/z-ratio caused by the SPAAC product. The peak around 4600 m/z-ratio that also appears in the MALDI-TOF spectrum of the T28 DBCO oligomer is the double charged species appearing at half its originally mass. The same is true for the peak appearing around 6300 m/z-ratio; it represents the double charged product species.

The mass peaks of MALDI-TOF do not match the theoretical masses of the used components for a technical reason. DNA collets multiple metal cations from the MALDI matrix and the target due it's negative charged phosphate backbone, which leads to immense mass alterations in the spectrometry. The effect increases the longer the DNA strand gets.

### 4.4.2 Reaction Ratio Azide to DBCO 1:2

### Reaction preparation

15.9 µmol DBCO modified T28 oligomer were dissolved in 32 µL 1x PBS buffer (pH 7.4). 13.5 µmol AzidoPEG T10 oligomer were dissolved in 28 µL 1x PBS buffer (pH 7.4). Therefore, both oligos resulted in 500 µM solutions. For reaction control MALDI-TOF spectra of both oligomer solutions were recorded. In this case the SPAAC reaction was conducted in a 2:1 ratio (DBCO to Azido) what was realized by using 30 µL of the 500 µM DBCO oligomer solution (15 nmol) and 15 µL of the 500 µM Azido-PEG oligomer (7.5 nmol) solution which resulted in 45 µL reaction volume. To bring the reaction concentration to a more realistic value that can be reproduced using the more complicated oligomers that will be used for the cDNA display, the reaction was diluted to 70 µL using 1x PBS buffer (pH 7.4). Another reason for further dilution was to have more reaction volume for HPLC investigations.

Therefore, the resulting reaction concentrations of the two oligomers were: ${c}_{T 28 - DBCO} = 214 μM$ ${c}_{T 10 - AzioPEG} = 107 μM$

The reaction was incubated over-night at 25 °C under light exclusion. The reaction was monitored by three time point MALDI-TOF measurements after 3 h, 6 h and 15 h. After 15 h the reaction was frozen and stored at -20 °C.

### Results

### Table: Molecular Weights

| **Name** | **Molecular Weight [Da]** |
|---|---|
| T10-AzidoPEG | 3407 |
| T28-DBCO | 8896 |
| T38-SPAAC (product) | 12303 |

MALDI-TOF spectra of the different oligos were recorded. All MALDI-TOF spectra were recorded using saturated 2,5-Dihydroxybezoeic 50% (v/v) acid in acetonitrile water solution as matrix. For probe preparation, 1 µL probe was mixed with 1 µL matrix solution. The results are shown in Fig. 25.

When comparing both the MALDI-TOF reaction control spectra to the spectra of the starting materials, it is to be noted that the T10-AzidoPEG oligomer nearly disappeared after 3 h reaction time, whereas the T28-DBCO oligomer was still present. Between the 3 h reaction time and 15 h over-night reaction no difference was observed.

The fact that there are no traces of the T10 oligomer indicates that it was completely consumed during the reaction. Thus, the reaction was quantitative after 3 h reaction time.

### 4.4.3: Reaction Ratio Azide to DBCO 2:1

16.09 nmol of DBCO modified T₂₈ oligomer were dissolved in 33.8 µL ddH₂O which results in a concentration of 0.5 mM. 100 nmol azide modified T₁₀ oligomer were dissolved in 200 µL ddH₂O to result in 0.5 mM. For Reaction, 15 µL of the DBCO-T₂₈ oligomer were merged with 30 mL of the azide-T₁₀ and incubated for 24 h at 25 °C and 350 rpm over-and -under rotation.

### Results

The complete disappearing of the T28-DBCO related peak around 9200 m/z-ratio shows that it was completely consumed during the reaction and the reaction therefore was complete. Setting the azide component in twofold exceed over the DBCO component leads to a quantitative reaction (consumption of the yield limiting compound).

The results are shown in Fig. 26.

### 5. Obtaining a bicyclic peptide using TBMB

The DNA polynucleotide encoding the peptide and including the hybridization sequence to the puromycin linker (figure 22, embodiments 42 and 43) is conserved in a bacterial expression vector of the type pRDV. To receive linear DNA polynucleotides to conduct the in vitro translation and up following cyclization of the peptide, the polynucleotide was amplified by PCR from the plasmid vector. Therefore, two custom primers were used. The 21 base forward primer named 'T7B' had the sequence (5' - 3') TTACTCGCTCCATGGGCGGC (SEQ ID NO:16). The used 39 base reverse primer named 'hybridization sequence reverse' or short Hybr.seq.rev had the sequence (5' - 3') ATAATCTTAAGCTTTCCATGATGATGATGATGATGGC (SEQ ID NO: 17). To guarantee that all PCR reaction preparations have the same concentration a 10x master mix was prepared and divided into 10 single reaction tubes afterwards.

### 5.1 PCR

**Table: Composition of the PCR reaction.**

| **Component** | **Volume [µL]** | **10x Master Mix [µL]** | **Negative control [µL]** |
|---|---|---|---|
| T7B pimer [5 µM] | 5 | 50 | 5 |
| Hybr.seq.rev. [5 µM] | 5 | 50 | 5 |
| 2x Phusion Master Mix | 12.5 | 125 | 12.5 |
| DNA Template | 1 | 15 | - |
| H₂O | 1.5 | 15 | 2.5 |
| total | 25 | 250 | 25 |

For chain reaction, the "Phusion 3 Step" program was used.

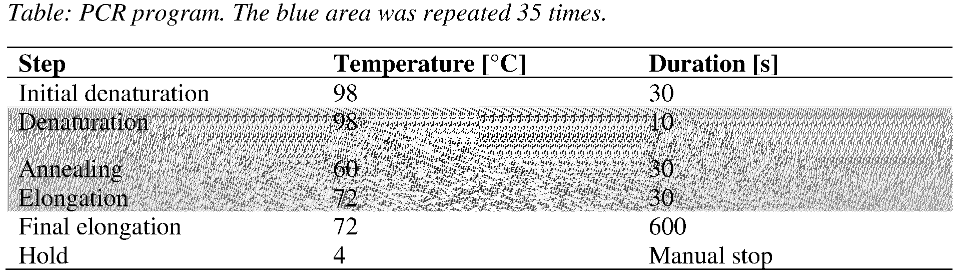

As a result of the PCR, a 222 base pair construct was expected. To ensure that the PCR reaction delivered the desired product, the reaction was examined by agarose gel electrophoresis using a 1.5% agarose gel. PCR cleanup was conducted using the "Machery Nagel Nucleospin Gel and PCR clean-up" kit. (see Fig,. 27).

The band appearing at 200 bp matches the theoretical expectation of 222 bp.

### 5.2 In vitro Transcription

| **Component** | **Volume [uL]** | **2x Master Mix** |
|---|---|---|
| 2x Buffer | 10 | 20 |
| PCR | 8 | 16 |
| Enzyme mix | 2 | 4 |
| H₂O | - | - |
| total | 20 | 40 |

30 min incubation at 37 °C. Thereafter, 1 µL DNase RQ1 was added and the reaction incubated for 15 min at 37 °C to digest the DNA template. The reaction was purified using the Promega "Relia-Prep RNA isolation and concentration" kit resulting in 30 µL of 2260 ng µL⁻¹ mRNA.

### 5.3 Crosslink Annealing

In preparation of the crosslink reaction between the puromycin linker and the mRNA, first they have to hybridize with teach other along their complementary sequences. This was accelerated and enhanced by a denaturation and slow cooling.

Table: Composition of the annealing and crosslink reaction. With the abbreviation PuL as puromycin linker.

| **Component** | **Volume [uL]** | **10x MasterMix [µL]** |
|---|---|---|
| Annealing Buffer | 2 | 20 |
| PuL [39 µM] | 2 | 20 |
| mRNA (2260 ng µL⁻¹) | 6 | 60 |
| Total | 10 | 100 |

The mixture was heated to 95 °C and cooled down to 25 °C over 45 min in a PCR cycler. The photo induced crosslink by [2+2] cycloaddition was conducted by 365 nm UV light 1200 mJ cm⁻². The resulting construct, a covalently connected hybrid molecule, consists of the puromycin linker and the peptideencoding mRNA.

### 5.4 Translation

The translation reaction in which the mRNA harboring at the puromycin linker was translated and afterwards trapped on the puromycin entity (Pur I) was conducted using an *E.coli* derived cell free translation kit named PUREfrex 1.0 by GeneFrontier Corporation. Therefore, the mRNA-ssDNA construct from the crosslinking step waas incubated in a two step procedure in the PUREfrex 1.0 composition. The first step was the translation reaction itself. Twenty *in vitro* reactions were prepared, because the later detection of the cyclic peptides by MALDI-TOF consumes a lot of material.

**Table: Composition of the. cell free in vitro translation reactions using the PUREfrex 1.0 kit 20 reactions.**

| **Componet** | **Volume [uL]** | **20x Master Mix [µL]** |
|---|---|---|
| Solution 1 (Amino acids, NTPs, tRNAs and substrates for enzymes) | 10 | 200 |
| Solution 2 (Proteins in 30% glycerol buffer) | 1 | 20 |
| Solution 3 (Ribosomes, 20 µM) | 1 | 20 |
| Crosslink | 8 | 90 |
| H₂O | 3.5 | 70 |
| total | 20 | 400 |

Though a 20x master mix was prepared, it was divided into 20 single reaction vials with 20 µL each, before incubation for 30 min at 37 °C. After this 30 min of incubation, a salt solution consistent of KCl (7.2 µL, 3.11 M), MgCl₂ (2.2 µL, 0.875 M) and H₂O (0.6 µL) was added to each vial which enhances the transfer of the still ribosome bound nescend peptide to the puromycin entity of the puromycin linker.

**Table: Salt additions for each reaction.**

| **Component** | **Volume [uL]** |
|---|---|
| Translation 30 min | 20 |
| KCl [3.11 M] | 7.2 |
| MgCl₂ [0.875 M] | 2.2 |
| H₂O | 0.6 |
| Total | 30 |

| | |
|---|---|
| 60 min incubation at 37 °C. | |

After the 60 min incubation step, the peptide was translated from the mRNA harboring at the puromycin linker and transferred to the puromycin entity.

### 5.5 SDS-PAGE

The success of the process of peptide production and transfer to the puromycin entity was examined by SDS-PAGE containing 8 M urea. Thereafter, probes of the puromycin linker alone, the crosslink reaction between the puromycin linker and the mRNA and the translation reaction were denaturated in Lämmli buffer (5 min at 72 °C).

**Table: Composition of the SDS-PAGE probes.**

| **Component** | **PuL [µL]** | **Crosslink [µL]** | **Translation reaction [µL]** |
|---|---|---|---|
| Probe | 0.5 | 1 | 5 |
| H₂O | 14.5 | 14 | 10 |
| 2x Lämmli buffer | 15 | 15 | 15 |
| total | 30 | 30 | 30 |

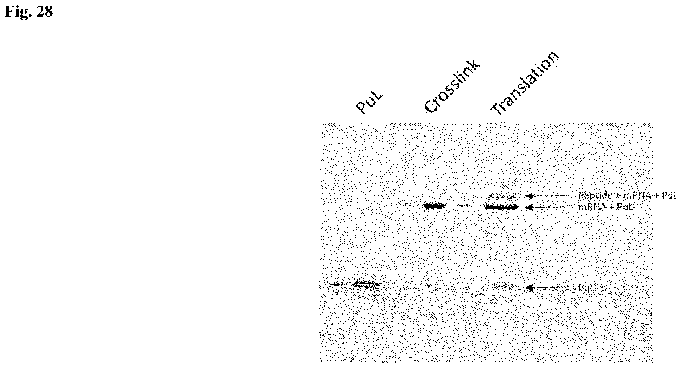

For examination, the SDS-PAGE was checked with a fluorescence scanner to detect the fluorescein modification of the puromycin linker.
SDS Page - see Fig. 28

The band scheme the figure shows a successful translation. The translation efficiency was determined with imageJ.

### 5.6 TBMB cyclization

For faster workflow and easier changing of buffers and reactants during the cyclization, the peptide comprising the puromycin linker and mRNA was immobilized on magnetic streptavidin beads. Therefor, 250 mL magnetic Straptavidin beads were equilibrated 3 times in 700 µL B/W buffer (20 mM Tris-HCl, 0.5 M NaCl, 1 mM EDTA, pH 7.5). Thereafter, the 20 PUREfrex 1.0 in vitro translation reactions were pooled in one vessel, which was afterwards divided into two fractions. This pooling was necessary to ensure that both of the later fractions, it was divided into, include the same amount of peptide-puromycin linker-mRNA construct. Thereafter, each of the two fractions (100 µL) was diluted with B/W buffer (20 mM Tris-HCl, 0.5 M NaCl, 1 mM EDTA, pH 7.5) to a total volume of 700 µL. The 700 µL reaction volume were pipetted onto the magnetic beads. Incubation for 45 min at 4 °C and 15 rpm (over and under rotation). To reduce possible disulfide bonds of the peptide's cysteine residues, one of the two fractions was equilibrated three times in 700 µL NH₄HCO₃ buffer (20 mM, 5 mM EDTA, pH 8). Incubation in NH₄HCO₃ buffer (693 µL) including dithiothreitol (DTT) (0.1 M, 7 µL) for 1 h at 37 °C and 15 rpm (over and under rotation). The reaction buffer was removed on magnetic stand. 2 µL TBMB in acetonitrile (100 mM) were merged with 698 mL NH₄HCO₃ buffer and the mixture was incubated for 1 h at 37 °C and 15 rpm (over and under rotation).

Meanwhile, the other fraction was stored on ice. Only one of the two fractions underwent reduction and cyclization process, because for comparison in MALDI-TOF mass spectrometry, a linear and cyclic peptide version was required.

### 5.7 Treatment with DNase RQ1 and lyophiliazation

After incubation, the beads of both fractions were washed (separately) with 700 µL DNase RQ1 buffer, before the beads were resuspended in 700 µL fresh DNase RQ1 buffer. To digest the puromycin linker, 5 µL DNase RQ1 enzyme were added and the mixture was incubated for 30 min at 37 °C and 15 rpm (over and under rotation). The buffer was exchanged for ddH₂O using a Satorius Vivaspin concentrator with 2000 MWCO. As a last step, the solution was lyophilized. This digestion is necessary, because the 222 base mRNA and the 38 base equivalent puromycin linker add so much weight to the peptide that it would not possible to examine it in the accessible MALDI-TOF mass spectrometer.

### 5.8. MALDI-TOF

The 4046.42 Da mass detected in the MALDI-TOF spectrum from HCCA-matrix (see Fig. 29) relates to the peptide with the sequence of
"MGGACYSVVCHVMWICGSGHHHHHHG-puromycin-cytosine-cytosine" (SEQ ID NO: 15)
and the structure

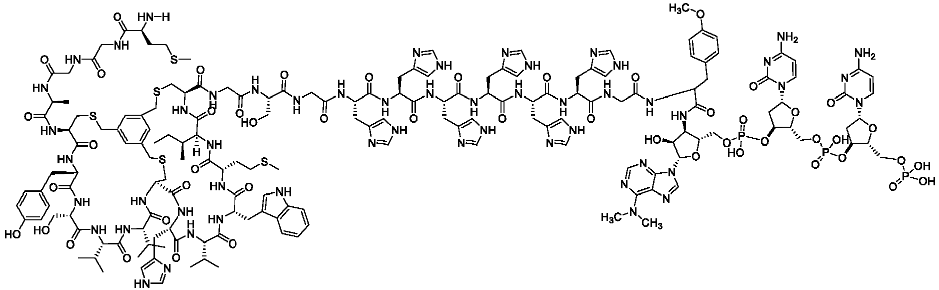

With the incorporated Tris-methylbenzene (former TBMB) it achieves a measured mass of 4064 Da. Reflecting, that the theoretical mass is 4063 Da and that in mass spectrometry ionization, there is a Cation addition which causes a mass shift about 1 Da (hydrogen) or about 23 (sodium). The peak around 4088 Da correlates with the sodium adduct of the cyclic peptide.

### 5.8.Summary

We created bicyclic peptides during cDNA-display by treating the hybrid molecule immobilized on magnetic beads with TBMB in the presence of NH₄HCO₃ buffer.

### 6. First panning round on EpCAM

### 6.1.Experimental section

### 6.1.1 Library

A library encoding peptides of the structure CysN4CysN5Cys was constructed, which has 9 positions for variable amino acids, so the number of possible variants (Nᵥ) is calculated according to formula (1). ${N}_{v} = {19}^{9} = 3.23 ⋅ {10}^{11}$ $\begin{matrix}\frac{3.23 ⋅ {10}^{11}}{6.022 ⋅ {10}^{23}} mol = 5.19 ⋅ {10}^{- 13} mol \\ = 0.5 pmol\end{matrix}$

Following Equation 1 and 2, a library theoretically comprising one copy of each variant requires a substance amount of at least 0.5 pmol.

### 6.1.2 In vitro transcription

**Table 1: In vitro transcription. Promega T7 ribo max express.**

| **Component** | **Volume [ µL** |
|---|---|
| 2x T7 buffer | 10 |
| DNA [104 µg/µL] | 8 |
| T7 Enzyme mix | 2 |
| Total | 20 |

- Incubation for 30 min at 37 °C.
- Addition of 2 µL DNase RQ1.
- Incubation for 15 min at 37 °C.

The clean-up was managed by using Cytivia "MicroSpin G-50 columns". The probe volume was filled up to 50 µL.
- Vortex column
- Place in delivered container and centrifuge for 1 min at 2000 rcf.
- Discard container and flow through.
- Place column in clean container and load IVT reaction.
- Spin for 2 min at 2000 rcf.
- Measure concentration.

Nano drop measurements resulted in 576 ng µL⁻¹.

At a length of 222 bp, 576 ng µL⁻¹ are 7.62 pmol µL⁻¹ and 14 library copies per µL.

### 6.1.3 Annealing

**Table 2: Annealing of Pu-linker and mRNA**

| **Component** | **Volume [µL]** |
|---|---|
| Puromycin linker [3.2 µM] | 4.8 |
| RNA [7.62 mM] | 2 |
| Annealing buffer (150 mM TrisHCL, 600 MM NaCl) | 2 |
| H₂O | 1.2 |
| Total | 10 |

The mixture was heated to 95 °C and cooled down to 25 °C over 45 min in a PCR cycler. The photo induced crosslink was conducted by 365 nm UV light 1200 mJ cm⁻² (see Figure 31)

For the calculation of the crosslink efficiency the grey values of the crosslink and PuL 490 nm fluorescence within the crosslink track were measured
Crosslink efficiency is 76%

### 6.2 in vitro translation

**Table 3: Translation mix PUREfrex 1.0**

| **Component** | **Volume [uL]** |
|---|---|
| Solution I | 10 |
| SolutionII | 1 |
| Solution III | 1 |
| Crosslink (from Example 6.1.3) | 7 |
| H₂O | 1 |
| total | 20 |

| | |
|---|---|
| Incubation for 30 min at 37 °C. | |

**Table 4: Salt additions after 30 min incubation.**

| **Component** | **Volume** |
|---|---|
| Translation mix | 20 |
| KCl [3.11 M] | 7.2 |
| MgCl₂ | 2.2 |
| H₂O | 0.6 |
| Total | 30 |

| | |
|---|---|
| Further incubation for 60 min at 37 °C. | |

The SDS - page of the translation is shown in Fig. 32.

For the calculation of the translation efficiency the grey values of the translation and crosslink 490 nm fluorescence within the translation track were measured.

**Table 5: Grey values.**

| **Status** | **Grey value** |
|---|---|
| Crosslink | 54846 |
| Efficiency | 67297 |

### Translation Efficiency 55%.

### 6.3 Reverse transcription

- Wash 100 µL streptavidin magnetic beds 3 times with 700 µL B/W buffer.
- Dilute translation reaction to 700 µL with B/W buffer.
- Incubate reaction for 45 min at 4 °C and 15 rpm rotation on magnetic beads.
- Wash 1x with 500 µL SS IV buffer.

**Table 6: Composition reverse transcription.**

| **Component** | **Volume** |
|---|---|
| 5x RT buffer SS IV | 10 |
| dNTPs | 2.5 |
| DTT [100 mM] | 1 |
| Reverse transcriptase | 1 |
| Streptavidin-beads + mRNA | solid |
| H₂O | 35.5 |
| Total | 50 |

| | |
|---|---|
| Incubation for 30 min at 52 °C and 450 rpm shaking. | |

### 6.4 TBMB cyclization

Preparation of a TBMB stock solution; 36 mg in 10 mL acetonitrile [10 mM]. The beads from Example 6.3 were washed 3 times with 500 µL 20 mM NH₄HCO₃ buffer pH 8 and. 5 µL TBMB [10 mM] was added to 495 µL NH₄HCO₃ [20 mM] buffer and pipetted onto the magnetic beads. Incubation was for 45 min at 37 °C and 450 rpm. The post translational modified cDNA-monoblock molecule was cleaved from the streptavidin beads by washing the beads 3 times with 500 µL "Dynabeads HIS isolation" B/W buffer and finally adding 100 µL "Dynabeads HIS isolation" B/W buffer including 2 µL RNase T1. The mixture was incubated for 15 min at 37 °C and 350 rpm orbital shaking. The reaction was placed on a magnet and the supernatant was frozen at -20 °C over-night.

### 6.5 Immobilization on His-Beads

- 100 µL Dynabeads HIS-Isolation were washed 3x with 500 µL B/W (his beads) buffer.
- The RNAse T1 reaction was filled up to 700 µL with His beads B/W buffer.
- Incubation of the reaction on the magnetic HIS beads for 30 min at RT and 10 rpm rotation.
- Washing 3 times with His B/W buffer.
- Adding 100 µL Elution buffer, 10 min RT, 10 rpm rotation.

For clean-up, a Bio Spin 6 column was equilibrated with "Dynabeads Protein A" buffer (3x 500 µL, spin 1 min at 1000 rcf). The 100 µL Reactions were loaded onto the column and centrifuged at 1000 rcf for 1 min.

### 6.6 Panning on EpCAM

50 µL "Dynabeads Protein A" were washed with 500 µL PBS 0.02% Tween20 (protein A buffer). 2 µL EpCAM (100 nM) were diluted in 198 mL Protein A buffer. The EpCAM dilution was pipette onto the magnetic beads and incubated 10 min at RT and 15 tpm rotation. Thereafter, the tube was placed on a magnetic stand and the supernatant was discarded. 100 µL cDNA-display molecule were added to the magnetic beads. Incubation for 1 h at 4 °C and 15 rpm. The supernatant was withdrawn from the beads and stored at -20 °C. The beads were washed with 1.5 mM MgCl₂ buffer. After washing, the beads were transferred into a 500 µL PCR-tube.

### 6.7 PCR

**Table 7: PCR composition**

| **Component** | **Volume [µL]** | **Volume negative control [µL]** |
|---|---|---|
| Phusion 2x Master Mix | 50 | 10 |
| Primer T7 B [10 µM] | 5 | 2.5 |
| Primer hybr. seq. rev [10 µM] | 5 | 2.5 |
| H₂O | 40 | 10 |
| Template | Solid on magnetic beads | - |
| Total | 100 | 25 |

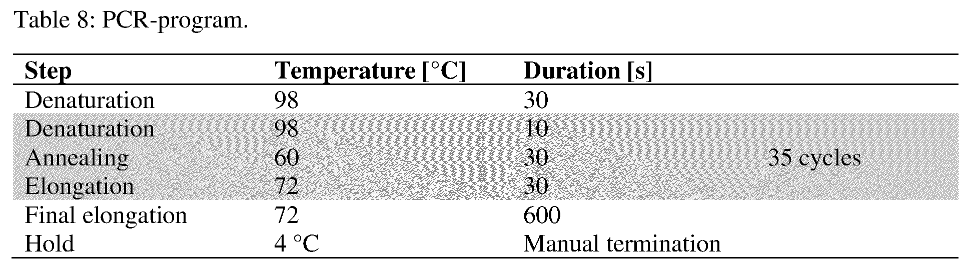

The PCR was prepared as listed in Table 7 and performed as listed in Table 8. After PCR, the supernatant was removed and stored at -20 °C over night. The PCR was analyzed by a 2% agarose gel electrophoresis. 20 µL of PCR or negative control were merged with 4 µL 6x Purple Loading Dye (see Fig. 33)

The 222 bp PCR product **(Fehler! Verweisquelle konnte nicht gefunden werden.3** A) was extracted from the gel at a concentration of 33 ng µL⁻¹ **(Fehler! Verweisquelle konnte nicht gefunden werden.3** B) and cloned into the pRDV_hybr.seq vector.

### 6.8 Cloning in pRDV_hybr.seq vector

**Table 9: Digestion for cloning into pRDV_hybr.seq**

| **Component** | **Volume [uL]** |
|---|---|
| DNA (33 ng µL⁻¹) | 10 |
| CutSmart 10x | 5 |
| NcoI | 1 |
| HindIII | 1 |
| H₂O | 33 |
| Total | 50 |

Incubation for 30 min at 37 °C. Purification was performed using the Machery-Nagel Nucleo Spin PCR and Gel cleanup kit. After cleanup, the DNA concentrations were measured.

**Table 10: DNA constructs after digestion.**

| **Construct** | **Concentration [ng µL⁻¹]** | **Length [bp]** |
|---|---|---|
| 3Cys4N5N from PCR | 20 | 80 |
| pRDV_hybr.seq | 54 | 3047 |

**Table 11: Ligation reaction of polynucleotides from panning process and pRDV_hybr.seq vector.**

| **Component** | **Volume [uL]** |
|---|---|
| T4 ligase buffer | 2 |
| T4 DNA ligase | 1 |
| pRDV_hybr.seq | 3 |
| 3Cys4N5N | 1 |
| H₂O | 13 |
| Total | 20 |

Incubation for 2 h at 25 °C. 3 µL of the ligation reaction (Table 11) were added to a 50 µL vial of DH10B cells and incubated for 1 min on ice. Thereafter, the bacteria cells were transferred into an electroporation cuvette and electroporated at 2500 V. The competent cells were suspended in 1 mL LB-medium (no antibiotics) and incubated for 1 h at 37 °C and 200 rpm shaking. 100 µL were plated on a 10 cm agar plate containing ampicillin and the plate was incubated over-night at 37 °C. 10 clones were picked and incubated in 3 mL LB-medium containing ampicillin over-night at 37 °C and 200 rpm shaking.

Of 10 clones sequenced, one (HQ0318, SEQ ID NO:18) encoded an expected Ep-Cam binding sequence (SEQ ID NO:19, Fig. 34.

## Claims

1. A method for producing a display complex, the method comprising
(I) providing a covalent mRNA-Puromycin Linker conjugate;
(II) contacting the covalent mRNA-Puromcyin linker conjugate with an in vitro translation composition to produce a display complex comprising a polypeptide encoded by said mRNA,
(III) optionally reverse transcribing said mRNA to a cDNA, and
(IV) cyclisation of the polypeptide comprised in said display complex.

2. The method of claim 1, wherein step (I) providing a covalent mRNA-Puromycin Linker conjugate comprises
(a) contacting a Puromycin Linker (I) with an mRNA;
(b) photocrosslinking said mRNA and said Puromycin Linker, and thereby
(c) producing a covalent mRNA-Puromcyin Linker conjugate.

3. The method of claim 1 or 2, wherein said Puromycin Linker (I) comprises a building block A comprising a Puromycin group and a building block B comprising a single-stranded polynucleotide segment essentially complementary to a sequence on the 3'-terminal side of the mRNA over a stretch of at least 3, preferably at least 12 nucleotides, more preferably at least 15, more preferably at least 22 nucleotides. wherein (A) and (B) are linked via the functional moiety a'-b', the linking group a'-b' having the structure

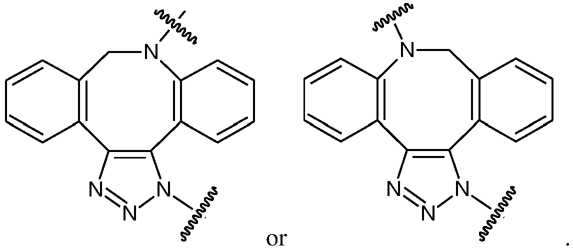

4. The method of claim 2, wherein the Puromycin Linker according to step (a) is prepared by a method (P) comprising reacting
- a building block A, said building block comprising a Puromycin group (Pur) and a functional group FG^{a}, via said functional group FG^{a}, wherein the functional group FG^{a} is -N₃,
- with a building block B comprising a functional group FG^{b} via said functional group FG⁸, wherein the functional group FG⁸ is 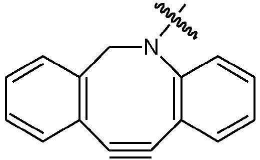
thereby forming a covalent linkage between A and B,
wherein B further comprises and a single-stranded polynucleotide segment essentially complementary to a sequence on the 3'-terminal side of the mRNA over a stretch of at least 3, preferably at least 12 nucleotides, more preferably at least 15, more preferably at least 22 nucleotides, wherein the functional group FG^{a} and the functional group FG^{b} are linked a strain-promoted azide-alkyne cycloaddition.
wherein upon reaction of the functional group FG^{a} with the functional group FG⁸, a linking group a'-b' is formed, the linking group a'-b' having the structure

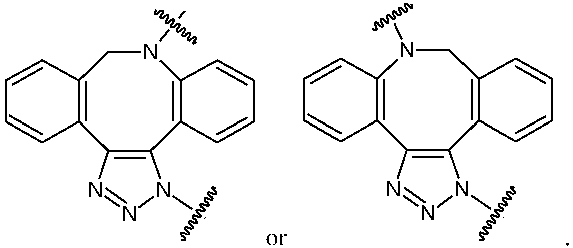

5. The method according to claim 4, wherein in method (P), the molar ratio of building block A to building block B is of from 1.1 : 1 to 5:1, preferably of from 1.5 :1 to 3:1, more preferably around 2:1.

6. The method according to claim 4 or 5, wherein in method (P), building block A and B are reacted with each other for a time in the range of from 5 min to 24h, preferably of from 10 min to 10 h, more preferably of from 2 h to 5 h, more preferably around 3 h.

7. The method according to any one of claims 1 to 6, wherein the the Puromycin Linker
(I) has the structure (Ia) 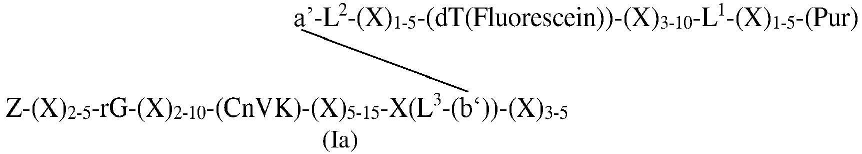
wherein Pur is a puromycin group, each X is independently selected from A, T, rG, G, and C, wherein rG is ribo-guanosine, and A, T, G, an C are deoxynucleotides and
wherein L³ is a linker and CnVK is a cyanovinylcarbazol group, and wherein Z is dT-Biotin or a group having the structure: 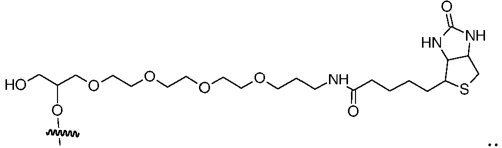
and wherein L1 and L2 are linker, and wherein dT(Fluorescein) is fluorescein-labeled deoxy-thymidin and preferably having the structure: 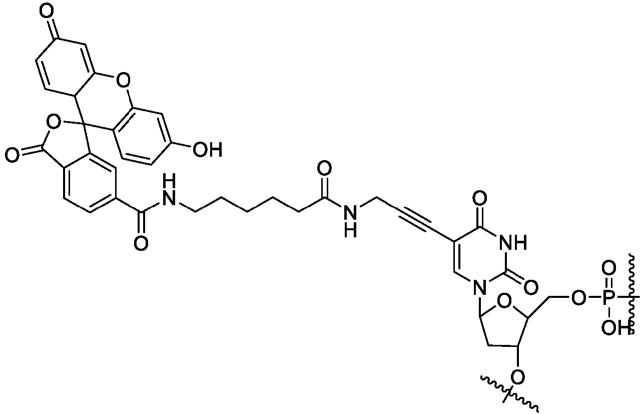
the linking group a'-b' having the structure

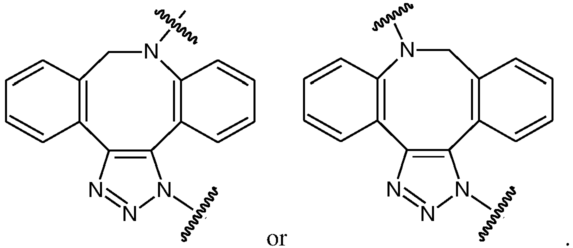

8. The method according to claim 7, wherein the the Puromycin Linker (I) has the structure (Iaaa) or the structure (Iaaa*) or a mixture thereof

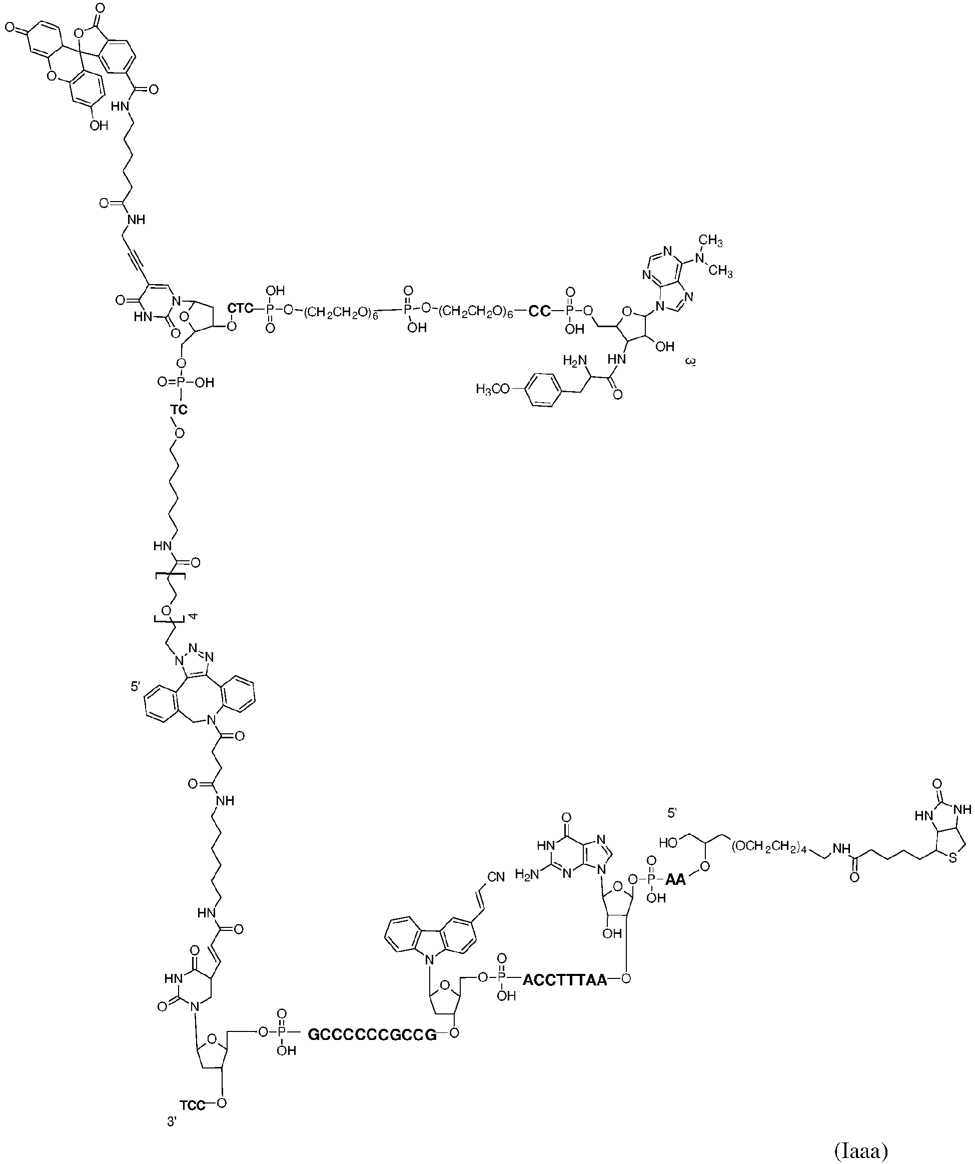

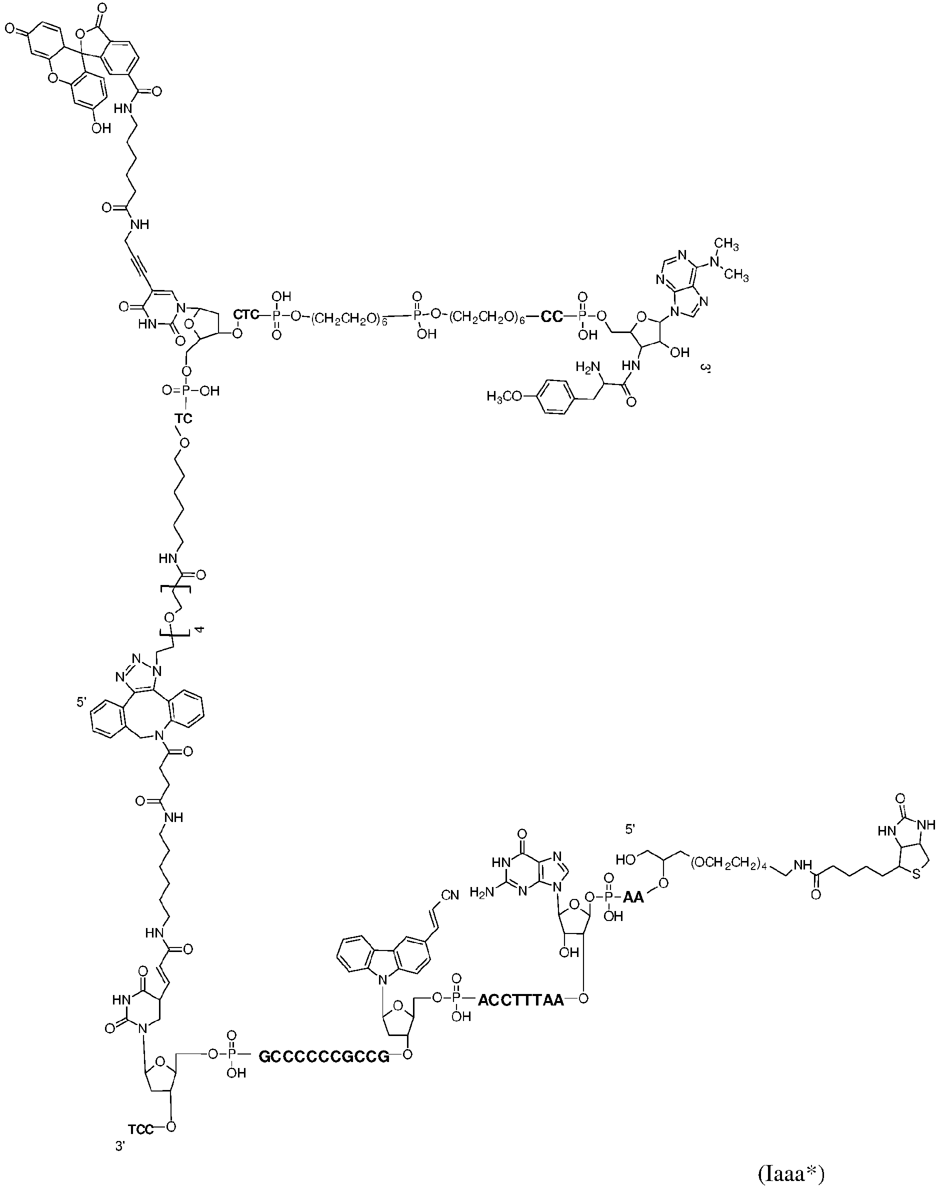

9. The method according to any one of claims 1 to 8, wherein the the Puromycin Linker (I) has the structure (Ib): 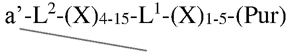 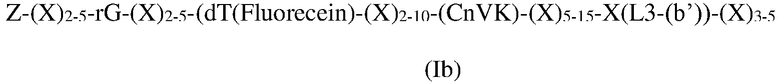
wherein Pur is a puromycin group, and wherein each X is independently selected from A, T, G, and C, wherein L¹ and L² are linker, wherein rG is ribo-guanosine, wherein A, T, G, an C are deoxynucleotides, and wherein L³ is a linker and CnVK is a cyanovinylcarbazol group, and wherein Z is dT-Biotin or a group having the structure: 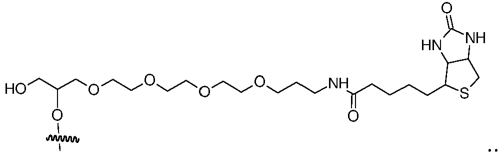
and wherein dT(Fluorescein) is fluorescein-labeled deoxy-thymidin and preferably having the structure : 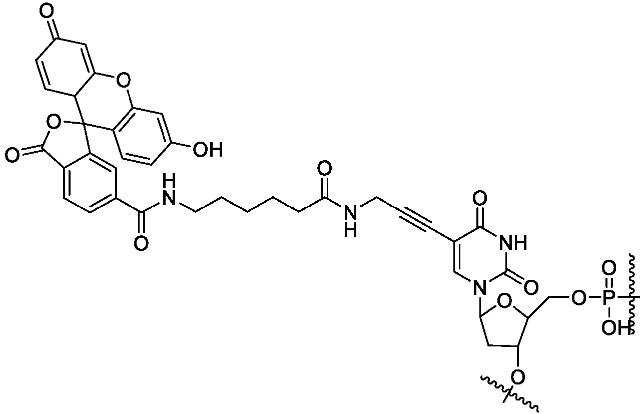
the linking group a'-b' having the structure

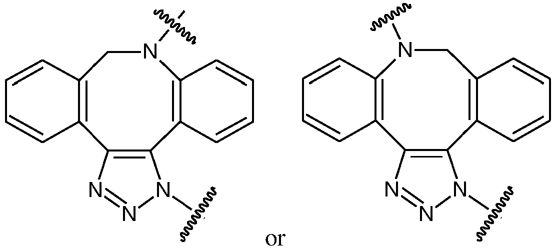

10. The method according to claim 9, wherein the the Puromycin Linker (I) has the structure (Ibbb) or the structure (Ibbb*) or a mixture thereof

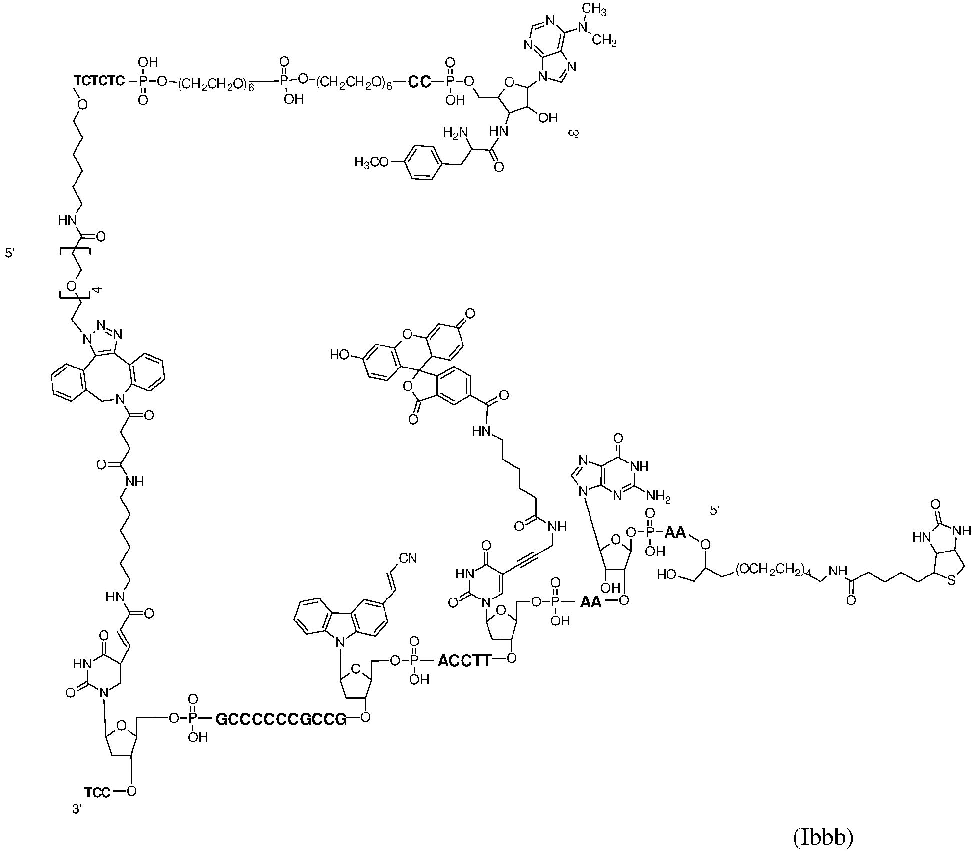

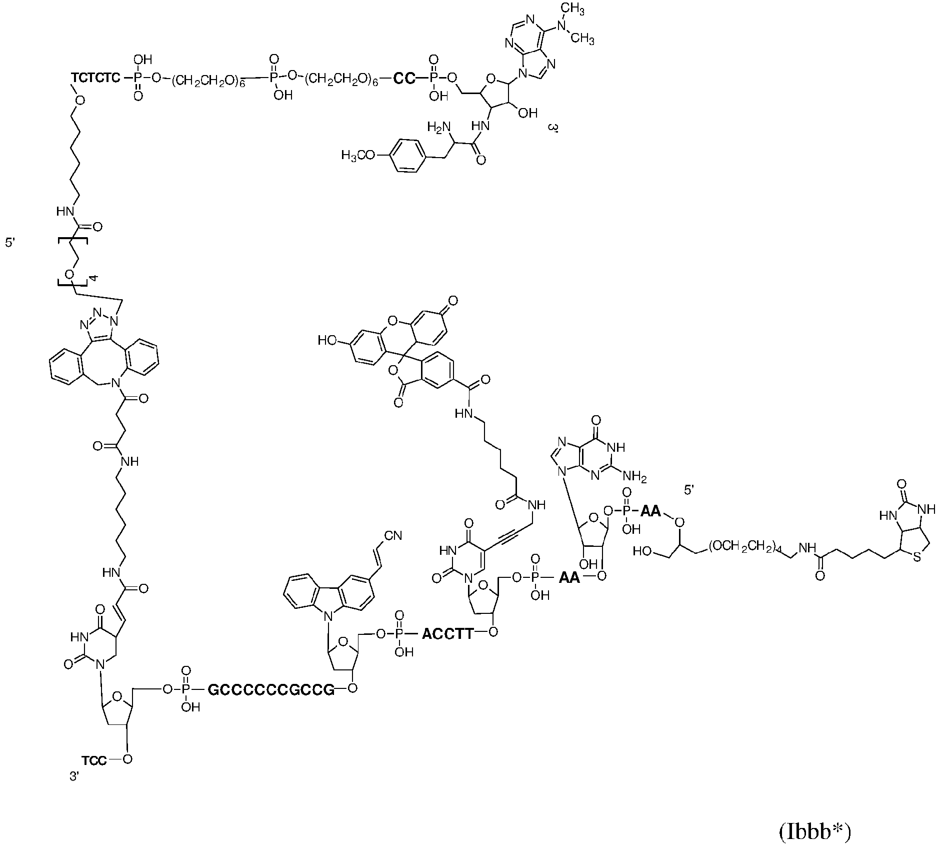

11. A display complex obtained by the method according to the method of any one of claims 1 to 10.

12. A protein array obtained by immobilizing a multitude of display complexes according to claim 11 on a substrate, preferably in a spatially ordered manner.

13. A method for identifying a binding compound binding to a compound of interest via cDNA display, comprising the steps of the method of any one of claims 1 to 10.
